(19) Europäisches Patentamt
European Patent Office
Office européen des brevets

(11) Veröffentlichungsnummer: **0 242 518 B1**

(12) # EUROPÄISCHE PATENTSCHRIFT

(45) Veröffentlichungstag der Patentschrift: **10.04.91 Patentblatt 91/15**

(51) Int. Cl.$^5$: **C07D 209/80**, A61K 31/40, C07C 311/15

(21) Anmeldenummer: **87101901.4**

(22) Anmeldetag: **11.02.87**

(54) **Cycloalkano[1,2-b]indol-sulfonamide.**

(30) Priorität: **21.02.86 DE 3605566**
**19.09.86 DE 3631824**

(43) Veröffentlichungstag der Anmeldung: **28.10.87 Patentblatt 87/44**

(45) Bekanntmachung des Hinweises auf die Patenterteilung: **10.04.91 Patentblatt 91/15**

(84) Benannte Vertragsstaaten:
**AT BE CH DE ES FR GB GR IT LI LU NL SE**

(56) Entgegenhaltungen:
**DE-A- 2 125 926**
**DE-A- 2 226 702**
**DE-B- 1 695 703**
**FR-A- 1 415 322**
**GB-A- 1 487 989**
**US-A- 4 235 901**

(73) Patentinhaber: **BAYER AG**
**W-5090 Leverkusen 1 Bayerwerk (DE)**

(72) Erfinder: **Böshagen, Horst, Dr.**
**Wiesenstrasse 4**
**W-5657 Haan (DE)**
Erfinder: **Rosentreter, Ulrich, Dr.**
**Kondorweg 23**
**W-5600 Wuppertal 1 (DE)**
Erfinder: **Lieb, Folker, Dr.**
**Alfred-Kubin-Strasse 1**
**W-5090 Leverkusen 1 (DE)**
Erfinder: **Oediger, Hermann, Dr.**
**Roggendorfstrasse 51**
**W-5000 Köln 80 (DE)**
Erfinder: **Seuter, Friedel, Dr.**
**Moospfad 16**
**W-5600 Wuppertal 1 (DE)**
Erfinder: **Perzborn, Elisabeth, Dr.**
**Am Tescher Busch 13**
**W-5600 Wuppertal 11 (DE)**
Erfinder: **Fiedler, Volker-Bernd, Dr.**
**Lehner Mühle 46**
**W-5090 Leverkusen 3 (DE)**

EP 0 242 518 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Cycloalkano[1.2-b]indol-sulfonamide, Verfahren zu ihrer Herstellung und ihre Verwendung in Arzneimitteln. Ebenfalls neue (Benzolsulfonamidoalkyl)cycloalkano[1.2-b]indole können als Zwischenprodukte bei der Herstellung der neuen Verbindungen verwendet werden.

Es wurden neue Cycloalkano[1.2-b]indol-sulfonamide der allgemeinen Formel (I)

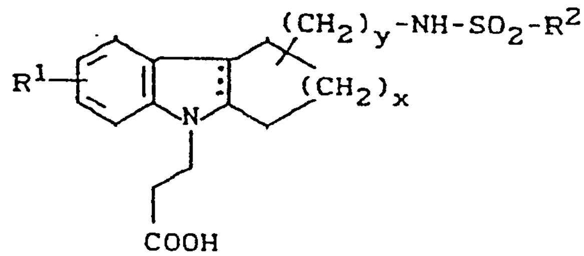

(I),

in welcher

$R^1$ – für Wasserstoff, Fluor, Chlor, Brom, Trifluormethyl, Carboxy, $C_1$-$C_6$-Alkoxycarbonyl steht, für eine Gruppe der Formel

$-S(O)_mR^3$ steht,

worin

$R^3$ – $C_1$-$C_6$-Alkyl oder Phenyl bedeutet und

m – eine Zahl 0 oder 2 bedeutet,

– für eine Gruppe der Formel

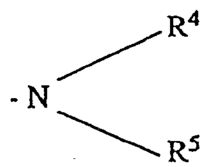

steht,

worin

$R^4$ und $R^5$ gleich oder verschieden sind und Wasserstoff, $C_1$-$C_6$-Alkyl, Phenyl oder Acetyl bedeuten,

– oder für eine Gruppe der Formel

$-OR^6$ steht,

worin

$R^6$ – Wasserstoff, $C_1$-$C_6$-Alkyl oder Phenyl bedeutet,

– oder für gegebenenfalls durch Carboxy, Methoxycarbonyl, Ethoxycarbonyl, Fluor, Chlor, Brom, Hydroxy, $C_1$-$C_6$-Alkoxy oder Cyano substituiertes $C_1$-$C_6$-Alkyl steht,

$R^2$ – für Phenyl steht, das gegebenenfalls bis zu dreifach durch Fluor, Chlor, Brom, Cyano, Trifluormethyl, Trifluormethoxy, Trifluormethylthio, $C_1$-$C_6$-Alkyl, Carboxymethyl, Carboxyethyl, Methoxymethyl, Ethoxymethyl, Methoxyethyl, Ethoxyethyl, $C_1$-$C_6$-Alkoxy, $C_1$-$C_6$-Alkylthio, Hydroxy, Carboxy, $C_1$-$C_6$-Alkoxycarbonyl, Phenyl, Phenoxy, Benzyloxy, Benzylthio oder durch die Gruppe

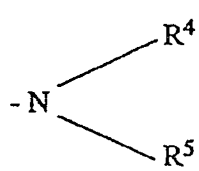

substituiert ist,

worin

$R^4$ und $R^5$ die bereits angegebene Bedeutung haben,

x – für die Zahl 1 oder 2 steht,

und

y – für die Zahl 0 oder 1 steht,

gegebenenfalls in einer isomeren Form, oder deren Salze, gefunden.

Die erfindungsgemäßen Cycloalkano[1.2-b]indol-sulfonamide haben mehrere asymmetrische Kohlenstoffatome und können daher in verschiedenen stereochemischen Formen existieren. Sowohl die einzelnen Isomeren als auch deren Mischungen sind Gegenstand der Erfindung.

Beispielsweise seien die folgenden isomeren Formen der Cycloalkano[1.2-b]indol-sulfonamide genannt :

a) Cycloalkano[1,2-b]indol-sulfonamide

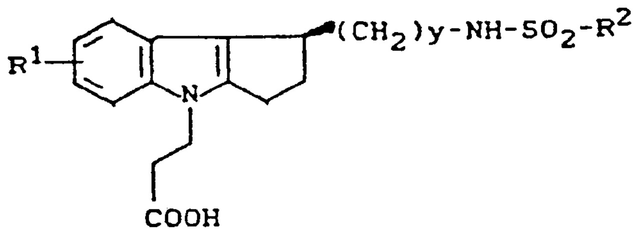

(II)

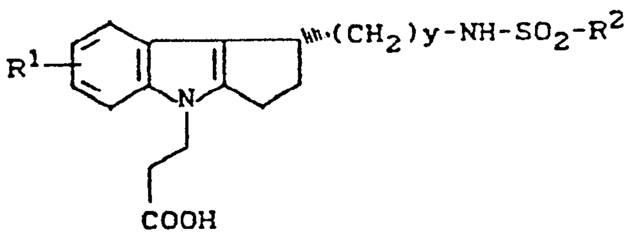

(III)

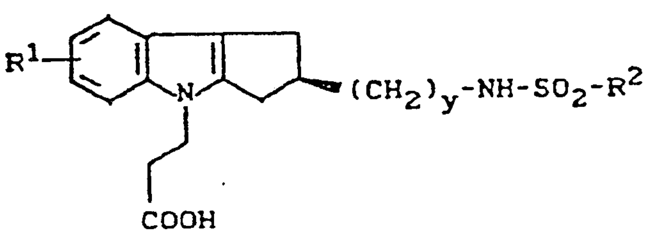

(IV)

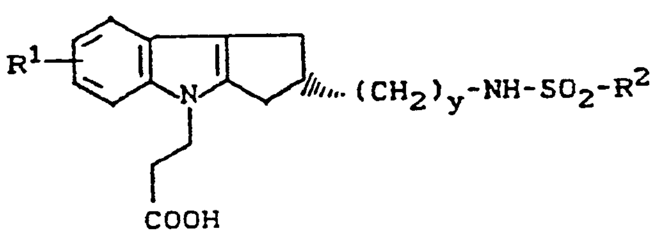

(V)

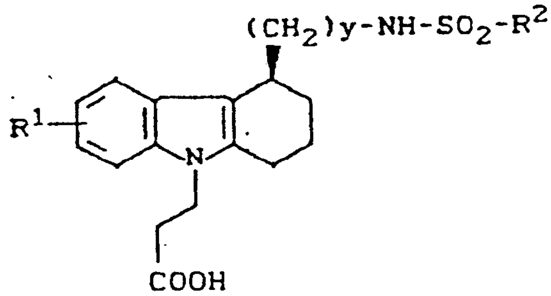

(VI)

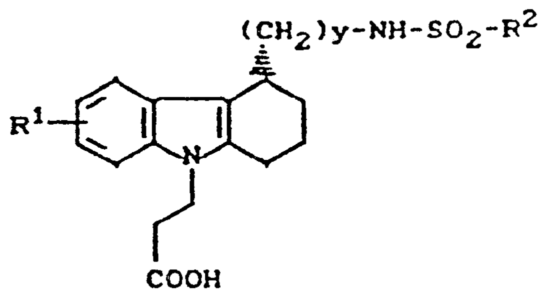

(VII)

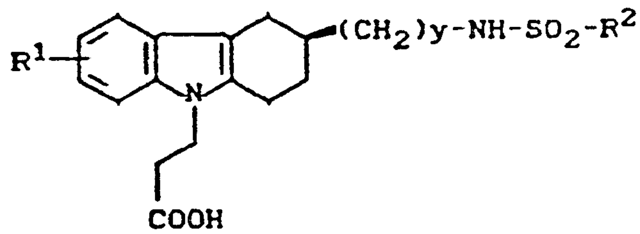

(VIII)

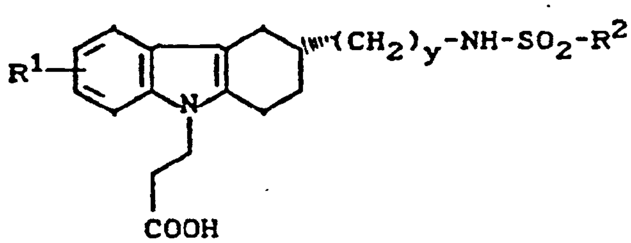

(IX)

b) Cycloalkano[1,2-b]dihydroindol-sulfonamide

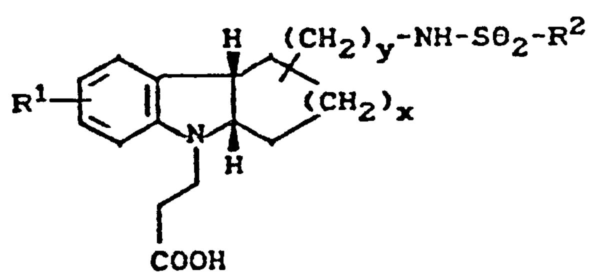

(X)

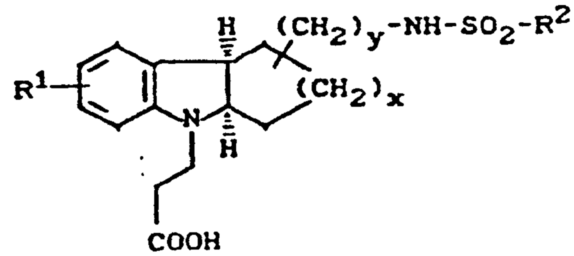

(XI)

wobei $R^1$, $R^2$, x und y die oben genannte Bedeutung haben.

Die erfindungsgemäßen Cycloalkano[1.2-b]indol-sulfonamide können auch in Form ihrer Salze vorliegen. Im allgemeinen seien hier Salze mit organischen oder anorganischen Basen genannt.

Im Rahmen der vorliegenden Erfindung werden physiologisch unbedenkliche Salze bevorzugt. Physiologisch unbedenkliche Salze der Cycloalkano[1.2-b]indol-sulfonamide können Metall- oder Ammoniumsalze der erfindungsgemäßen Stoffe, welche eine freie Carboxylgruppe besitzen, sein. Besonders bevorzugt sind z.B. Natrinum-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin oder Ethylendiamin.

Die erfindungsgemäßen Stoffe zeigen überraschenderweise eine thrombozytenaggregationshemmende Wirkung und können zur therapeutischen Behandlung von Menschen und Tieren verwendet werden.

Bevorzugt sind solche Verbindungen der allgemeinen Formel (I), in welcher

$R^1$ – für Wasserstoff, Fluor, Chlor, Brom, Trifluormethyl, Methylthio, Ethylthio, Methylsulfonyl, Phenylthio, Phenylsulfonyl, Amino, Dimethylamino, Diethylamino, Acetylamino steht, oder

– für eine Gruppe der Formel

– $OR^6$ steht,

worin

$R^6$ – Wasserstoff, $C_1$-$C_4$-Alkyl oder Phenyl bedeutet,

– oder für $C_1$-$C_4$-Alkyl steht,

$R^2$ – für Phenyl steht, das bis zu zweifach gleich oder verschieden durch Fluor, Chlor, Brom, Cyano, Trifluormethyl, Trifluormethoxy, $C_1$-$C_4$-Alkyl, $C_1$-$C_4$-Alkoxy, Methylthio, Hydroxy, Methoxycarbonyl, Ethoxycarbonyl, Dimethylamino, Acetylamino, Diethylamino substituiert ist,

x – für die Zahl 1 oder 2 steht, und

y – für die Zahl 0 oder 1 steht, gegebenenfalls in einer isomeren Form,

oder deren Salze.

Besonders bevorzugte Verbindungen der allgemeinen Formel (I) sind solche, in denen

$R^1$ – für Wasserstoff, Fluor, Methyl, Methoxy, Benzyloxy oder Hydroxy steht,

$R^2$ – für Phenyl steht, das durch Fluor, Chlor, Trifluormethyl, Methyl, Ethyl, Propyl, Isopropyl oder Methoxy substituiert ist,

x – für die Zahl 1 oder 2 steht,

und

y – für die Zahl 0 oder 1 steht, gegebenenfalls in einer isomeren form, oder deren Salze.

Insbesondere bevorzugt werden (+)- oder (–)-isomere Cycloalkano[1,2-b]indol-sulfonamide der Formel

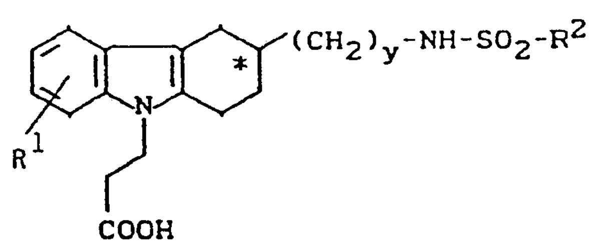

(XII)

in der

$R^1$ für Wasserstoff, fluor, Methyl, Methoxy, Benzyloxy oder Hydroxy steht,

$R^2$ für Phenyl steht, das durch fluor, Chlor, Trifluormethyl, Methyl, Propyl, Isopropyl oder Methoxy substituiert ist, und

y für die Zahl 0 oder 1 steht,
oder deren Salze.
Beispielhaft seien folgende Cycloalkano[1.2-b]indol-sulfonamide genannt :
1-(Benzolsulfonamidomethyl)-4-(2-carboxyethyl)cyclopentano[1.2-b]indol
4-(2-Carboxyethyl)-1-(4-fluorphenylsulfonamidomethyl)cyclopentano[1.2-b]indol
4-(2-Carboxyethyl)-1-(4-chlorphenylsulfonamidomethyl)cyclopentano[1.2-b]indol
1-(Benzolsulfonamidomethyl)-4-(2-carboxyethyl)-7-methoxycyclopentano[1.2-b]indol
4-(2-Carboxyethyl)-1-(4-fluorphenylsulfonamidomethyl)-7-methoxy-cyclopentano[1.2-b]indol
4-(2-Carboxyethyl)-1-(4-chlorphenylsulfonamidomethyl)-7-methoxy-cyclopentano(1.2-b]indol
1-(Benzolsulfonamido)-4-(2-carboxyethyl)cyclopentano-[1.2-b]indol
4-(2-Carboxyethyl)-1-(4-fluorphenylsulfonamido)cyclopentano[1.2-b]indol
4-(2-Carboxyethyl)-1-(4-chlorphenylsulfonamido)cyclopentano[1.2-b]indol
1-(Benzolsulfonamido)-4-(2-carboxyethyl)-7-methoxy-cyclopentano[1.2-b]indol
4-(2-Carboxyethyl)-1-(4-fluorphenylsulfonamido)-7-methoxycyclopentano[1.2-b]indol
4-(2-Carboxyethyl)-1-(4-chlorphenylsulfonamido)-7-methoxycyclopentano[1.2-b]indol
1-(Benzolsulfonamidomethyl)-4-(2-carboxyethyl)-7-methylcyclopentano[1.2-b]indol
4-(2-Carboxyethyl)-1-(4-fluorphenylsulfonamidomethyl)-7-methyl-cyclopentano[1.2-b]indol
4-(2-Carboxyethyl)-1-(4-chlorphenylsulfonamidomethyl)-7-methyl-cyclopentano[1.2-b]indol
1-(Benzolsulfonamido)-4-(2-carboxyethyl)-7-methyl-cyclopentano[1.2-b]indol
4-(2-Carboxyethyl)-1-(4-fluorphenylsulfonamido)-7-methylcyclopentano[1.2-b]indol
4-(2-Carboxyethyl)-1-(4-chlorphanylsulfonamido)-7-methylcyclopentano[1.2-b]indol
4-(2-Carboxyethyl)-1-(4-tolylsulfonamidomethyl)-cyclopentano[1.2-b]indol
4-(2-Carboxyethyl)-1-(4-tolylsulfonamido)-cyclopentano[1.2-b]indol
3-r-(Benzolsulfonamido)-9-(2-carboxyethyl)-6-methoxy-1,2,3,4,4a-t,9a-t-hexahydrocarbazol
3-r-(Benzolsulfonamido)-9-(2-carboxyethyl)-6-methoxy-1,2,3,4,4a-c,9a-c-hexahydrocarbazol
3-r-(Benzolsulfonamidomethyl)-9-(2-carboxyethyl)-1,2,3,4,4a-t,9a-t-hexahydrocarbazol
3-r-(Benzolsulfonamidomethyl)-9-(2-carboxyethyl)-1,2,3,4,4a-c,9a-c-hexahydrocarbazol
3-r-(Benzolsulfonamidomethyl)-9-(2-carboxyethyl)-6-methoxy-1,2,3,4,4a-t,9a-t-hexahydrocarbazol
3-r-(Benzolsulfonamidomethyl)-9-(2-carboxyethyl)-6-methoxy-1,2,3,4,4a-c,9a-c-hexahydrocarbazol
9-(2-Carboxyethyl)-3-r-(4-chlorphenylsulfonamido)-1,2,3,4,4a-t,9a-t-hexahydrocarbazol
9-(2-Carboxyethyl)-3-r-(4-chlorphenylsulfonamido)-1,2,3,4,4a-c,9a-c-hexahydrocarbazol
9-(2-Carboxyethyl)-3-r-(4-fluorphenylsulfonamido)-1,2,3,4,4a-t,9a-t-hexahydrocarbazol
9-(2-Carboxyethyl)-3-r-(4-fluorphenylsulfonamido)-1,2,3,4,4a-c,9a-c-hexahydrocarbazol
9-(2-Carboxyethyl)-3-r-(4-tolylsulfonamido)-1,2,3,4,4a-t,9a-t-hexahydrocarbazol
9-(2-Carboxyethyl)-3-r-(4-tolylsulfonamido)-1,2,3,4,4a-c,9a-c-hexahydrocarbazol
9-(2-Carboxyethyl)-3-r-(4-fluorphenylsulfonamido)-6-methoxy-1,2,3,4,4a-t,9a-t-hexahydrocarbazol
9-(2-Carboxyethyl)-3-r-(4-fluorphenylsulfonamido)-6-methoxy-1,2,3,4,4a-c,9a-c-hexahydrocarbazol
(+)-3(-4-Chlorphenylsulfonamido)-9-(2-carboxyethyl)-1,2,3,4-tetrahydrocarbazol,
(+)-3-(4-Fluorphenylsulfonamido)-9-(2-carboxyethyl)-1,2,3,4-tetrahydrocarbazol,
(−)-3-(4-Chlorphenylsulfonamido)-9-(2-carboxyethyl)-1,2,3,4-tetrahydrocarbazol,
(−)-3-(4-Fluorphenylsulfonamido)-9-(2-carboxyethyl)-1,2,3,4-tetrahydrocarbazol
(±)-3-(4-Chlorphenylsulfonamido)-9-(2-carboxyethyl)-1,2,3,4-tetrahydrocarbazol
(±)-3-(4-Fluorphenylsulfonamido)-9-(2-carboxyethyl)-1,2,3,4-tetrahydrocarbazol

Insbesondere bevorzugt werden :

(+)-3-(4-Fluorphenyl-sulfonamido)-9-(2-carboxyethyl)-1,2,3,4-tetrahydrocarbazol und
(−)-3-(4-Fluorphenyl-sulfonamido)-9-(2-carboxyethyl)-1,2,3,4-tetrahydrocarbazol
Weiterhin wurde ein Verfahren zur Herstellung der erfindungsgemäßen Cycloalkano[1,2-b]indol-sulfonamide und deren Salze gefunden, das dadurch gekennzeichnet ist, daß man [Benzolsulfonamidoalkyl]cycloalkano[1.2-b]indole der allgemeinen Formel (XIII)

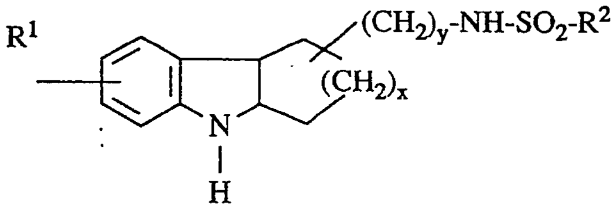

(XIII)

in welcher

$R^1$, $R^2$, y und x die oben angegebene Bedeutung haben, mit Acrylnitril in Gegenwart eines inerten Lösungsmittels, gegebenenfalls in Gegenwart einer Base, umsetzt,

dann die N,N'-Biscyanoethylverbindungen verseift,

dann im Fall der Herstellung der Cycloalkano[1.2-b]dihydroindol-sulfonamide die Cycloalkano[1.2-b]indol-sulfonamide gegebenenfalls in Gegenwart eines inerten Lösungsmittels in Gegenwart einer Säure und eines Reduktionsmittels hydriert,

gegebenenfalls die Isomeren in üblicher Weise trennt,

und dann gegebenenfalls im Fall der Herstellung der Salze mit einer entsprechenden Base umsetzt.

Das erfindungsgemäße Verfahren kann durch das folgende Formelschema erläutert werden :

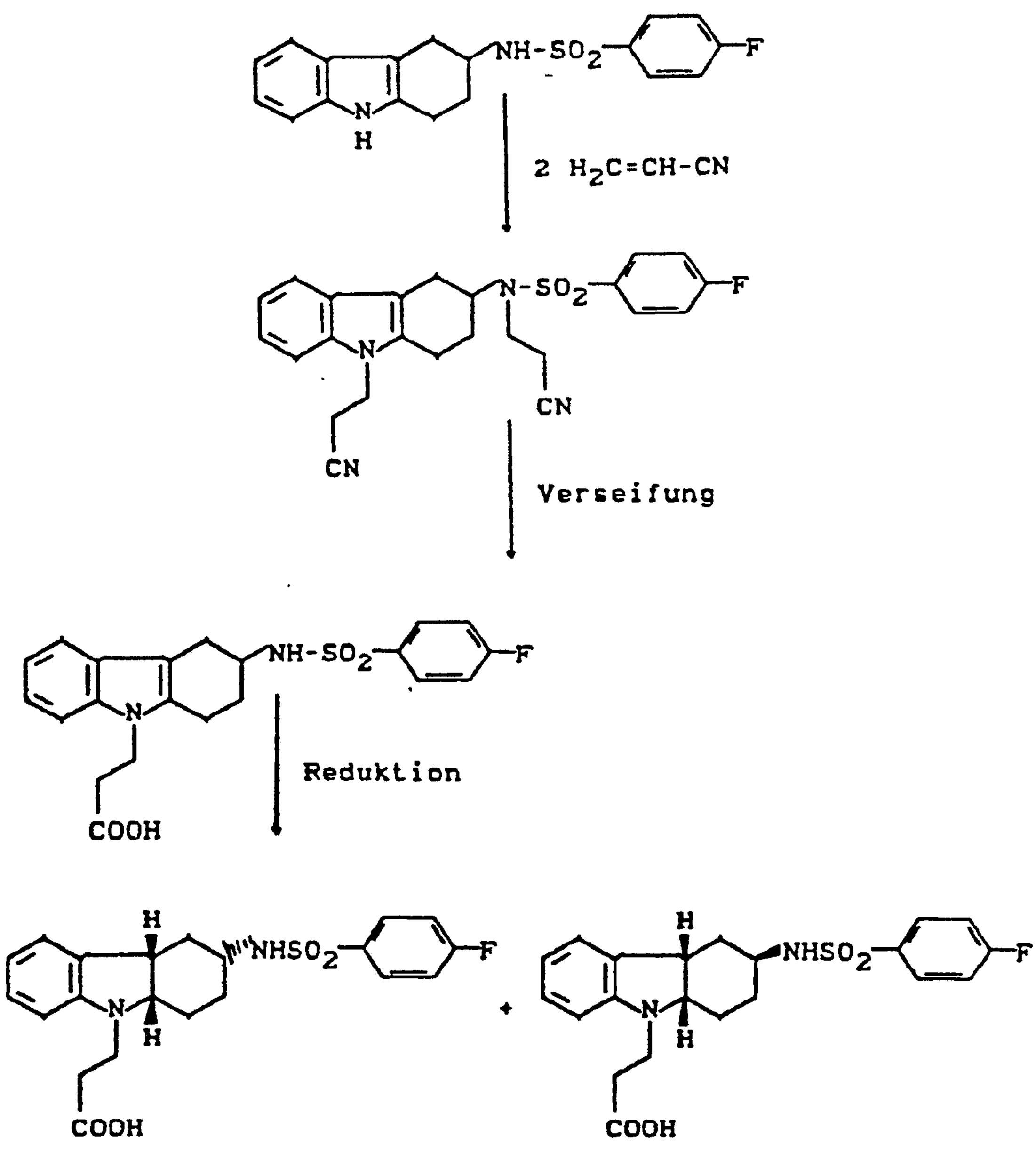

Die Cycloalkano[1.2-b]dihydroindol-sulfonamide im Rahmen der Formel (I) entsprechen der Formel (Ia)

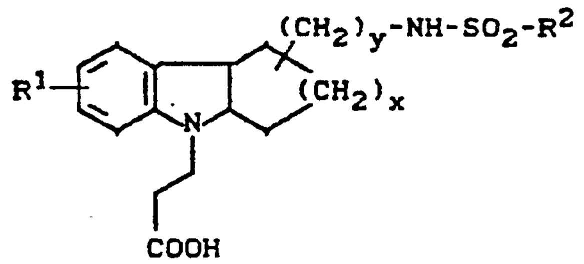

(Ia)

in der

$R^1$, $R^2$, x und y die obengenannte Bedeutung haben.

Bei der Durchführung des erfindungsgemäßen Verfahrens entstehen im allgemeinen Zwischenprodukte, die isoliert werden können. So ist es möglich, das erfindungsgemäße Verfahren in mehreren Verfahrensstufen auszuführen. Es kann aber auch möglich sein, verschiedene Verfahrensschritte zu kombinieren.

Lösungsmittel für das erfindungsgemäße Verfahren können Wasser und organische Lösungsmittel sein, die sich unter den Reaktionsbedingungen nicht verändern. Hierzu gehören bevorzugt Alkohole wie Methanol, Ethanol, Propanol oder Isopropanol, Ether wie Diethylether, Tetrahydrofuran, Dioxan, Glykolmono- oder -dimethylether, Kohlenwasserstoffe wie Benzol, Toluol, Xylol, Cyclohexan, Hexan oder Erdölfraktionen, Dimethylsulfoxid, Dimethylformamid, Hexamethylphosphorsäuretriamid, Essigester, Acetonitril oder Pyridin. Ebenso ist es möglich, Gemische der genannten Lösungsmittel zu verwenden.

Basen für das erfindungsgemäße Verfahren können übliche basische Verbindungen sein. Hierzu gehören vorzugsweise Alkali- und Erdalkalihydroxide wie Lithiumhydroxid, Alkalihydride wie Natriumcarbonat, Kaliumcarbonat, oder Alkalialkololate wie beispielsweise Natriummethanolat oder -ethanolat, Kaliummethanolat-oder -ethanolat oder Kaliumtert.-butylat oder Amide wie Natriumamid oder Lithiumdiisopropylamid, oder organische Amine wie Benzyltrimethylammoniumhydroxid, Tetrabutylammoniumhydroxid, Pyridin, Triethylamin oder N-Methylpiperidin.

Das erfindungsgemäße Verfahren wird im allgemeinen in einem Temperaturbereich von 0°C bis 150°C, bevorzugt von 20°C bis 100°C, durchgeführt.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen setzt man 1 bis 20 Mol, bevorzugt 1 bis 10 Mol Acrylnitril bezogen auf 1 Mol (Benzolsulfonamidoalkyl)cycloalkano[1.2-]indol ein.

Die Verseifung der N,N'-Bis-cyanoethylverbindungen erfolgt in an sich bekannter Weise in Gegenwart von Basen, wie Alkali- oder Erdalkalihydroxiden oder -alkanolaten, in inerten Lösungsmitteln wie Wasser oder Alkoholen. Bevorzugt werden als Basen natrium-, Kalium- oder Bariumhydroxid, Natriummethanolat, Kaliummethanolat, Natriumethanolat oder Kaliumethanolat, bevorzugt in Wasser oder Methanol, Ethanol, Propanol oder Isopropanol oder in Gemischen dieser Lösungsmittel eingesetzt.

Im allgemeinen setzt man 1 bis 100 Mol, bevorzugt 2 bis 50 Mol Base, bezogen auf 1 Mol N,N'-Biscyanoethylverbindung ein.

Die Verseifung wird in einem Temperaturbereich von 0°C bis 100°C, bevorzugt von 20°C bis 80°C durchgeführt.

Die Hydrierung wird in an sich bekannter Weise durchgeführt. Es ist hierbei möglich, die eingesetzte Säure als Lösungsmittel zu verwenden.

Als Lösungsmittel für die Hydrierung kommen inerte organische Lösungsmittel in Frage, die sich unter den Reaktionsbedingungen nicht verändern. Hierzu gehören bevorzugt Ether wie Diethylether, Dioxan oder Tetrahydrofuran, oder Eisessig, Trifluoressigsäure, Methansulfonsäure oder Trifluormethansulfonsäure.

Als Säuren für alle erfindungsgemäßen Verfahrensschritte können organische Säuren eingesetzt werden. Hierzu gehören vorzugsweise Carbonsäuren wie zum Beispiel Essigsäure, Propionsäure, Chloressigsäure, Dichloressigsäure oder Trifluoressigsäure, oder Sulfonsäuren wie zum Beispiel Methansulfonsäure, Ethansulfonsäure, Toluolsulfonsäure oder Benzolsulfonsäure oder Trifluormethansulfonsäure.

Als Reduktionsmittel für die erfindungsgemäße Hydrierung kommen die üblichen Reduktionsmittel in Frage. Hierzu gehören vorzugsweise Hydride wie zum Beispiel Natriumborhydrid, Natriumcyanoborhydrid, Tetrabutylammoniumborhydrid, Tetrabutylammoniumcyanoborhydrid, Tributylzinnhydrid, Triethylsilan, Dimethylphenylsilan oder Triphenylsilan.

Die Hydrierung erfolgt im allgemeinen in einem Temperaturbereich von −40°C bis +80°C, bevorzugt von −20°C bis +60°C.

Die eingesetzten (Benzolsulfonamidoalkyl)cycloalkano[1.2-b]indole der allgemeinen Formel XIII sind neu. Es wurde ebenfalls ein Verfahren zur Herstellung der (Benzosulfonamidoalkyl)cycloalkano/1.2-b/indole gefunden, das dadurch gekennzeichnet ist, daß man Phenylhydrazine der allgemeinen Formel (XIV)

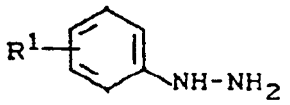

(XIV),

in welcher

$R^1$ die oben angegebene Bedeutung hat,

mit Cycloalkanonsulfonamiden der allgemeinen Formel (XV)

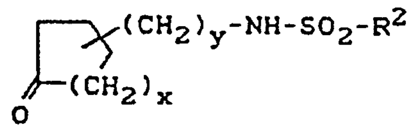

(XV)

in welcher

$R^2$, x und y die oben angegebene Bedeutung haben, in Gegenwart von inerten Lösungsmitteln, gegebenenfalls in Anwesenheit eines Katalysators umsetzt.

Die Herstellung der erfindungsgemäßen (Benzolsulfonamidoalkyl)cycloalkano/12-b/indole kann durch folgendes Formelschema erläutert werden :

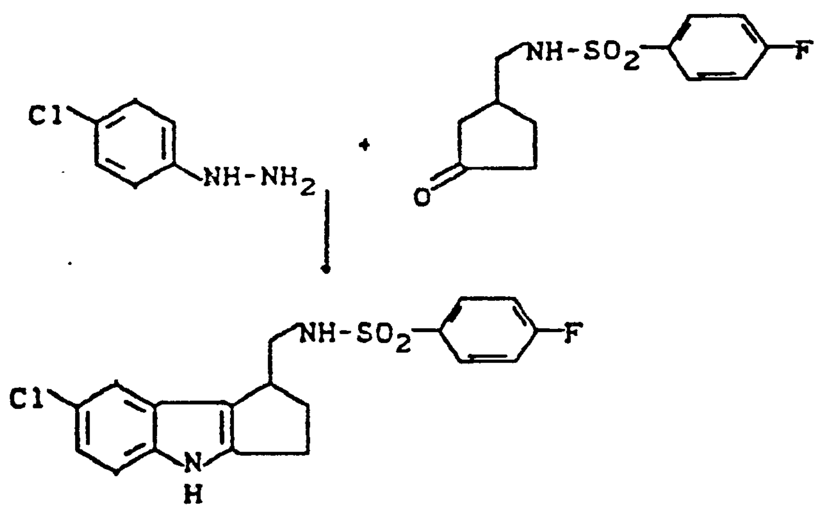

Lösungsmittel für das erfindungsgemäße Verfahren können hier inerte organische Lösungsmittel sein, die sich unter den Reaktionsbedingungen nicht verändern. Hierzu gehören bevorzugt Alkohole, wie beispielsweise Methanol, Ethanol, n-Propanol, iso-Propanol, Glykol, Ether wie beispielsweise Diethylether, Dioxan, Tetrahydrofuran, Glykolmono- oder -dimethylether, Halogenkohlenwasserstoffe wie Di-, Tri-oder Tetrachlormethan, Dichlorethylen, Trichlorethylen, Essigester, Toluol, Acetonitril, Eisessig, Hexamethylphosphorsäuretriamid, Pyridin und Aceton. Selbstverständlich ist es möglich, Gemische der Lösungsmittel einzusetzen.

Als Katalysatoren für das erfindungsgemäße Verfahren eignen sich die üblichen Säuren bzw. Lewis-Säuren. Hierzu gehören bevorzugt anorganische Säuren wie Salzsäure, Bromwasserstoffsäure, Schwefelsäure oder organische Säuren wie Carbonsäuren oder Sulfonsäuren z.B. Essigsäure, Methansulfonsäure, Toluolsulfonsäure, oder Lewis-Säuren wie z.B. Zinkchlorid, Zinkbromid oder Bortrifluoridetherat.

Das erfindungsgemäße Verfahren wird im allgemeinen in einem Temperaturbereich von 0°C bis 200°C, bevorzugt von 20°C bis 150°C durchgeführt.

Das erfindungsgemäße Verfahren wird im allgemeinen bei Normaldruck durchgeführt. Ebenso ist es möglich bei Überoder Unterdruck zu arbeiten (z.B. von 0.5 bis 5 bar).

Im allgemeinen wird das Hydrazin in einer Menge von 1 bis 3 Mol, bevorzugt 1 bis 1,5 Mol, bezogen auf das Keton, eingesetzt.

Als Hydrazine für das erfindungsgemäße Verfahren werden beispielsweise eingesetzt : Phenylhydrazin, 4-Methoxyphenylhydrazin, 4-Chlorphenylhydrazin, 4-Fluorphenylhydrazin, 4-Methylphenylhydrazin.

Als Ketone werden beispielsweise erfindungsgemäß verwendet :

3-(Benzolsulfonamidomethyl)cyclopentanon
3-(Benzolsulfonamidomethyl)cyclohexanon
4-(Benzolsulfonamidomethyl)cyclohexanon
3-(Benzolsulfonamido)cyclopentanon
3-(Benzolsulfonamido)cyclohexanon
4-(Benzolsulfonamido)cyclohexanon
3-(4-Chlorphenylsulfonamidomethyl)cyclopentanon
3-(4-Fluorphenylsulfonamidomethyl)cyclopentanon
3-(4-Methylphenylsulfonamidomethyl)cyclopentanon
3-(4-chlorphenylsulfonamidomethyl)cyclohexanon
3-(4-Fluorphenylsulfonamidomethyl)cyclohexanon
3-(4-Methylphenylsulfonamidomethyl)cyclohexanon
4-(4-Chlorphenylsulfonamidomethyl)cyclohexanon
4-(4-Fluorphenylsulfonamidomethyl)cyclohexanon
4-(Methylphenylsulfonamidomethyl)cyclohexanon
3-(4-Chlorphenylsulfonamido)cyclopentanon
3-(4-Fluorphenylsulfonamido)cyclopentanon
3-(4-Methylphenylsulfonamido)cyclopentanon
3-(4-Chlorphenylsulfonamido)cyclohexanon
3-(4-Fluorphenylsulfonamido)cyclohexanon
3-(4-Methylphenylsulfonamido)cyclohexanon
4-(4-Chlorphenylsulfonamido)cyclohexanon
4-(4-Fluorphenylsulfonamido)cyclohexanon
4-(4-Methylphenylsulfonamido)cyclohexanon

Die als Ausgangsstoffe verwendeten Hydrazine XIV sind bekannt oder können nach bekannten Methoden hergestellt werden (vgl. Houben-Weyl, "Methoden der organischen Chemie", X/2, Seite 1, 123, 693).

Die als Ausgangsstoffe verwendeten Cyclohexanonsulfonamide der allgemeinen Formel (XVa)

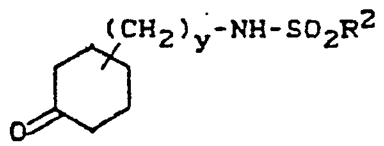

(XVa),

in welcher

y und $R^2$ die oben angegebene Bedeutung haben, sind zum Teil bekannt und können nach an sich bekannten Methoden hergestellt werden (vgl. Houben-Weyl "Methoden der organischen Chemie", IX. 605 ; A. Mooradian et al., J. Med. Chem. 20 (4), 487 (1977)).

Die als Ausgangsstoffe eingesetzten Cyclopentanonsulfonamide der allgemeinen Formel (XVb)

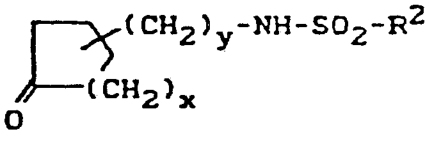

(XVb)

in welcher

x für die Zahl 1 steht und

y und $R^2$ die oben angegebene Bedeutung haben, sind neu.

Es wurde auch ein Verfahren zur Herstellung der neuen Cycloalkanonsulfonamide gefunden, das dadurch gekennzeichnet ist, daß man Cycloalkanole der allgemeinen Formel (XVI)

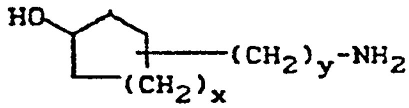 (XVI),

in welcher

x und y die oben angegebene Bedeutung haben, mit Sulfonsäurehalogeniden der allgemeinen Formel (XVII)

$$Hal-SO_2-R^2 \quad (XVII),$$

in welcher

$R^2$ die oben angegebene Bedeutung hat und

Hal für Fluor, Chlor, Brom, Iod, bevorzugt für Chlor oder Brom, steht,

in inerten organischen Lösungsmitteln gegebenenfalls in Anwesenheit von Basen umsetzt,

und anschließend in inerten Lösungsmitteln oxidiert.

Die Cycloalkanole können hergestellt werden, indem man Cycloalkenone (XVIII)

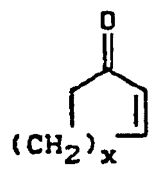 (XVIII),

in inerten organischen Lösungsmitteln, gegebenenfalls in Anwesenheit von Basen mit Nitromethan umsetzt und anschließend die Verbindungen (XIX)

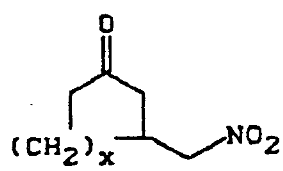 (XIX),

reduziert (CA 92, 89 849 und CA 87, 22 191).

Die Sulfonsäurehalogenide können nach an sich bekannten Methoden hergestellt werden (Houben-Weyl's "Methoden der organischen Chemie" IX, 564).

Die Herstellung der erfindungsgemäßen Cycloalkano[1.2-b] indol-sulfonamide kann durch das folgende Reaktionsschema erläutert werden :

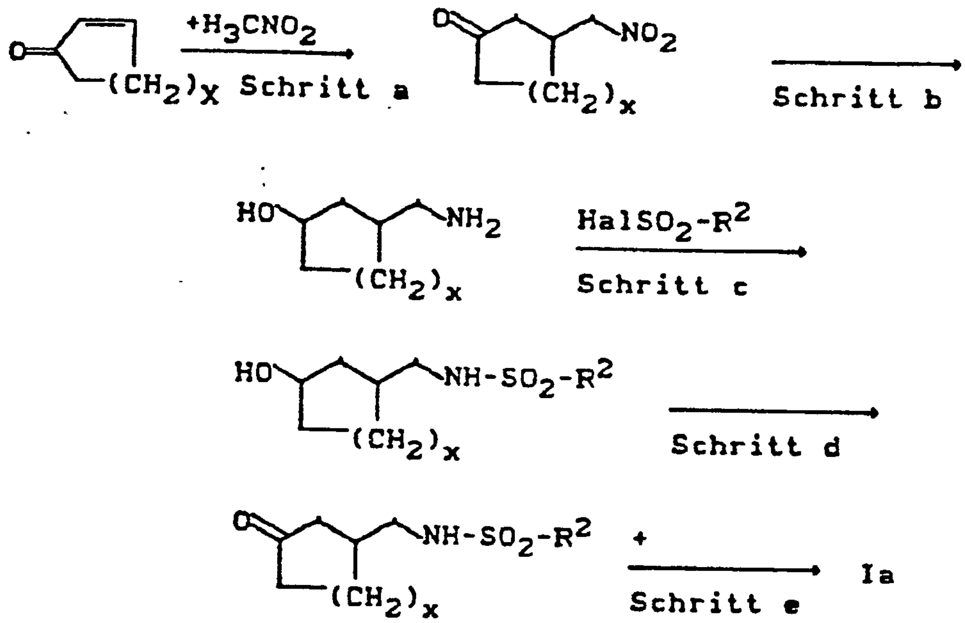


Danach werden im ersten Schritt a) Cycloalkenone in inerten Lösungsmitteln wie Alkoholen z.B. Methanol, Ethanol, Propanol oder Ethern, z.B. Diethylether, Tetrahydrofuran oder Dioxan, oder Chlorkohlenwasserstoffen, z.B. Methylenchlorid, Chloroform oder Tetrachlorkohlenstoff, in Anwesenheit von Basen wie beispielsweise Natriumhydrid, Natrium- oder Kaliummethanolat, Natrium- oder Kaliumethanolat, Kalium-tert.-butanolat, 1,5-Diazabicyclo[4.3.0]non-5-en, 1,5-Diazabicyclo[5.4.0]undec-5-en, Pyridin oder Triethylamin mit Nitroverbindungen wie Nitromethan, bei Temperaturen im Bereich von 0°C bis 100°C zu Nitroverbindungen umsetzt.

In Schritt b) werden die nitroverbindungen in inerten Lösungsmitteln wie Ether, z.B. Tetrahydrofuran, Dioxan oder Diethylether, in Gegenwart eines Reduktionsmittels wie Hydriden, z.B. $LiAlH_4$, $Na[Al(OCH_2CH_2OCH_3)_2H_2]$ oder Di-iso-butyl-aluminium-hydrid bei Temperaturen im Bereich von −20°C bis +60°C zu Cycloalkanolen reduziert.

In Schritt c) werden die Cycloalkanole mit Sulfonsäurehalogeniden in inerten Lösungsmitteln wie Ethern z.B. Dioxan, Tetrahydrofuran oder Diethylether oder Chlorkohlenwasserstoffen wie Methylenchlorid, Chloroform oder Tetrachlorkohlenstoff oder Essigester oder Pyridin, gegebenenfalls in Anwesenheit von Basen,wie 1,5-Diazabicyclo [4.3.0]non-5-en, 1,5-Diazabicyclo[5.4.0]undec-5-en, Pyridin oder Triethylamin bei Temperaturen von −20°C bis +60°C in Sulfonamide überführt.

Im Schritt d) werden die Sulfonamide in inerten Lösungsmitteln wie Wasser, Eisessig, Aceton, Pyridin oder Gemische derselben mit Oxidationsmitteln wie Chrom(VI)Verbindungen, z.B. $CrO_3$, $K_2Cr_2O_7$, $Na_2Cr_2O_7$, bei Temperaturen von −20°C bis +100°C zu Cycloalkanonsulfonamiden oxidiert.

In Schritt e) werden Cycloalkanonsulfonamide (XVb) und Hydrazine XIV wie vorne beschrieben, zu den entsprechenden (Benzolsulfonamidoalkyl)cycloalkano[1.2-b]indolen der Formel XIII

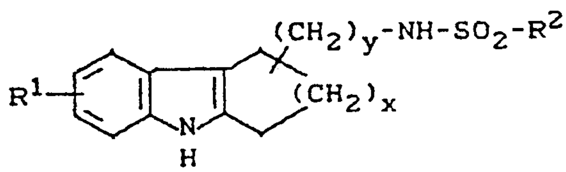 (XIII),

in der

$R^1$, $R^2$, x und y die oben genannte Bedeutung haben, umgesetzt.

Die enantiomerenreinen (Benzolsulfonamido)cyclohexano[1,2-b]indole der allgemeinen Formel (XIIIa)

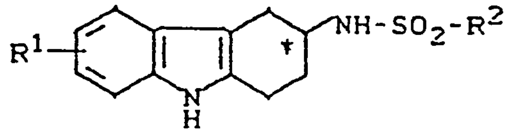 (XIIIa),

in welchen

$R^1$ und $R^2$ die angegebene Bedeutung haben, sind ebenfalls neu.

Es wurde ein Verfahren zur Herstellung der enantiomerenreinen (Benzolsulfonamido)-cyclohexano[1,2-b]indole gefunden, das dadurch gekennzeichnet ist, daß man enantiomerenreine Cyclohexano[1,2-b]indol-amine der allgemeinen Formel (XX)

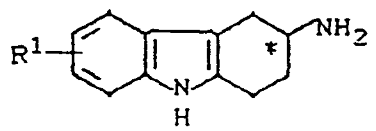

(XX),

in welcher

$R^1$ die angegebene Bedeutung hat,

mit Sulfonsäurehalogeniden der allgemeinen Formel (XVII)

$$Hal-SO_2-R^2 \qquad (XVII),$$

in welcher

$R^2$ die angegebene Bedeutung hat und

Hal für Fluor, Chlor, Brom, Iod, bevorzugt für Chlor oder Brom steht,

in Gegenwart inerter Lösemittel, gegebenenfalls in Gegenwart einer Base umsetzt.

Als Lösemittel für das Verfahren eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan, oder Erdölfraktionen, oder Halogenkohlenwasserstoffe wie Dichlormethan, Trichlormethan, Tetrachlormethan, Dichlorethylen, Trichlorethylen, oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel einzusetzen.

Basen für das Verfahren können übliche basische Verbindungen sein. Hierzu gehören vorzugsweise Alkali- oder Erdalkalihydroxide wie Lithiumhydroxid, Natriumhydroxid Kaliumhydroxid oder Bariumhydroxid, oder Alkalihydride wie Natriumhydrid, oder Alkali- oder Erdalkalicarbonate wie Natriumcarbonat, Natriumhydrogencarbonat, Kaliumcarbonat oder Calciumcarbonat, oder Alkalialkoholate wie beispielsweise Natriummethanolat, Natriumethanolat, Kaliummethanolat, Kaliumethanolat oder Kalium-tert-butylat, oder Alkaliamide wie Lithiumdiisopropylamid, oder Natriumamid, oder organische Amine wie Ethyldiisopropylamin, Benzyltrimethylammoniumhydroxid, Tetrabutylammoniumhydroxid, Pyridin, Dimethylaminopyridin, Triethylamin, N-Methylpiperidin, 1,5-Diazabicyclo[4,3,0]-non-5-en oder 1,5-Diazabicyclo[5,4,0]undec-5-en.

Das erfindungsgemäße Verfahren wird im allgemeinen in einem Temperaturbereich von −30°C bis +150°C, bevorzugt von −20°C bis +80°C durchgeführt.

Das erfindungsgemäße Verfahren wird im allgemeinen bei Normaldruck durchgeführt. Es ist ebenso möglich, bei unterdruck oder bei Überdruck zu arbeiten (z.B. in einem Bereich von 0,5 bis 200 bar).

Die erfindungsgemäße enantiomerenreinen Cyclohexano[1,2-b]indol-amine der allgemeinen Formel (XX) sind neu und können nach folgenden Syntheseprinzipien A, B oder C hergestellt werden.

Syntheseprinzip A

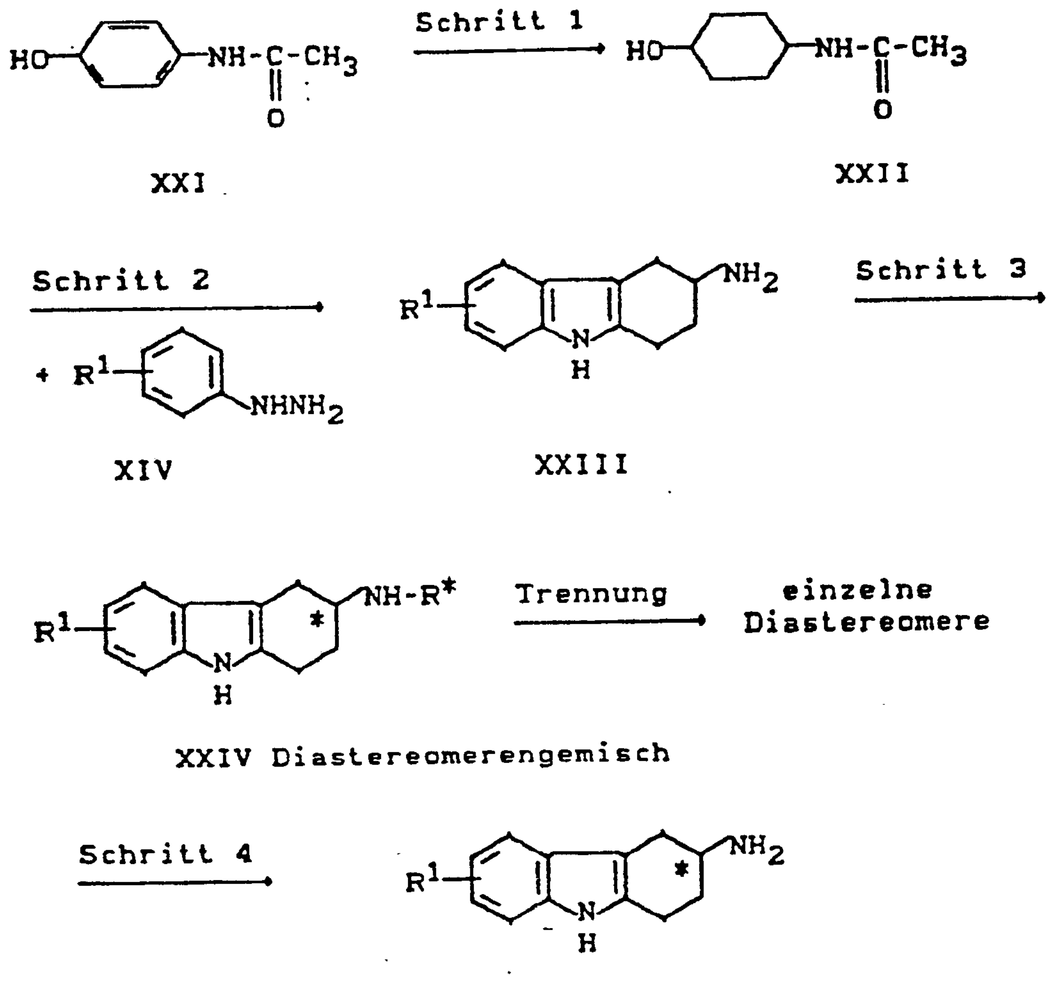


XXI

XXII

XIV

XXIII

XXIV Diastereomerengemisch

XX

$R^1$ hat die angegebene Bedeutung und

$R^*$ steht für einen enantiomerenreinen D-, oder L- Aminosäurerest, bevorzugt für den 2S-(Chloracetamido)-3-phenylpropionylrest

Syntheseprinzip B

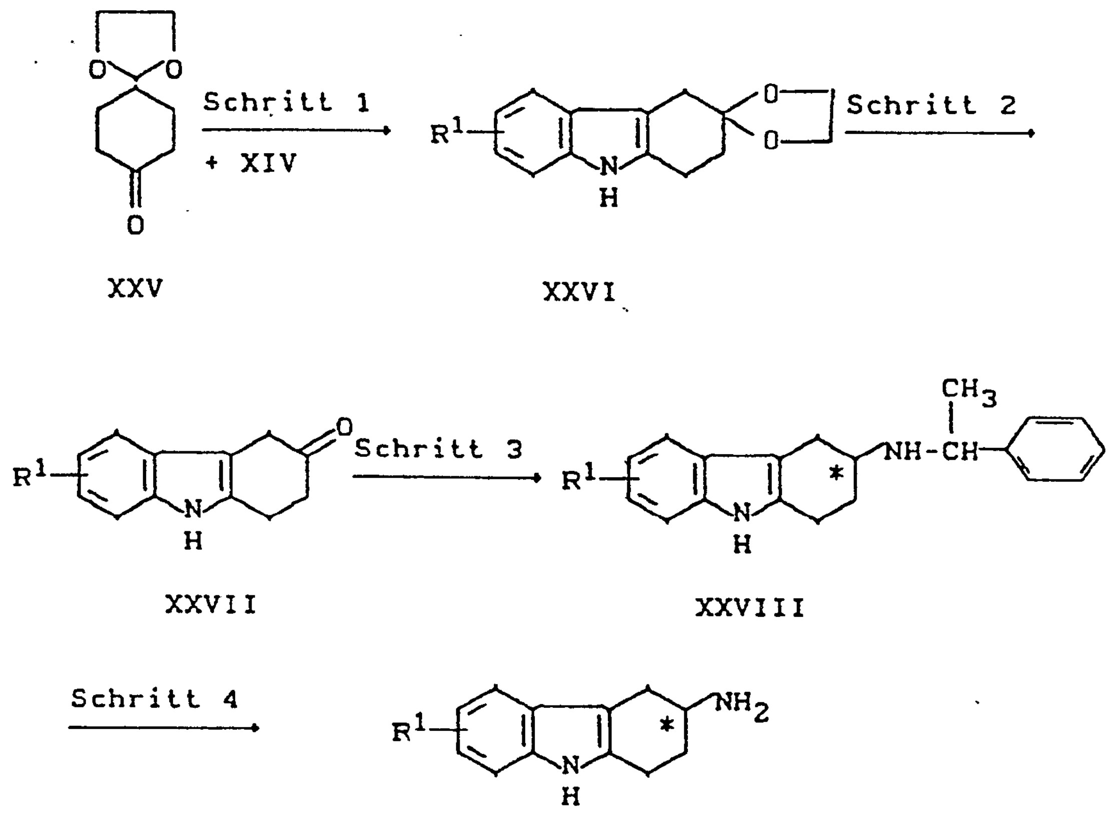


XXV XXVI XXVII XXVIII

XX

$R^1$ hat die angegebene Bedeutung.

Syntheseprinzip A

Hiernach wird entsprechend Schritt 1 Paracetamol (XXI) an Raney-Nickel zu einem cis/trans-Gemisch von 4-Acetamidocyclohexanol (XXII) hydriert wie es von Billman, J.H., Bühler, J.A., in J. Am. Chem. Soc. 75, 1345 (1953) beschrieben wird. In Schritt 2 wird 4-Acetamidocyclohexanol (XXII) in einem Eintopfverfahren nach einer Oxidation z.B. mit $CrO_3$

Phenylhydrazinen (XIV) einer Fischerschen Indol-Synthese unterzogen und dann die Acetylgruppe sauer abhydrolysiert.

Dieser Verfahrensschritt wird in Lösemitteln wie Wasser, Essigsäure und/oder Propionsäure, bei Temperaturen von 0°C bis +150°C, bevorzugt von 0°C bis 110°C durchgeführt. Die auf diese Art gut zugänglichen racemischen 3-Amino-1,2,3,4-tetrahydrocarbazole (XXIII) werden in Schritt 3 durch Kupplung mit enantiomerenreinen Aminosäuren, gegebenenfalls in ihrer aktivierten Form in die entsprechenden Diastereomerengemische überführt, die durch übliche Methoden wie Kristallisation oder Säulenchromatographie in die einzelnen Diastereomere getrennt werden können.

Als enantiomerenreine Aminosäurederivate eignen sich bevorzugt Acetylphenylalanin, N-tert-Butoxycarbonylphenylalanin, Chloracetylphenylalanin, Carbobenzoxyphenylalanin, Methoxy-phenylessigsäure oder Acetoxyphenylessigsäure, bevorzugt N-Chloracetyl- N-phenylalanin.

Als Aktivierungsmittel werden im allgemeinen die üblichen Peptidkupplungsreagentien verwendet. Hierzu gehören bevorzugt Carbodiimide wie beispielsweise Diisopropylcarbodiimid, Dicyclohexylcarbodiimid, oder N-(3-Dimethylaminoisopropyl)-N'-ethylcarbodiimid Hydrochlorid, oder Carbonylverbindungen wie Carbonyldiimidazol, oder 1,2-Oxazoliumverbindungen wie 2-Ethyl-5-phenyl-1,2-oxazolium-3-sulfonat, oder Propanphosphonsäureanhydrid, oder Isobutylchloroformat, oder Benzotriazolyloxy-tris-(dimethylamino)-phosphonium-hexafluorophosphat, oder Methansulfonsäurechlorid, gegebenenfalls in Anwesenheit von Basen wie Triethylamin, oder N-Ethylmorpholin oder N-Methylpiperidin, oder Dicyclohexylcarbodiimid und N-Hydroxysuccinimid.

Die Kupplung wird im allgemeinen in inerten organischen Lösemitteln, bevorzugt in Chlorkohlenwasserstoffen wie Methylenchlorid oder Chloroform, oder Kohlenwasserstoffen wie Benzol, Toluol, Xylol oder Erdölfraktionen, oder in Ethern wie Dioxan, Tetrahydrofuran oder Diethylether, oder in Essigester, Dimethylformamid, Dimethylsulfoxid, Aceton, Acetonitril oder Nitromethan bei Temperaturen von −80°C bis +50°C, bevorzugt von −40°C bis +30°C durchgeführt.

Nach Trennung der Diastereomerengemische (XXIV) werden in Schritt 4 die einzelnen Diastereomeren sauer zu den enantiomerenreinen Aminen (XX) hydrolysiert.

Die Hydrolyse erfolgt im allgemeinen mit anorganischen oder organischen Säuren wie Salzsäure, Bromwasserstoffsäure, Schwefelsäure, Phosphorsäure, Ameisensäure, Essigsäure, Propionsäure, Methansulfonsäure oder Trifluoressigsäure, oder Gemischen der genannten Säuren.

Als Lösemittel wird im allgemeinen hierbei Wasser oder die wäßrige Lösung der entsprechenden eingesetzten Säure oder des Säuregemisches verwendet.

Die Hydrolyse wird im allgemeinen in einem Temperaturbereich von +20°C bis +150°C, bevorzugt von +20°C bis +120°C durchgeführt.

Das erfindungsgemäße Verfahren wird im allgemeinen bei Normaldruck durchgeführt. Es ist ebenso möglich bei erniedrigtem oder bei erhöhtem Druck, z.B. in einem Autoklaven oder Bombenrohr durchzuführen. Es hat sich hierbei als vorteilhaft erwiesen, Thioglykolsäure als Oxidationsinhibitor zur Reaktionsmischung zuzusetzen.

Syntheseprinzip B

Hiernach wird 1,4-Cyclohexandion-monoethylenketal (XXV) mit Phenylhydrazinen (XIV) in einer Fischerschen Indol-Synthese zu Ketalen (XXVI) wie es durch A. Britten und G. Lockwood in J. Chem. Soc., Perkin Trans. 1, 1974, 1824-1827 beschrieben wird, umgesetzt.

Hydrolyse der Ketale (XXVI) in Schritt 2 ergibt die Ketone (XXVII), die in Schritt 3 durch reduktive Aminierung mit S-Phenethylamin in die Diastereomerengemische (XXVIII) übergeführt werden.

Die reduktive Aminierung erfolgt im allgemeinen mit Reduktionsmitteln wie Wasserstoff, gegebenenfalls in Anwesenheit von Palladium, Platin oder Palladium auf Tierkohle als Katalysator, oder komplexen Hydriden, bevorzugt Natriumborhydrid, Kaliumborhydrid, Lithiumborhydrid, Zinkborhydrid, Lithiumaluminiumborhydrid, Aluminiumhydrid ; Di-iso-butyl-aluminium-hydrid, Lithiumtriethylhydridoborat, Natriumcyanotrihydridoborat, Tetrabutylammoiniumcyanotrihydridoborat, Tetrabutylammoniumhydridoborat, Lithiumaluminiumhydrid, Natrium-bis-[2-methoxyethoxy]dihydridoaluminat oder Lithiumhydrido-tris[1-methylpropyl)borat in inerten Lösemitteln wie Kohlenwasserstoffe, bevorzugt Benzol, Toluol oder Xylol, oder Chlorkohlenwasserstoffen wie Methylenchlorid oder Chloroform, oder Ethern wie Diethylether, Tetrahydrofuran, Dioxan oder 1,2-Dimethoxymethan, oder Acetonitril, Dimethylformamid, Dimethylsulfoxid, oder Alkohlen wie Methanol, Ethanol, Propanol oder Isopropanol, in einem Temperaturbereich von −80°C bis +100°C, bevorzugt von −80°C bis +50°C.

Das Diastereomerengemisch (XXVIII) wird nach üblichen Methoden wie Chromatographie oder Kristallisation, bevorzugt durch Kristallisation, gegebenenfalls in Form geeigneter Säureadditionsprodukte, in die einzelnen Diasteromeren getrennt.

Geeignete Säureadditionsprodukte sind hierbei Additionsprodukte der erfindungsgemäßen Enantiomeren mit anorganischen oder organischen Säuren. Hierzu gehören bevorzugt Salzsäure, Schwefelsäure, Phosphorsäure oder Methansulfonsäure, Benzolsulfonsäure, Naphthalindisulfonsäure, oder Essigsäure, Maleinsäure, Fumarsäure, Citronensäure oder Milchsäure.

Die Entfernung der Phenylethylgruppen in den getrennten Diastereomeren (XXVIII) erfolgt in Schritt 4 durch katalytische Transferhydrierung zu den enantiomerenreinen Aminen (XX).

Schritt 4 wird im allgemeinen mit Reduktionsmitteln wie Wasserstoff, gegebenenfalls in Anwesenheit von Palladium, Palladium auf Tierkohle oder Platin, oder Ammoniumformiat in Wasser oder organischen Lösemitteln wie Alkohole z.B. Methanol, Ethanol, Propanol oder Isopropanol, oder Dimethylformamid oder Dimethylsulfoxid oder Mischungen dieser Lösemittel, in einem Temperaturbereich von 0°C bis +200°C, bevorzugt von +20°C bis +150°C durchgeführt (L.E. Overman und S. Sugai, J. Org. Chem. 50, 4154-4155 (1985)).

Syntheseprinzip C

Hiernach wird eine Racematspaltung der Verbindungen (XXIII) durch Salzbildung mit optisch aktiven Säuren und ein- oder mehrfacher Kristallisation dieser Salze aus geeigneten Lösemitteln vorgenommen. Die enantiomerenreinen Verbindungen (XXIII) werden aus den so gewonnenen Salzen durch Behandeln mit Base freigesetzt.

Als optisch aktive Säuren eignen sich : (+)-Camphersulfonsäure, (−)-Camphersulfonsäure, (+)-Campher-3-carbonsäure, (–)-Campher-3-carbonsäure, (+)-Camphersäure, (–)-Camphersäure, (+)-Äpfelsäure, (–)-Äpfelsäure, (+)-Mandelsäure, (–)-Mandelsäure, (+)-Milchsäure, (–)-Milchsäure, (+)-2-/(Phenylamino)carbonyloxy/propionsäure, (–)-2-/(Phenylamino)carbonyloxy/propionsäure, (–)-α-Methoxyphenylessigsäure, (–)-Di-O-Benzoylweinsäure, (–)-Di-O-4-Toluoylweinsäure, (–)-Methoxyessigsäure, (–)-1,1'-Binaphthyl-2,2'-diylhydrogenphosphat.

Als Lösemittel für die Kristallisation eignen sich Lösemittel wie Wasser, Alkohole, wie Methanol, Ethanol, Isopropanol, n-Propanol, n-Butanol, sec-Butanol, tert-Butanol, Ether wie Diethylether, Tetrahydrofuran, Dioxan, Glykoldimethylether, Ketone wie Aceton, Methylethylketon, Methylisobutylketon, Kohlenwasserstoffe wie Benzol, Toluol, Xylol, Hexan, Cyclohexan, Chlorkohlenwasserstoffe wie Dichlormethan, Chloroform, oder Essigester, Acetonitril, Nitromethan, Dimethylsulfoxid, Dimethylformamid, Sulfolan, Ebenso ist es möglich, Gemische der genannten Lösemittel zu verwenden.

Basen für das Verfahren können die üblichen basischen Verbindungen sein. Hierzu gehören vorzugsweise Alkalioder Erdalkalihydroxide wie Lithiumhydroxid, Natriumhydroxid, Kaliumhydroxid oder Bariumhydroxid, oder Alkalihydride wie Natriumhydrid, oder Alkali- oder Erdalkalicarbonate wie Natriumcarbonat, Natriumhydrogencarbonat, Kaliumcarbonat oder Calciumcarbonat, oder Alkalialkoholate wie beispielsweise Natriummethanolat, Natriumethanolat, Kaliummethanolat, Kaliumethanolat oder Kalium-tert-butylat.

Die neuen Cycloalkano[1.2-b]indol-sulfonamide bzw. deren Salze können als Wirkstoffe in Arzneimitteln eingesetzt werden. Die Wirkstoffe weisen eine thrombozytenaggregationshemmende und Thromboxan $A_2$-antagonistische Wirkung auf. Sie können bevorzugt zur Behandlung von Thrombosen, Thromboembolien, Ischämien, als Antiasthmatika und als Antiallergika eingesetzt werden. Die neuen Wirkstoffe können in an sich bekannter Weise unter Verwendung inerter nichttoxischer, pharmazeutisch geeigneter Trägerstoffe oder Lösungsmittel in die üblichen formulierungen überführt werden, wie Tabletten, Kapseln, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.-%, bevorzugt von 5 bis 70 Gew.-% vorliegen, die ausreichend ist, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nichttoxische organische Lösungsmittel wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäureester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation kann in üblicher Weise erfolgen, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen. Bei oraler Applikation beträgt die Dosierung im allgemeinen etwa 0,01 bis 20 mg/kg, vorzugsweise 0,1 bis 10 mg/kg Körpergewicht.

Es kann aber gegebenenfalls vorteilhaft sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehrere Einzelgaben über den Tag zu verteilen.

Die erfindungsgemäßen Cycloalkano[1.2-b]indol-sulfonamide können sowohl in der Humanmedizin als auch in der Veterinärmedizin angewendet werden.

Herstellungsbeispiele

Beispiel 1

3-(Nitromethyl)cyclopentanon

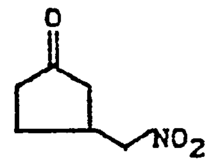

100 g 2-Cyclopentenon werden zusammen mit 666 ml Nitromethan und 5 g 1.5-Diazabicyclo[4.3.0]non-5-en (DBN) in 1.1 l Isopropanol gelöst und 5 h bei Raumtemperatur stehen gelassen. Das Isopropanol wird dann weitgehend im Vakuum destilliert, der Rückstand in Essigester gelöst und 2 mal mit je 0,5 l verdünnter Schwefelsäure gewaschen. Die organische Phase wird mit Natriumsulfat getrocknet und eingedampft. Man erhält so 154 g (88% der Theorie) 3-(Nitromethyl)cyclopentanon, das für die nächste Umsetzung rein genug ist.

$R_f$ = 0.52 $CH_2Cl_2 : CH_3OH$ = 99 : 1

Beispiel 2

3-(Aminomethyl)cyclopentanol

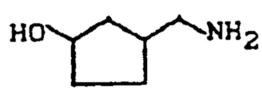

57,2 g (0,4 Mol) 3-(Nitromethyl)cyclopentanon werden unter Stickstoff in 573 ml absolutem Tetrahydrofuran gelöst. Bei 0°C werden zu dieser Lösung 800 ml einer einmolaren Lösung von Lithiumaluminiumhydrid in Tetrahydrofuran getropft. Nach Beendigung des Zutropfens wird 1 h bei 0°C nachgerührt. Dann wird das Kühlbad entfernt, die Temperatur der Reaktionslösung steigt daraufhin bis auf 40°C an. Nach Abfallen der Temperatur auf 20°C wird noch 1 h bei dieser Temperatur nachgerührt. Zu der auf 0°C gekühlen Reaktionsmischung werden dann vorsichtig 100 ml 45%ige Natronlauge zugetropft. Nach beendigung des Zutropfens wird noch 1 h bei Raumtemperatur nachgerührt, die Reaktionsmischung über Kieselgur filtriert und das Kieselgur mit 1,5 l Tetrahydrofuran nachgewaschen. Die vereinigten Filtrate werden im Vakuum gründlich eingedampft. Man erhält so 22,5 g (49% der Theorie) zähes, öliges Produkt.

$R_f$ = 0.01 $CH_2Cl_2 : CH_3OH$ = 9 : 1

Beispiel 3

3-(Benzolsulfonamidomethyl)cyclopentanol

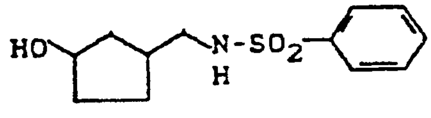

9 g (0,078 Mol) 3-(Aminomethyl)cyclopentanol werden in 200 ml Tetrahydrofuran zusammen mit 13,8 g = 10 ml (0,078 Mol) Triethylamin gelöst. Bei 0-5°C werden dann 7,9 g = 10,8 ml (0,078 Mol) Benzolsulfonsäurechlorid zugetropft. Nach Beendigung des Zutropfens wird 1 h bei 0°C nachgerührt. Die Reaktionsmischung wird dann mit 200 ml Methylenchlorid verdünnt und 2 mal mit je 150 ml verdünnter Schwefelsäure gewaschen. Die organische Phase wird dann 2 mal mit je 150 ml 2 n Natronlauge extrahiert, die vereinigten Extrakte mit konzentrierter Salzsäure angesäuert und 2 mal mit je 150 ml Methylenchlorid extrahiert. Die vereinigten Methylenchloridphasen werden mit Natriumsulfat getrocknet und im Vakuum eingedampft. Man erhält so 9,1 g (39%

der Theorie) zähes, öliges Isomerengemisch als Produkt.

$R_f$ = 0.51 und 0.45 $CH_2Cl_2$ : $CH_3OH$ = 95 : 5

Beispiel 4

3-(Benzolsulfonamidomethyl)cyclopentanon

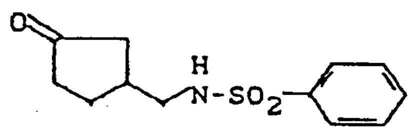

7,5 g (0,0294 Mol) 3-(Benzolsulfonamidomethyl)cyclopentanol werden in 60 ml Eisessig gelöst. Bei 0-5°C werden 2,79 g (0,0279 Mol) Chromtrioxid in 2 ml Wasser und 8,8 ml Eisessig gelöst zugetropft, dann läßt man die Temperatur der Reaktionsmischung auf Raumtemperatur ansteigen. Nach 1 h Rühren bei Raumtemperatur wird die Reaktionsmischung mit 200 ml Ether verdünnt und 2 mal mit je 150 ml Wasser gewaschen. Die organische Phase wird dann 2 mal mit je 200 ml

2 n Natronlauge extrahiert, die vereinigten Natronlaugephasen mit konzentrierter Salzsäure sauer gestellt und 2 mal mit je 200 ml Methylenchlorid extrahiert. Die vereinigten Methylenchloridphasen werden mit Natriumsulfat getrocknet und im Vakuum eingedampft. Man erhält so 4,4 g (59% der Theorie) zähes, öliges Produkt.

$R_f$ = 0,51 $CH_2Cl_2$ : $CH_3OH$ = 95 : 5

Beispiel 5

1-(Benzolsulfonamidomethyl)cyclopentano[1.2-b]indol

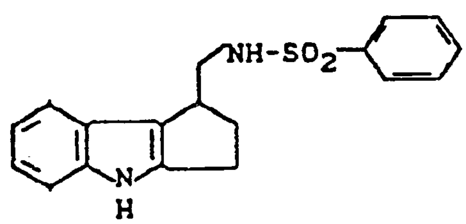

21 g (0,0826 Mol) 3-(Benzolsulfonamidomethyl)cyclopentanon werden zusammen mit 9 g (0,0826 Mol) Phenylhydrazin in 200 ml Eisessig gelöst und 4 h unter Rückfluß erhitzt. Dann wird die Reaktionslösung mit 1,3 l Ether verdünnt und mit 500 ml Wasser versetzt. Unter Kühlung und Rühren wird mit 45%iger Natronlauge alkalisch gestellt, dann die organische Phase abgetrennt. Die wäßrige Phase wird noch einmal mit 500 ml Ether extrahiert, die vereinigten organischen Phasen mit Natriumsulfat getrocknet und eingedampf. Der so erhaltene Rückstand wird an 2 kg Kieselgel (Merck 0,04-0,063 mm) mit einem Gemisch von Toluol und Essigester im Verhältnis 85 zu 15 chromatographiert. Man erhält so eine Fraktion, die nach dem Eindampfen 1,9 g (7% der Theorie) kristallines Produkt ergibt, Fp. : 161-164°C.

$R_f$ = 0,92 $CH_2Cl_2$ : $CH_3OH$ = 95 : 5

Beispiel 6

1-[N-(Benzolsulfonyl)-N-(2-cyanoethyl)aminomethyl]-4-(2-cyanoethyl)cyclopentano[1.2-b]indol

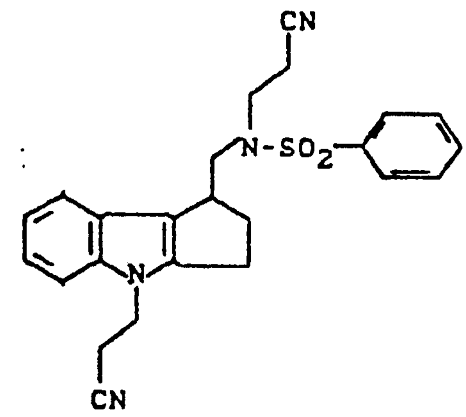

1,9 g (0,0058 Mol) 1-(Benzolsulfonamidomethyl)cyclopentano[1.2-b]indol werden zusammen mit 1,83 g = 2,3 ml (0,0346 Mol) Acrylnitril und 0,24 g (0,00058 Mol) einer 40%igen Lösung von Benzyltrimethylammoniumhydroxid in Methanol in 60 ml Dioxan 2 h bei 60-70°C gerührt. Dann wird die Reaktionsmischung im Vakuum eingedampft, der Rückstand in Methylenchlorid aufgenommen und mit verdünnter Schwefeltäure 2 mal extrahiert. Die organische Phase wird mit ges. Bicarbonatlösung gewaschen, mit Natriumsulfat getrocknet und eingedampft. Man erhält so 2,4 g (95% dur Theorie) Produkt alt festen Schaum.

$R_f$ = 0,45 $CH_2Cl_2$ : $CH_3OH$ = 99 : 1

Beispiel 7

1-(Benzolsulfonamidomethyl)-4-(2-carboxyethyl)cyclopentano[1.2-b]indol

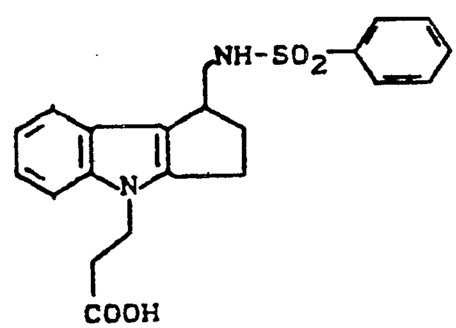

2,4 g (0,0055 Mol) 1-[N-(Benzolsulfonyl)-N-(2-cyanoethyl)-aminomethyl]-4-(2-cyanoethyl)cyclopentano[1.2-b]indol werden in 35 ml Isopropanol gelöst und mit 55 ml 10%iger Kaliumhydroxidlösung versetzt. Die Reaktionsmischung wird 4 h bei 70°C gerührt, dann mit 100 ml Wasser verdünnt und mit 100 ml Methylenchlorid extrahiert. Die wäßrige Phase wird mit verdünnter Schwetelsäure angesäuert und 3 mal mit je 100 ml Methylenchlorid extrahiert. Die vereinigten organischen Phasen werden mit Natriumsulfat getrocknet und eingedampft. Der ölige Rückstand (1,9 g) wird in Methanol gelöst und mit 0,26 g Natriummethylat versetzt. Eindampfen dieser Lösung liefert 2,0 g (69,2% der Theorie) des Produktes als mikrokristallines Hatriumsalz.

$R_f$ = 0,37 $CH_2Cl_2$ : $CH_3OH$ = 95 : 5

Beispiel 8

3-(4-Fluorphenylsulfonamidomethyl)cyclopentanol

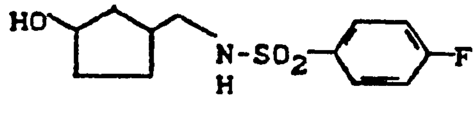

Analog der Vorschrift für Beispiel 3 werden 19,8 g (0,172 Mol) 3-(Aminomethyl)cyclopentanol mit 28,3 g

(0,172 Mol) 4-Fluorphenylsulfonamid umgesetzt. Es werden dabei 17,3 g (36% der Theorie) zähes, öliges Isomerengemisch als Produkt erhalten.

$R_f$ = 0,53 und 0,46 $CH_2Cl_2$ : $CH_3OH$ = 95 : 5

Beispiel 9

3-(4-Fluorphenylsulfonamidomethyl)cyclopentanon

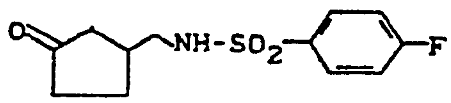

Analog der Vorschrift für Beispiel 4 wurden 17,3 g (0,0638 Mol) 3-(4-Fluorphenylsulfonamidomathyl)cyclopentanol oxidiert. Man erhält dabei 14,3 g (83% der Theorie) zähes, öliges Produkt.

$R_f$ = 0,76 $CH_2Cl_2$ : $CH_3OH$ = 9 : 1

Bespiel 10

1-(4-Fluorphenylsulfonamidomethyl)cyclopentano[1.2-b]-indol

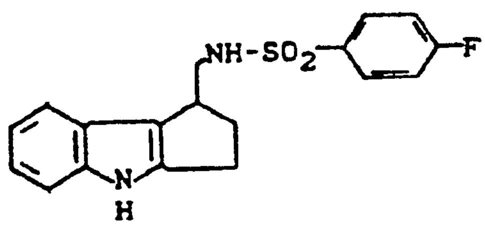

14,3 g (0,0527 Mol) 3-(4-Fluorphanylsulfonamidomethyl)-cyclopentanon wurden analog zu Beispiel 5 mit Phenylhydrazin umgesetzt. Man erhält so nach Chromatographie an Kieselgel 0,67 g (3,7% der Theorie) mikrokristallines Produkt.

$R_f$ = 0,47 $CH_2Cl_2$ : $CH_3OH$ = 99 : 1

Beispiel 11

4-(2-Cyanoethyl)-1-[N-(4-fluorphenylsulfonyl)-N-(2-cyanoethyl)aminomethyl]cyclopentano[1.2-b]indol

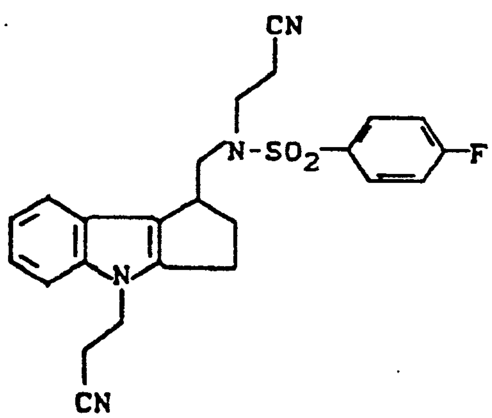

0,67 g (0,00195 Mol) 1-(4-Fluorphenylsulfonamidomethyl)-cyclopentano[1.2-b]indol wurden analog zu Beispiel 6 umgesetzt. Man erhält so 0,83 g (95% der Theorie) Produkt als festen Schaum.

$R_f$ = 0,39 Toluol : Essigester = 8 : 2

Beispiel 12

4-(2-Carboxyethyl)-1-(4-fluorphenylsulfonamidomethyl)-cyclopentano[1.2-b]indol

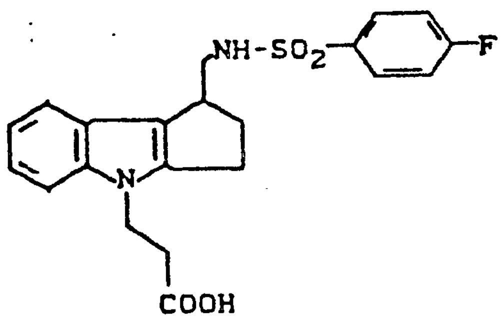

0,83 g (0,00184 Mol) 4-(2-Cyanoethyl)-1-[N-(4-Fluorphenylsulfonyl)-N-(2-cyanoethyl)aminomethyl]cyclopentano[1.2-b]-indol werden analog Beispiel 7 hydrolysiert. Man erhält so 0,67 g (87% der Theorie) kristallines Produkt als Natriumsalz, Fp. : 150-160°C.

$R_f$ = 0,59 $CH_2Cl_2$ : $CH_3OH$ = 9 : 1

Beispiel 13

3-(4-Chlorphenylsulfonamidomethyl)cyclopentanol

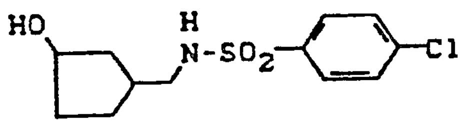

16,8 g (0,146 Mol) 3-(Aminomethyl)cyclopentanol werden analog zu Beispiel 3 mit 4-Chlorphenylsulfonsäurechlorid umgesetzt. Man erhält so 16,6 g (39% der Theorie) zähes, öliges Produkt als Isomerengemisch.

$R_f$ = 0,46 und 0,44 $CH_2Cl_2$ : $CH_3OH$ = 95 : 5

Beispiel 14

3-(4-Chlorphenylsulfonamidomethyl)cyclopentanon

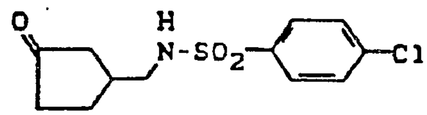

16,6 g (0,0573 Mol) 3-(4-Chlorphenylsulfonamidomethyl)cyclopentanol werden analog zu Beispiel 4 oxidiert. Man erhält so 13,8 g (83,7% der Theorie) zähes, öliges Produkt.

$R_f$ = 0,7 $CH_2Cl_2$ : $CH_3OH$ = 95 : 5

Beispiel 15

1-(4-Chlorphenysulfonamidomethyl)cyclopentano[1,2-b]indol

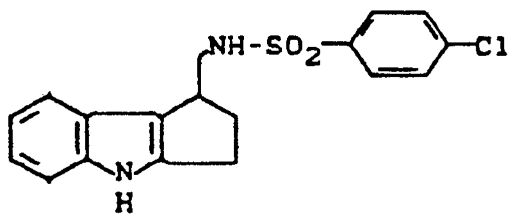

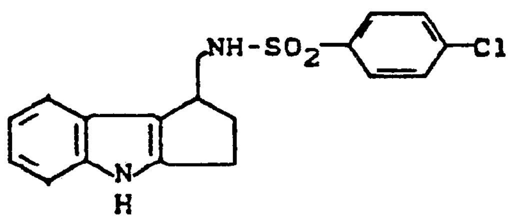

13,8 g (0,048 Mol) 3-(4-Chlorphenylsulfonamidomethyl)cyclopentanon werden mit Phenylhydrazin analog zu Beispiel 5 umgesetzt. Man erhält so nach Chromatographie an Kieselgel 1,65 g (9,5% der Theorie) Produkt als festen Schaum.

$R_f$ = 0,46 $CH_2Cl_2$ : $CH_3OH$ = 99 : 1

Beispiel 16

1-[N-(2-Chlorphenylsulfonyl)-N-(2-cyanoethyl)amino-methyl]-4-(2-cyanoethyl)cyclopentano[1.2-b]-indol

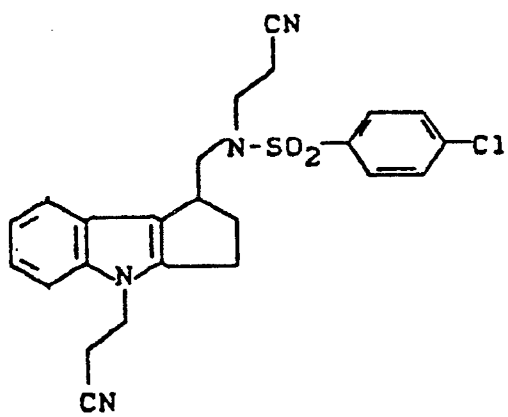

1,65 g (0,0046 Mol) 1-(4-Chlorphenylsulfonamidomethyl)-cyclopentano[1.2-b]indol werden analog zu Beispiel 6 umgesetzt. Man erhält so 1,8 g (84% der Theorie) Produkt als festen Schaum.

$R_f$ = 0,38 Toluol : Essigester = 8 : 2

Beispiel 17

4-(2-Carboxyethyl)-1-(4-Chlorphenylsulfonamidomethyl)-cyclopentano[1.2-b]indol

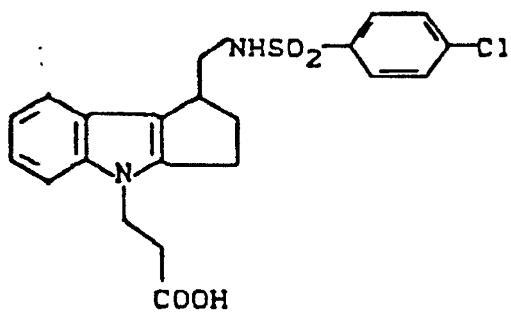

1,9 g (0,0038 Mol) 1-[N-(4-Chlorphenylsulfonyl)-N-(2-cyanoethyl)amidomethyl]-4-(2-cyanoethyl)cyclopentano[1.2-b]indol werden analog zu Beispiel 7 verseift. Man erhält so 1,33 g (81,3% der Theorie) Produkt als kristallines Natriumsalz, Fp. : 160°C.

$R_f$ = 0,55 $CH_2Cl_2$ : $CH_3OH$ = 9 : 1

Beispiel 18

4-(Benzolsulfonamido)cyclohexanol

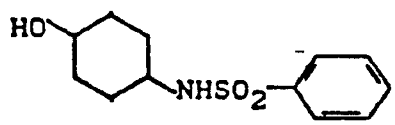

69 g (0,6 Mol) 4-Aminocyclohexanol werden mit 107 g (0,6 Mol) Benzolsulfonsäurechlorid analog zu Beispiel 3 umgesetzt. Man erhält so 72,8 g (47% der Theorie) kristallines Produkt, Fp. : 106-108°C.

$R_f$ = 0,38 $CH_2Cl_2$ : $CH_3OH$ = 95 : 5

Beispiel 19

4-(Benzolsulfonamido)cyclohexanon

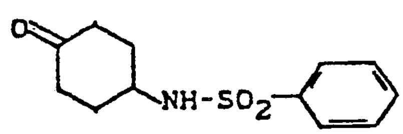

72,8 g (0,285 Mol) 4-(Benzolsulfonamido)cyclohexanol werden analog zu Beispiel 4 oxidiert. Man erhält nach Kristallisation aus Petrolether 57,5 g (80% der Theorie) Produkt, Fp. : 80-82°C.

$R_f$ = 0,66 $CH_2Cl_2$ : $CH_3OH$ = 95 : 5

Beispiel 20

3-(Benzolsulfonamido)-1.2.3.4-tetrahydrocarbazol

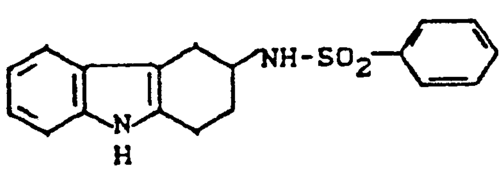

57,5 g (0,227 Mol) 4-(Benzolsulfonamido)cyclohexanon werden analog Beispiel 5 mit Phenylhydrazin umgesetzt. Man erhält so 41,5 g (56% der Theorie) aus Isopropanol kristallisiertes Produkt, Fp. : 155°C.

$R_f$ = 0,82 $CH_2Cl_2$ : $CH_3OH$ = 95 : 5

Beispiel 21

3-[N-(Benzolsulfonyl)-N-(2-cyanoethyl)amino]-9-(2-cyanoethyl)-1.2.3.4-tetrahydrocarbazol

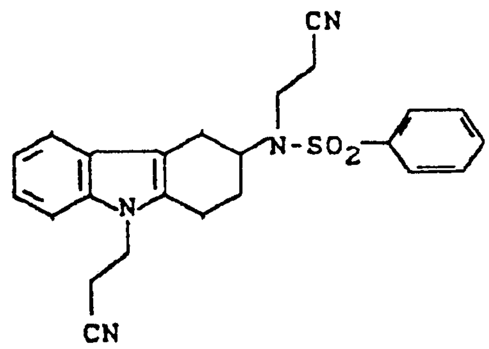

10 g (0,0306 Mol) 3-(Benzolsulfonamido)-1.2.3.4-tetrahydrocarbazol werden analog Beispiel 6 umgesetzt. Man erhält so 10 g (75% der Theorie) aus Ether kristallisiertes Produkt, Fp. : 180-190°C

$R_f$ = 0,29 Toluol : Essigester = 8 : 2

Beispiel 22

3-(Benzolsulfonamido)-9-(2-carboxyethyl)-1.2.3.4-tetrahydrocarbazol

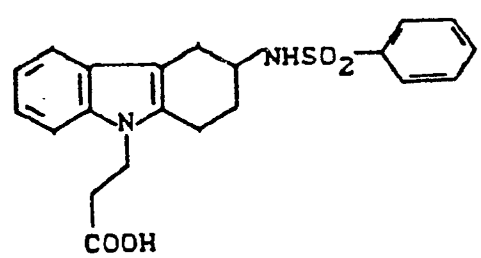

10 g (0,0263 Mol) 3-[N-(Benzolsulfonyl)-N-(2-cyanoethyl)-amino]-9-(2-cyanoethyl)-1.2.3.4-tetrahydrocarbazol werden analog zu Beispiel 7 verseift. Man erhält so 7,57 g (68% der Theorie) kristallines Produkt als Natriumsalz, Fp. : 160-165°C.

$R_f$ = 0,44 $CH_2Cl_2$ : $CH_3OH$ = 95 : 5

Analog zu Beispiel 18 wurden folgende in Tabelle 1 aufgeführten Verbindungen hergestellt :

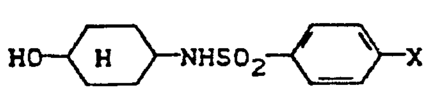

Tabelle 1

| Beispiel Nr. | X | Ausbeute | $R_f$-Wert | |
|---|---|---|---|---|
| 23 | Cl | 80 % | 0,37 | $CH_2Cl_2$ : $CH_3OH$ = 95:5 |
| 28 | F | 75 % | 0,4 | $CH_2Cl_2$ : $CH_3OH$ = 95:5 |
| 33 | $CH_3$ | 48,7 % | 0,5 | $CH_2Cl_2$ : $CH_3OH$ = 95:5 |

Analog zu Beispiel 19 wurden folgende in Tabelle 2 aufgeführten Verbindungen hergestellt:

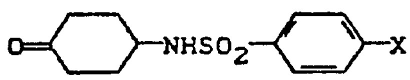

Tabelle 2

| Bsp.-Nr. | X | Ausbeute | $R_f$-Wert | | Fp.: |
|---|---|---|---|---|---|
| 24 | Cl | 86 % | 0,77 | $CH_2Cl_2$ : $CH_3OH$ = 9:1 | 103-4° C aus Petrolether |
| 29 | F | 94 % | 0,7 | $CH_2Cl_2$ : $CH_3OH$ = 9:1 | 104-8° C aus Petrolether |
| 34 | $CH_3$ | 90,7 % | 0,57 | $CH_2Cl_2$ : $CH_3OH$ = 95:5 | |

Analog zu Beispiel 20 wurden folgende in Tabelle 3 aufgeführten Verbindungen hergestellt:

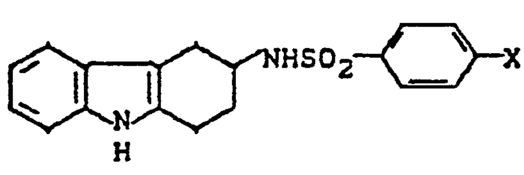

Tabelle 3

| Bsp.-Nr. | X | Ausbeute | $R_f$-Wert | Fp.: |
|---|---|---|---|---|
| 25 | Cl | 75,4 % | 0,52 Toluol:Essigester 8:2 | 163° C aus Ether |
| 30 | F | 73 % | 0,39 $CH_2Cl_2$:$CH_3OH$ = 99:1 | 146-9° C aus Ether |
| 35 | $CH_3$ | 55 % | 0,42 $CH_2Cl_2$:$CH_3OH$ = 8:2 | 136-8° C aus Isopropanol |

Analog Beispiel 21 wurden folgende in Tabelle 4 aufgeführten Verbindungen hergestellt :

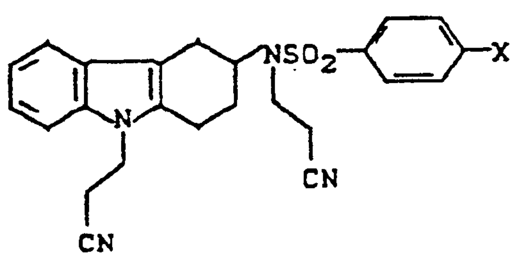

Tabelle 4

| Bsp. Nr. | X | Ausbeute | $R_f$-Wert | Fp.: |
|---|---|---|---|---|
| 26 | Cl | 47 % | 0,35 Toluol:Essigester 8:2 | 204-6° C aus Ether/ Isopropanol |
| 31 | F | 53 % | 0,29 Toluol:Essigester 8:2 | 206-8° C aus Ether/ Isopropanol |
| 36 | $CH_3$ | 85 % | 0,37 Toluol:Essigester 8:2 | 180-90° C aus Ether |

Analog zu Beispiel 22 wurden folgende in Tabelle 5 aufgeführte Verbindungen hergestellt :

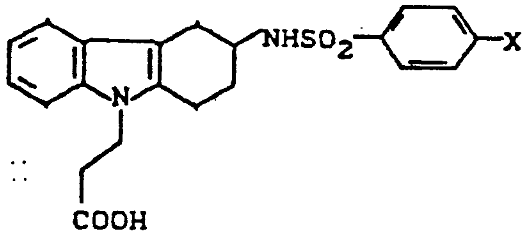

Tabelle 5

| Bsp. Nr. | X | Ausbeute | $R_f$-Wert | Fp.: |
|---|---|---|---|---|
| 27 | Cl | 89,5 % | 0,61 $CH_2Cl_2:CH_3OH$ = 9:1 | 150° C Na-Salz |
| 32 | F | 98,5 % | 0,57 $CH_2Cl_2:CH_3OH$ = 9:1 | 160-70° C Na-Salz |
| 37 | $CH_3$ | 95 % | 0,53 $CH_2Cl_2:CH_3OH$ = 9:1 | 150-60° C Na-Salz |

Beispiel 38 und 39

3-r-(4-Fluorphenylsulfonamido)-9-(2-carboxyethyl)-1,2,3,4,4a-t,9a-t-hexahydrocarbazol (Isomer A) und 3-r-(4-Fluorphenylsulfonamido)-9-(2-carboxyethyl)-1,2,3,4,4a-c,9a-c-hexahydrocarbazol (Isomer B)

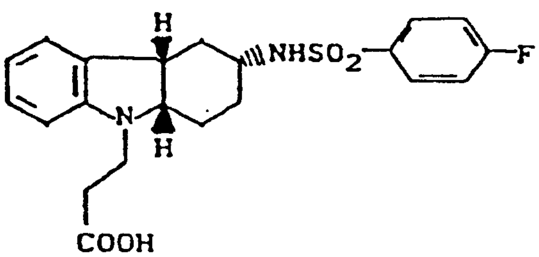

Isomer A

38

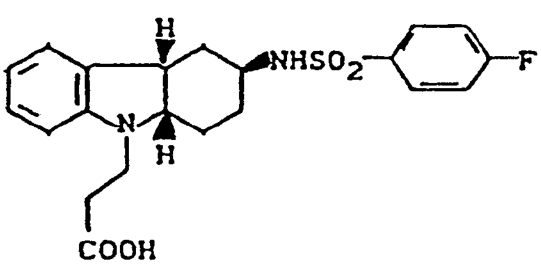

Isomer B

39

5 g (0,0114 Mol) 3-(4-Fluorphenylsulfonamido)-9-(2-carboxyethyl)-1.2.3.4-tetrahydrocarbazol-Natriumsalz werden in 50 ml Trifluoressigsäure gelöst und bei 0°C portionsweise mit 5,01 g (0,08 Mol) Natriumcyanoborhydrid versetzt. Man läßt die Reaktionsmischung auf Raumtemperatur kommen, verdünnt mit Wasser und extrahiert mit 200 ml Essigester. Die Essigesterphase wird 2 mal mit je 100 ml 2 n Natronlauge extrahiert, die vereinigten Natronlaugephasen auf pH = 5 gebracht und 3 mal mit je 150 ml Methylenchlorid extrahiert, mit Natriumsulfat getrocknet und im Vakuum gründlich eingedampft. Der Rückstand wird an 500 g Kieselgel (Merck, 0,040-0,063 mm) mit einem 100 zu 1-Gemisch von Methylenchlorid mit Eisessig chromatographiert. Man erhält so zwei Fraktionen, die nach dem Eindampfen 2,87 g

(60,2% der Theorie) Isomer (A) bzw. 0,7 g (14,9% der Theorie) Isomer (B) als festen Schaum liefern.

$R_f$ von Isomer (A) : 0,24

$CH_2Cl_2 : CH_3COOH = 100 : 2$

$R_f$ von Isomer (B) : 0,14

Beispiele 40 und 41

3-r-(Benzolsulfonamido)-9-(2-carboxyethyl)-1,2,3,4,4a-t,9a-t-hexahydrocorbazol (Isomer A) und 3-r-(Benzolsulfonamido)-9-(2-carboxyethyl)-1,2,3,4,4a-c,9a-c-hexahydrocarbazol (Isomer B)

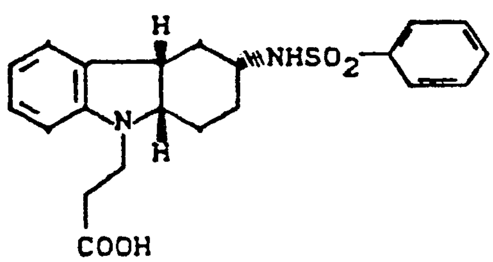

(Isomer A)

40

und

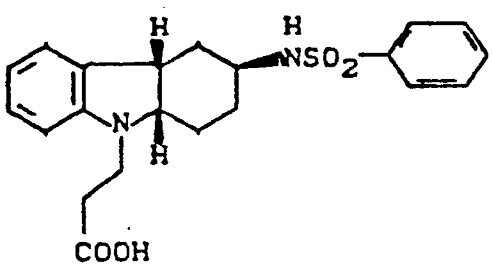

(Isomer B)

41

1,18 g (0,0028 Mol) 3-(Benzolsulfonamido)-9-(2-carboxymethyl)-1,2,3,4-tetrahydrocarbazol-Natriumsalz werden analog zu Beispiel 38 reduziert. Nach Chromatographie erhält man zwei Fraktionen, die nach dem Eindampfen 0,45 g (40% der Theorie) Isomer A und 0,2 g (18% der Theorie) Isomer B als feston Schaum ergeben.

$R_f$ von Isomer A : 0,4

$CH_2Cl_2 : CH_3COOH = 100 : 4$

$R_f$ von Isomer B : 0,22

Beispiel 42 und Beispiel 43

3-r-(4-Methylphenylsulfonamido)-9-(2-carboxyethyl)-1,2,3,4,4a-t,9a-t-hexahydrocarbazol (Isomer A)

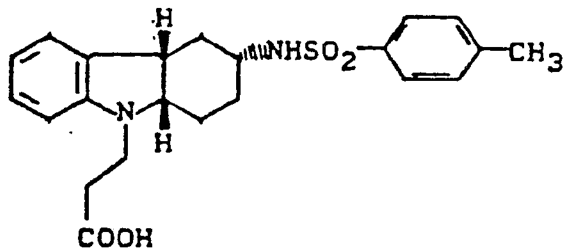

Isomer A

und

3-r-(4-Methylphenylsulfonylamido)-9-(2-carboxyethyl)-1,2,3,4,4a-c,9a-c,hexahydrocarbazol (Isomer B)

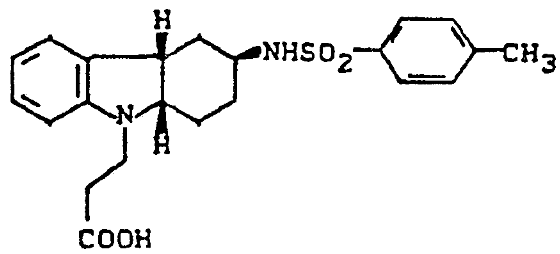

Isomer B

18,06 g 3-(4-Methylphenylsulfonamido)-9-(2-carboxyethyl)-1,2,3,4-tetrahydrocarbazol-Natriumsalz werden analog zu Beispiel 38 reduziert. Nach Chromatographie erhält man zwei Fraktionen, die nach dem Eindampfen 3,65 g (20% der Theorie) Isomer A als kristallinen Rückstand, Fp.: 156-62°C, und 1,11 g (6% der Theorie) Isomer B als festen Schaum ergeben.

$R_f$ von Isomer A : 0,39
$R_f$ von Isomer B : 0,20
$CH_2Cl_2 : CH_3COOH = 100 : 2$

Beispiel 44

3-(4-Chlorphenylsulfonamido)-6-fluor-1,2,3,4-tetrahydrocarbazol

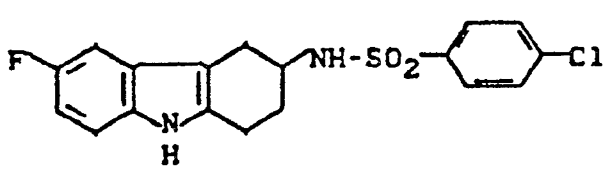

26,5 g 4-(4-Chlorphenylsulfonamido)cyclohexanon werden mit 4-Fluorphenylhydrazin analog zu Beispiel 5 umgesetzt. Man erhält dabei 35,4 g (100% der Theorie) Produkt als festen Schaum.

$R_f = 0,53$ Toluol : Essigester = 8 : 2

Beispiel 45

3-[N-(4-Chlorphenylsulfonyl)-N-(2-cyanoethyl)amino]-9-(2-cyanoethyl)-6-fluor-1,2,3,4-tetrahydrocarbazol

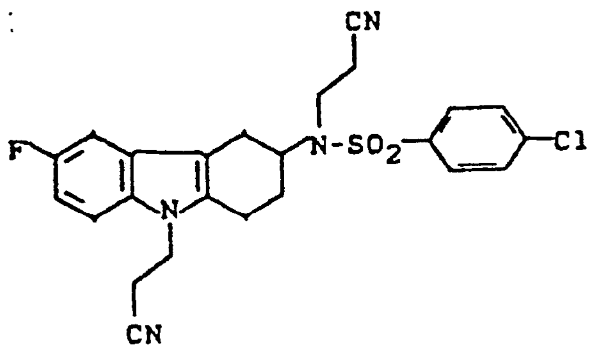

3,4 g 4-(4-Chlorphenylsulfonamido)-6-fluor-1,2,3,4-tetrahydrocarbazol werden analog Beispiel 6 umgesetzt. Man erhält so 27,6 g (61% der Theorie) Produkt als festen Schaum.

$R_f$ = 0,25 Toluol : Essigester = 8 : 2

Beispiel 46

3-(4-Chlorphenylsulfonamido)-9-(2-carboxyethyl)-6-fluor-1,2,3,4-tetrahydrocarbazol

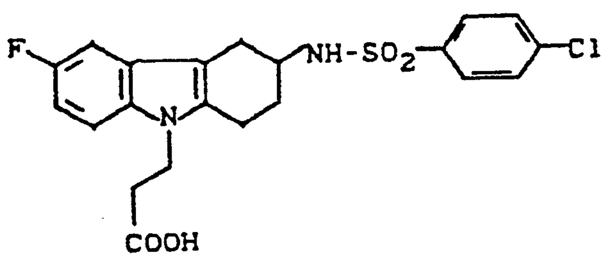

27,6 g 3-[N-(Chlorphenylsulfonyl)-N-(2-cyanoethyl)-amino]-9-(2-cyanoethyl)-6-fluor-1,2,3,4-tetrahydrocarbazol werden analog zu Beispiel 7 verseift. Man erhält so 25,6 g (100% der Theorie) kristallines Produkt, Fp.: 118-130°C.

$R_f$ = 0,52 $CH_2Cl_2$ : $CH_3OH$ = 9 : 1

Beispiel 47

3-(Nitromethyl)cyclohexanon

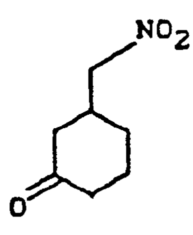

21,9 g Cyclohexanon werden zusammen mit 175 ml Nitromethan und 2,1 g 5-Diazobicyclo-[4,3,0]-non-5-en (DBN) in 250 ml Isopropanol 2 Tage bei Raumtemperatur stehen gelassen. Die Aufarbeitung erfolgt analog der Vorschrift für Beispiel 1 und ergibt 37,2 g (100% der theorie) 3-(Nitromethyl)cyclohexanon, das für die nächste Umsetzung rein genug ist.

$R_f$ = 0,62 $CH_2Cl_2$ : $CH_3OH$ = 99 : 1

Beispiel 48

3-(Aminomethyl)cyclohexanol

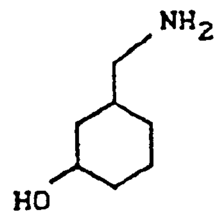

37,2 g 3-(Nitromethyl)cyclohexanon werden analog der Vorschrift für Beispiel 2 mit Lithiumaluminiumhydrid reduziert. Man erhält so 7,5 g (24,5% der Theorie) zähes, öliges 3-(Aminomethyl)cyclohexanol.

$R_f$ = 0,04 $CH_2Cl_2 : CH_3OH = 9 : 1$

Beispiel 49

3-(4-Fluorphenylsulfonamidomethyl)cyclohexanol

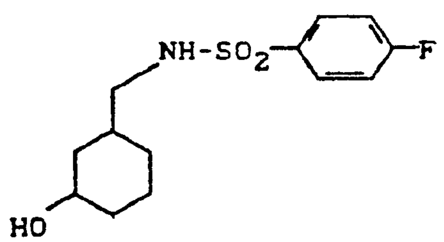

Analog der Vorschrift für Beispiel 3 werden 7,5 g 3-(Aminomethyl)cyclohexanol mit 11,3 g 4-Fluorphenylsulfonamid umgesetzt. Es werden dabei 11,05 g (66% der Theorie) zähes, öliges Isomerengemisch als Produkt erhalten.

$R_f$ = 0,41 und 0,38 $CH_2Cl_2 : CH_3OH = 95 : 5$

Beispiel 50

3-(4-Fluorphenylsulfonamidomethyl)cyclohexanon

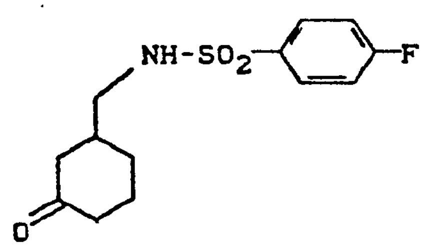

Analog der Vorschrift für Beispiel 4 werden 11 g 3-(4-Fluorphenylsulfonamidomethyl)cyclohexanol mit Chromtrioxid oxidiert. Man erhält dabei 9,3 g (86% der Theorie) Produkt als festen Schaum.

$R_f$ = 0,86 $CH_2Cl_2 : CH_3OH = 9 : 1$

Beispiel 51

4-(4-Fluorphenylsulfonamidomethyl)-1,2,3,4-tetrahydrocarbazol

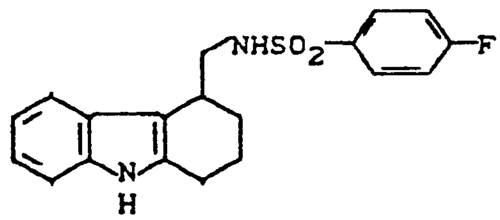

Analog der Vorschrift für Beispiel 5 werden 9 g 3-(4-Fluorphenylsulfonamidomethyl)cyclohexanon mit Phenylhydrazin umgesetzt. Man erhält dabei 9 g Rohprodukt, das an 1 kg Kieselgel (Merck 0,04-0,063 mm) in einem Gemisch von Toluol und Essigester im Verhältnis 8 zu 2 chromatographiert wird. Eine Fraktion ergibt dabei nach Eindampfen 0,8 g (7,2% der Theorie) Produkt als festen Schaum.

$R_f$ : 0,44 Toluol : Essigester = 8 : 2

Beispiel 52

9-(2-Cyanoethyl)-4-[N-(4-fluorphenylsulfonyl)-N-(2-cyanoethyl)aminomethyl]-1,2,3,4-tetrahydrocarbazol

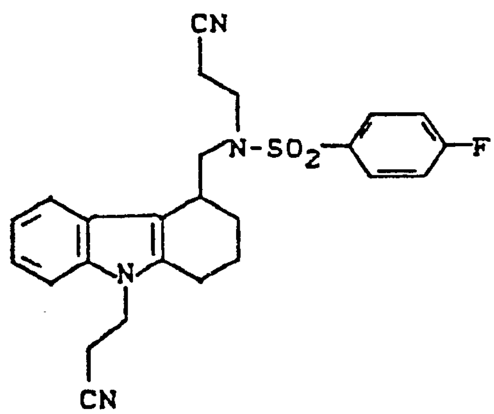

Analog der Vorschrift für Beispiel 6 werden 0,8 g 4-(4-Fluorphenylsulfonamidomethyl)-1,2,3,4-tetrahydrocarbazol mit Acrylnitril umgesetzt. Dabei erhält man 0,91 g (88% der Theorie) Produkt als Öl.

$R_f$ = 0,37 Toluol : Essigester = 8 : 2

Beispiel 53

9-(2-Carboxyethyl)-4-(4-fluorphenylsulfonamidomethyl)-1,2,3,4-tetrahydrocarbazol

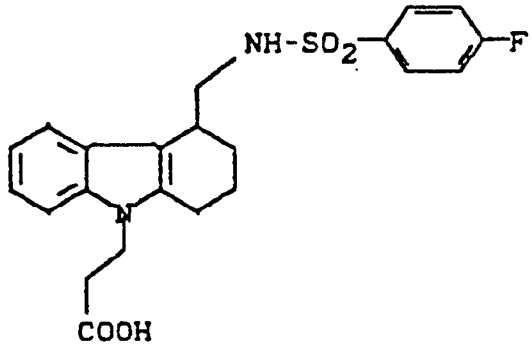

0,91 g 9-(2-Cyanoethyl)-4-[N-(4-fluorphenylsulfonyl)-N-(2-cyanoethyl)aminomethyl]-1,2,3,4-tetrahydrocarbazol werden analog zu Beispiel 7 verseift. Man erhält so 0,77 g (89% der Theorie) kristallines Produkt als Natriumsalz.

$F_p$ : 160°C

$R_f$ = 0,57 $CH_2Cl_2$ : $CH_3OH$ = 9 : 1

Beispiel 54

4-N-Acetamidocyclohexanol

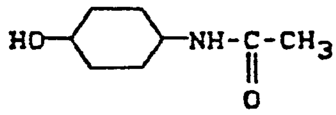

300 g Paracetamol werden in 750 ml Ethanol an 30 g Raney-Nickel bei 180°C und 100 bar hydriert. Nach Beendigung der Wasserstoffaufnahme wird der Katalysator abgesaugt und nach Zugabe von 30 g Raney-Nickel erneut bei 180°C und 100 bar Überdruck hydriert. Dann wird der Katalysator abfiltriert, das Filtrat im Vakuum eingedampft und der noch feuchte Rückstand mit 200 ml Aceton versetzt und ausgerührt. Nach Absaugen der Kristalle wird die Mutterlauge weiter eingeengt, von den ausgefallenen Kristallen wieder abgesaugt und die Mutterlauge erneut eingeengt. Man erhält zusammen mit dieser 3. Charge insgesamt 342,4 g (80,8% der Theorie) des Produktes.

Schmp. : 100-103°C

Beispiel 55

3-Amino-1,2,3,4-tetrahydrocarbazol (Racemat)

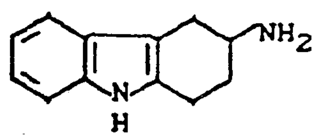

50 g (0,318 mol) 4-N-Acetamidocyclohexanol werden in 400 ml Eisessig gelöst und unter Rühren bei Raumtemperatur mit einer Lösung von 31,8 g (0,318 mol) Chromtrioxid in einem Gemisch von 26 ml Wasser und 105 ml Eisessig versetzt. Dabei steigt die Temperatur der Reaktionslösung auf 6O°C an. Die Reaktionsmischung wird 3 Stunden nachgerührt und dann mit 45,7 g (0,423 mol) Phenylhydrazin versetzt. Dabei tritt eine Erwärmung der Reaktionslösung auf 80°C und anfangs eine Stickstoffentwicklung auf. Die Reaktionsmischung wird dann 2,5 Stunden unter Rückfluß erhitzt. Nach Abkühlen wird die Reaktionsmischung mit 500 ml konz. Salzsäure und 59 ml Thioglykolsäure versetzt und unter Stickstoff 16 Stunden unter Rückfluß erhitzt. Nach Abkühlen wird mit 500 ml Essigester verdünnt und unter Kühlung mit 45%iger Natronlauge alkalisch gestellt. Das ausgefällte Chromhydroxid wird über eine Schicht Kieselgur abgesaugt und mit einem Gemisch aus Methylenchlorid/Methanol im Verhältnis 9 : 1 gewaschen. Vom Filtrat wird die organische Phase abgetrennt und die wäßrige Phase noch 3 mal mit Essigester extrahiert. Die vereinigten organischen Phasen werden mit 2 N Natronlauge zweimal gewaschen, dann mit je 1 l 2 N Schwefelsäure zweimal extrahiert. Die saure wäßrige Phase wird mit 45%iger Natronlauge alkalisch gestellt und 3 mal mit je 1 l Methylenchlorid extrahiert. Die vereinigten Methylenchloridphasen werden mit Natriumphosphat getrocknet und eingedampft. Der Rückstand wird mit 300 ml Ether und 50 ml Isopropanol versetzt und ausgerührt. Das ausgefallene Produkt wird abgesaugt, mit Ether gewaschen und im Vakuum getrocknet. Man erhält 28.6 g (48,3% der Theorie) Produkt.

Schmp. : 174-176°C.

Beispiel 56

3-[2S-(Chloracetamido)-3-phenylpropionamido]-1,2,3,4-tetrahydrocarbazol (Diasteromerengemisch)

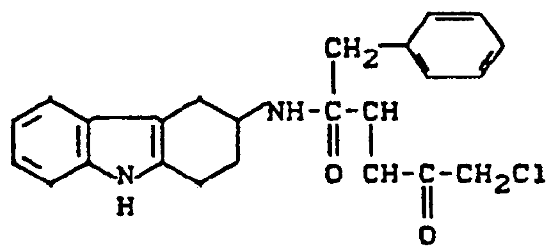

43 g (0,231 mol) 3-Amino-1,2,3,4-tetrahydrocarbazol und 55,87 g (0,231 mol) N-Chloracetyl-L-phenylalanin werden in 1,5 l Methylenchlorid unter Stickstoff suspendiert und bei 0°C mit 115,2 ml (0,832 mol) Triethylamin versetzt. Zu der Reaktionsmischung werden dann bei −20°C 150 ml (0,231 mol) einer 50%igen Lösung von Propanphosphonsäureanhydrid in Methylenchlorid zugetropft. Es wird bei −20°C 30 Minuten nachgerührt, anschließend wird 1,5 Stunden bei 0°C gerührt. Zur Aufarbeitung wird die Reaktionsmischung mit 1 l 2 N Schwefelsäure, mit 1 l Wasser und zweimal mit je 1 l gesättigter Bicarbonatlösung gewaschen. Nach Trocknen mit Natriumsulfat und eindampfen erhält man 100 g festen Rückstand.

Beispiel 57 und Beispiel 58

3-[2S-(Chloracetamido)-3-phenylpropionamido]-1,2,3,4-tetrahydrocarbazol (Diasteromeres A und Diasteromeres B)

a) Diasteromerentrennung durch Säulenchromatographie
100 g Rohprodukt aus Beispiel 56 werden an 2,5 kg Kieselgel (0,063 bis 0,2 mm, Merck) mit einem Gemisch von Toluol/Essigester im Verhältnis 6 : 4 als Laufmittel chromatographiert. Man erhält so 2 Fraktionen von denen die erste nach Eindampfen 34 g (35,9% der Theorie) des Diastereomeres A (Beispiel 57) ergibt.
Schmp. : 217-220°C
Die zweite Fraktion ergibt nach Eindampfen 24,3 g (25,7% der Theorie) des anderen Diasteromeres B (Beispiel 58).
Schmp. : 193-195°C
Drehwert des Diastereomeren A : $[\alpha]_D^{20}$ = 32,59° ($CH_3OH$) (Beispiel 57)
Drehwert des Diastereomeren B : $[\alpha]_D^{20}$ = 5,09° ($CH_3OH$) (Beispiel 58)
b) Diasteromerentrennung durch Kristallisation
11,5 g Rohprodukt aus Beispiel 56 wird in einem Gemisch von Ether und Isopropanol ausgerührt. Die abgesaugten Kristalle wurden in 40 ml Aceton 3 Stunden unter Rückfluß erhitzt. Nach Abkühlen und Stehenlassen über Nacht wurde abgesaugt und mit Aceton gewaschen. Man erhält so 1,2 g (5,5% der Theorie) sauberes Diasteromer A (Beispiel 57).

Beispiel 59

3-Amino-1,2,3,4-tetrahydrocarbazol (Enantiomer A)

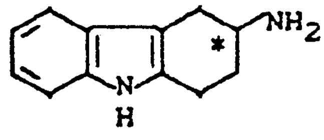

24,1 g (0,059 mol) des Diastereomeres 57 werden in 460 ml Eisessig gelöst, mit 460 ml konz. Salzsäure und 24 ml Thioglykolsäure versetzt und unter Stickstoff 3 Tage unter Rückfluß erhitzt. Dann wird die Reaktionsmischung mit 200 ml Wasser verdünnt und unter Kühlung mit 45%iger Natronlauge auf pH = 5 gestellt. Es wird zweimal mit je 1,5 l Essigester extrahiert, die wäßrige Phase dann mit 45%iger Natronlauge alkalisch gestellt

und 3 mal mit je 1,5 l Essigester extrahiert. Diese Essigesterextrakte werden vereinigt, mit Natriumsulfat getrocknet und eingedampft. Der Rückstand wird in 150 ml Ether ausgerührt. Das ausgefallene Produkt wird abgesaugt und im Vakuum getrocknet. Man erhält 7,8 g (71,3% der Theorie) des Enantiomers A. Schmp. : 160-166°C Drehwert $[\alpha]_D^{20}$ = 78,38° (DMSO +10% Wasser)

Beispiel 60

3-Amino-1,2,3,4-tetrahydrocarbazol (Enantiomer B)

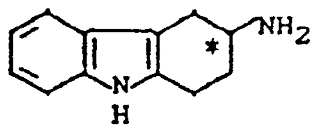

Die Herstellung von Enantiomer B erfolgt durch Hydrolyse von 58 analog zur Vorschrift von 59 aus 57.
Schmp. : 162-167°C
Drehwert $[\alpha]_D^{20}$ = – 78,11° (DMSO +10% $H_2O$)

Beispiel 61

3,3-Ethylendioxy-1,2,3,4-tetrahydrocarbazol

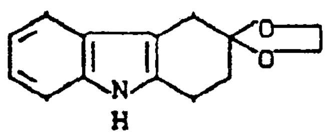

77,2 g (0,5 mol) 1,4-Cyclohexan-dion-monoethylenketal werden zusammen mit 48,4 ml (0,5 mol) Phenylhydrazin in 2 l Methylenchlorid gelöst mit 300 g Magnesiumsulfat versetzt und 30 min gerührt. Das Magnesiumsulfat wird dann abgesaugt, mit Methylenchlorid gewaschen und das Filtrat eingedampft. Der Rückstand wird in 1,5 l Benzol aufgenommen, mit 62,1 g (0,46 mol) wasserfreiem Zinkchlorid versetzt und 3 h am Wasserabscheider unter Rückfluß erhitzt. Dann wird die Reaktionslösung eingeengt, mit 2 N Natronlauge versetzt und mit Essigester 3 mal extrahiert. Die vereinigten Essigesterphasen werden mit Natriumsulfat getrocknet und eingedampft. Der Rückstand kristallisiert aus wenig Ether. Man erhält so 3,5 g (72,9% der Theorie) des Produktes.
Schmp. : 145-146°C

Beispiel 62

1,2,4,9-Tetrahydrocarbazol-3-on

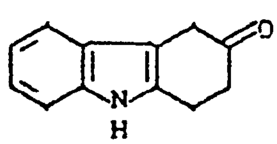

165 g (0,72 mol) 3,3-Ethylendioxy-1,2,3,4-tetrahydrocarbazol werden in 2 l Aceton gelöst und mit 3 g p-Toluolsulfonsäure versetzt. Nach 4 h Erhitzen unter Rückfluß wird die Reaktionslösung eingeengt mit 2 l Essigester versetzt und 3 mal mit je 1 l gesättigter Bicarbonatlösung extrahiert. Die organische Phase wird mit Natriumsulfat getrocknet und eingedampft. Der Rückstand kristallisiert aus Ether. Man erhält so 118,7 g (89,1% der Theorie) des Produktes.
Schmp. : 145-148°C

Beispiel 63

3-(1S-Phenylethylamino)-1,2,3,4-tetrahydrocarbazol

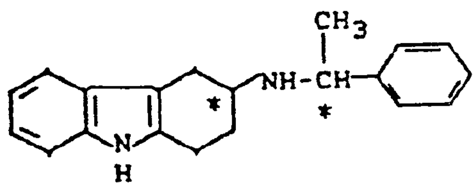

11,06 g (0,0595 mol) 1,2,4,9-Tetrahydrocarbazol-3-on werden zusammen mit 7,78 g (0,065 mol) 1S-Phenylethylamin in 300 ml Benzol 1 h am Wasserabscheider unter Rückfluß erhitzt. Nach Abdampfen des Benzols wird der Rückstand in 50 ml Methylenchlorid gelöst und zu einer Lösung von 15,3 g (0,0595 mol) Tetrabutylammoniumborhydrid in 120 ml Methylenchlorid bei –50°C zugetropft. Innerhalb von 1 h läßt man die Reaktionsmischung wieder auf Raumtemperatur kommen, gibt 6 ml Methanol dazu und versetzt vorsichtig (Wasserstoffentwicklung) mit 120 ml 2 N Schwefelsäure. Nach 1 h führen bei Raumtemperatur werden die ausgefallenen Kristalle abgesaugt, 2 mal mit Wasser und 1 mal mit Methylenchlorid gewaschen. Nach Trocknen im Hochvakuum erhält man 0,16 g (39,7% der Theorie) des Produktes als Hydrogensulfat.

Schmp. : 160-170°C

Drehwert : $[\alpha]_D^{20}$ = 26,36° ($CH_3OH/H_2O$ = 80 : 20)

Beispiel 64

3-Amino-1,2,3,4-Tetrahydrocarbazol (Enantiomer A)

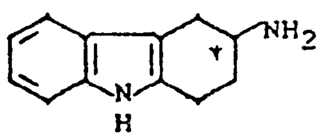

[Beispiel 64, hergestellt nach Verfahren B, ist identisch mit Beispiel 59]

10 g des erhaltenen Hydrogensulfats von Beispiel 63 werden zur Überführung in das Hydrochlorid in 50 ml Methanol suspendiert, mit 30 ml 2 N Natronlauge versetzt und mit Essigester extrahiert. Die organische Phase wird eingedampft, der Rückstand in 50 ml Methanol gelöst und mit 20 ml konz. Salzsäure versetzt. Beim Einengen im Vakuum fällt das Hydrochlorid aus. Nach Absaugen, Waschen mit Wasser und Trocknen im Vakuum erhält man 7,6 g Hydrochlorid. Diese 7,6 g (0,023 mol) Hydrochlorid werden zusammen mit 7,17 g (0,115 mol) Ammoniumformiat und 7,2 g 10% Palladium auf Aktivkohle in 80 ml trockenem Dimethylformamid 20 min unter Rückfluß (unter Stickstoff) erhitzt. Nach dem Abkühlen wird mit Wasser verdünnt, der Katalysator abgesaugt und mit Wasser gewaschen. Die vereinigten Filtrate werden mit 2 N Schwefelsäure angesäuert und 2 mal mit Essigester extrahiert. Die wäßrige Phase wird mit 2 N Natronlauge alkalisch gestellt und mit Essigester dreimal extrahiert. Die organische Phasen werden mit Natriumsulfat getrocknet und eingedampft. Der Rückstand wird im Hochvakuum zur Entfernung von Dimethylformamid weiter eingedampft. Aus Ether erhält man 3 g (70% der Theorie) kristallines Enantiomer A.

Schmp. : 160-166°C

Drehwert : $[\alpha]_D^{20}$ = 78,38° (DMSO +10% Wasser)

Beispiel 65

3-(4-Fluorphenylsulfonamido)-1,2,3,4-tetrahydrocarbazol (Enantiomer A)

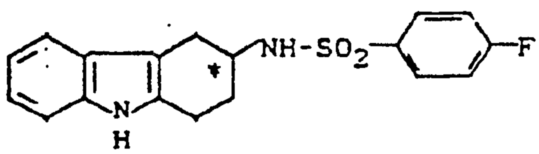

3,72 g (0,02 mol) des Beispiels 59 werden zusammen mit 3 ml (0,022 mol) Triethylamin in 30 ml Methylenchlorid suspendiert und unter Kühlung mit 3,9 g (0,02 mol) 4-Fluorbenzolsulfonsäurechlorid versetzt. Die Reaktionsmischung wird 1 h bei Raumtemperatur gelöst, dann mit 200 ml Essigester verrührt und 2 mal mit 2 N Schwefelsäure und 2 mal mit 2 N Natronlauge extrahiert. Die organische Phase wird mit Natriumsulfat getrocknet und eingedampft. Der feste Rückstand wird mit Ether versetzt und kristallisiert. Man erhält 5,8 g (84% der Theorie) des Produktes.

Schmp. : 150-152°C

Drehwert : $[\alpha]_D^{20}$ = 50,43°($CHCl_3$)

Beispiel 66

3-(4-Fluorphenylsulfonamido)-1,2,3,4-tetrahydrocarbazol (Enantiomer B)

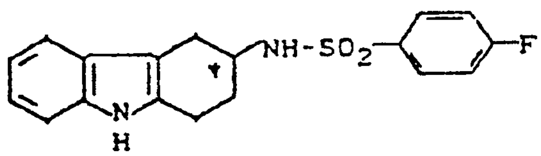

Die Herstellung des Enantiomers B erfolgt aus Beispiel 60 analog zur Herstellung von Beispiel 65 aus Beispiel 59.

Schmp. : 150-152°C

Drehwert : $[\alpha]_D^{20}$ = – 48,99°($CHCl_3$)

Beispiel 67

3-[N-(4-Fluorphenylsulfonyl)-amino]-9-(2-cyanoethyl)-1,2,3,4-tetrahydrocarbazol (Enantiomer A)

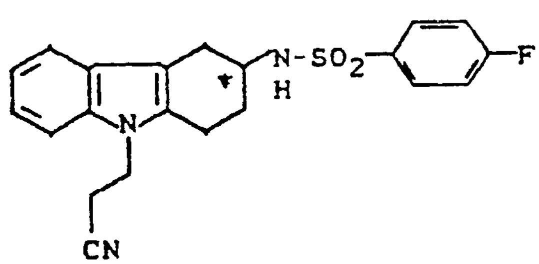

5,16 g (0,015 mol) des Beispiels 65 werden in 200 ml trockenem Dimethylformamid unter Stickstoff gelöst und portionsweise mit 0,5 g (0,0165 mol) Natriumhydrid mit 20% Spindelöl versetzt. Nach Beendigungder Wasserstoffentwicklung werden 2 ml (0,03 mol) Acrylnitril zur Reaktionsmischung gegeben. Nach 1 h Rühren bei Raumtemperatur werden noch einmal 0,5 ml Acrylnitril zugegeben und 1 h bei Raumtemperatur gerührt. Dann wird mit 1 l Essigester verdünnt und mit Wasser dreimal extrahiert. Die Essigesterphase wird mit Natriumsulfat getrocknet und eingedampft. Man erhält so 7,8 g Rohprodukt, das an 150 g Kieselgel (0,063 bis 0,2 mm, Merck) mit einem Gemisch von Toluol/Essigester im Verhältnis 1 : 1 chromatographiert wird. Man erhält eine Fraktion, die nach dem Eindampfen 5,8 g (86% der Theorie) Produkt als festem Schaum ergibt.

Beispiel 68

3-[N-(4-Fluorphenylsulfonyl)-N-(2-cyanoethyl)-amino]-9-(2-cyanoethyl)-1,2,3,4-tetrahydrocarbazol (Enantiomer B)

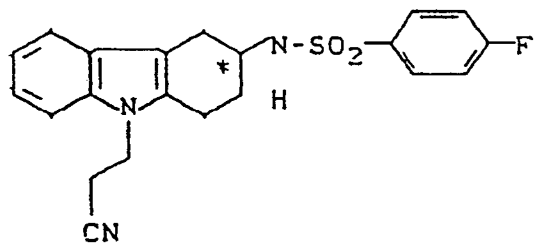

Die Herstellung von Beispiel 68 aus Beispiel 66 erfolgt analog zur Herstellung von Beispiel 67 aus Beispiel 65.

Beispiel 69

(+)-3-(4-Fluorphenylsulfonamido)-9-(2-carboxyethyl)-1,2,3,4-tetrahydrocarbazol

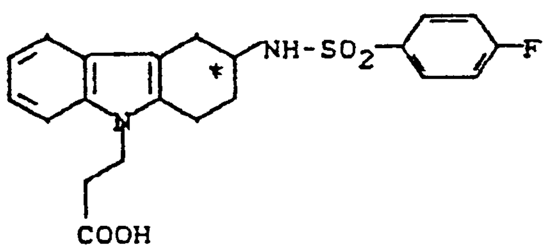

5,8 g (0,0128 mol) des Beispiels 67 werden in 60 ml Isopropanol gelöst, mit 130 ml 10%iger Kalilauge versetzt, nach 16 h Erhitzen unter Rückfluß wird abgekühlt, mit Wasser verdünnt und mit Essigester extrahiert. Die wäßrige Phase wird im Vakuum eingeengt und dann unter heftigem Rühren tropfenweise mit konz. Salzsäure angesäuert. Die dabei ausgefallene Säure wird abgesaugt, mit Wasser gewaschen und im Vakuum gründlich getrocknet. Man erhält 4,4 g (86,6% der Theorie) des Produktes.

Schmp.: 85-95°C

Drehwert $[\alpha]_D^{20}$ = 42,55° ($CHCl_3$)

Beispiel 70

(–)-3-(4-Fluorphenylsulfonamido)-9-(2-carboxyethyl)-1,2,3,4-tetrahydrocarbazol

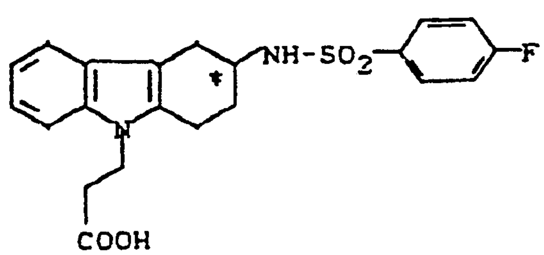

Die Herstellung von Beispiel 70 aus Beispiel 68 erfolgt analog zur Herstellung von Beispiel 69 aus Beispiel 67.

Schmp.: 85-95°C

Drehwert: $[\alpha]_D^{20}$ = – 37,83° ($CHCl_3$)

Beispiel 71

(+)-3-Amino-1,2,3,4-tetrahydrocarbazol

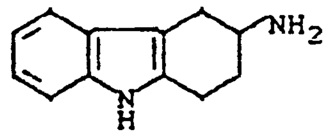

18,6 g (0,1 Mol) racemisches 3-Amino-1,2,3,4-tetrahydrocarbazol werden zusammen mit 15,2 g (0,1 Mol) (+)-Mandelsäure in 100 ml Tetrahydrofuran unter Rückfluß erhitzt. Nach Erhalt einer klaren Lösung läßt man abkühlen und gibt als Impfkristalle eine Spatelspitze (+)-Mandelsäuresalz von (+)-3-Amino-1,2,3,4-tetrahydrocarbazol (Enantiomer A, Beispiel 59) dazu. Es wird über Nacht gerührt und die ausgefallenen Kristalle werden abgesaugt. Man erhält so 6,05 g enantiomerenangereichertes Material. 4,7 g dieser Kristalle werden in 330 ml Methylisobutylketon in der Siedehitze gelöst, nach geringfügiger Abkühlung angeimpft und während des weiteren Abkühlens gerührt. Nach Absaugen und Waschen mit Methylisobutylketon erhält man 3,4 g (+)-3-Amino-1,2,3,4-tetrahydrocarbazol als (+)-Mandelsäuresalz.

Beispiel 72

Zur Bestimmung der thrombozytenaggregationshemmenden Wirkung wurde Blut von gesunden Probanden beiderlei Geschlechts verwendet. Als Antikoagulans wurden einem Teil 3,8%iger wäßriger Natriumzitratlösung 9 Teile Blut zugemischt. Mittels Zentrifugation erhält man aus diesem Blut plättchenreiches Zitratplasma (PRP) (Jürgens/Beller, Klinische Methoden der Blutgerinnungsanalyse ; Thieme Verlag, Stuttgart 1959).

Für diese Untersuchungen wurden 0,8 ml PRP und 0,1 ml der Wirkstofflösung bei 37°C im Wasserbad vorinkubiert. Anschließend wurde die Thrombozytenaggregation nach der turbidometrischen Methode im Aggregometer bei 37°C bestimmt (Born, G.V.R., J. Physiol. (London), 162, 1962 und Therapeutische Berichte 47, 80-86, 1975). Hierzu wurde die vorinkubierte Probe mit 0,1 ml Kollagen, einem aggregationsauslösenden Agens, versetzt. Die Veränderung der optischen Dichte in der Probe der PRP wurde während einer Zeitdauer von 6 Minuten aufgezeichnet und der Ausschlag nach 6 Minuten bestimmt. Hierzu wird die prozentuale Hemmung gegenüber der Kontrolle errechnet.

| Cycloalkano[1.2-b]indolsulfonamid nach Beispiel-Nr. | Grenzkonzentration für Hemmung (mg/l) |
|---|---|
| 7 | 10 - 3 |
| 12 | 0,03 - 0,01 |
| 17 | 0,03 - 0,01 |
| 22 | 3 - 1 |
| 27 | 0,1 - 0,03 |
| 32 | 0,1 - 0,03 |
| 38 | 1,0 - 0,3 |
| 39 | 0,3 - 0,1 |
| 40 | 1,0 - 0,3 |
| 41 | 0,3 - 0,1 |
| 46 | 0,1 - 0,01 |
| 53 | 0,3 - 0,1 |

Vergleich

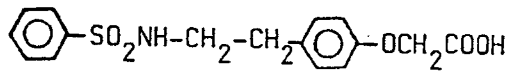

1 - 0,3

(4-/2-(Phenylsulfonamido)-ethyl/-phenoxyessigsäure)

## Ansprüche

**Patentansprüche für die Vertragsstaaten : AT, BE, CH, DE, FR, GB, GR, IT, LI, LU, NL, SE**

1. Cycloalkano[1.2-b]indol-sulfonamide der Formel

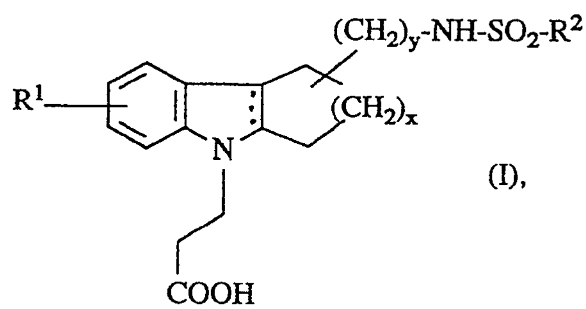

(I),

worin

$R^1$ – für Wasserstoff, Fluor, Chlor, Brom, Trifluormethyl, Carboxy, $C_1$-$C_6$-Alkoxycarbonyl steht,

– für eine Gruppe der Formel

$-S(O)_mR^3$ steht,

worin

$R^3$ – $C_1$-$C_6$-Alkyl oder Phenyl bedeutet und

m – eine Zahl 0 oder 2 bedeutet,

– für eine Gruppe der Formel

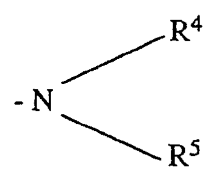

steht,

worin

$R^4$ und $R^5$ gleich oder verschieden sind und Wasserstoff, $C_1$-$C_6$-Alkyl, Phenyl oder Acetyl bedeuten,

– oder für eine Gruppe der Formel

$-OR^6$ steht,

worin

$R^6$ – Wasserstoff, $C_1$-$C_6$-Alkyl oder Phenyl bedeutet,

– oder für gegebenenfalls durch Carboxy, Methoxycarbonyl, Ethoxycarbonyl, Fluor, Chlor, Brom, Hydroxy, $C_1$-$C_6$-Alkoxy oder Cyano substituiertes $C_1$-$C_6$-Alkyl steht,

$R^2$ – für Phenyl steht, das gegebenenfalls bis zu dreifach durch Fluor, Chlor, Brom, Cyano, Trifluormethyl, Trifluormethoxy, Trifluormethylthio, $C_1$-$C_6$-Alkyl, Carboxymethyl, Carboxyethyl, Methoxymethyl, Ethoxmyethyl, Methoxyethyl, Ethoxyethyl, $C_1$-$C_6$-Alkoxy, $C_1$-$C_6$-Alkylthio, Hydroxy, Carboxy, $C_1$-$C_6$-Alkoxycarbonyl, Phenyl, Phenoxy, Benzyloxy, Benzylthio oder durch die Gruppe

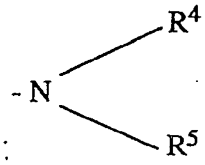

substituiert ist,

worin

$R^4$ und $R^5$ die bereits angegebene Bedeutung haben,
x – für die Zahl 1 oder 2 steht, und
y – für die Zahl 0 oder 1 steht, gegebenenfalls in einer isomeren Form, oder deren Salze.

2. Cycloalkano[1.2-b]indol-sulfonamide nach Anspruch 1, worin
$R^1$ – für Wasserstoff, Fluor, Chlor, Brom, Trifluormethyl, Methylthio, Ethylthio, Methylsulfonyl, Phenylthio, Phenylsulfonyl, Amino, Dimethylamino, Diethylamino, Acetylamino steht, oder
– für eine Gruppe der Formel

$-OR^6$ steht,

worin
$R^6$ – Wasserstoff, $C_1$-$C_4$-Alkyl oder Phenyl bedeutet,
– oder für $C_1$-$C_4$-Alkyl steht,
$R^2$ – für Phenyl steht, das bis zu zweifach gleich oder verschieden durch Fluor, Chlor, Brom, Cyano, Trifluormethyl, Trifluormethoxy, $C_1$-$C_4$-Alkyl, $C_1$-$C_4$-Alkoxy, Methylthio, Hydroxy, Methoxycarbonyl, Ethoxycarbonyl, Dimethylamino, Acetylamino, Diethylamino substituiert ist,
x – für die zahl 1 oder 2 steht, und
y – für die Zahl 0 oder 1 steht, gegebenenfalls in einer isomeren Form, oder deren Salze.

3. Cycloalkano[1.2-b]indol-sulfonamide nach Anspruch 1 worin
$R^1$ – für Wasserstoff, Fluor, Methyl, Methoxy, Benzyloxy oder Hydroxy steht,
$R^2$ – für Phenyl steht, das durch Fluor, Chlor, Trifluormethyl, Methyl, Ethyl, Propyl, Isopropyl oder Methoxy substituiert ist,
x – für die Zahl 1 oder 2 steht, und
y – für die Zahl 0 oder 1 steht, gegebenenfalls in einer isomeren Form, oder deren Salze.

4. (+) oder (–) isomere Cycloalkano[1.2-b]-indolsulfonamide nach den Ansprüchen 1 bis 3 der Formel

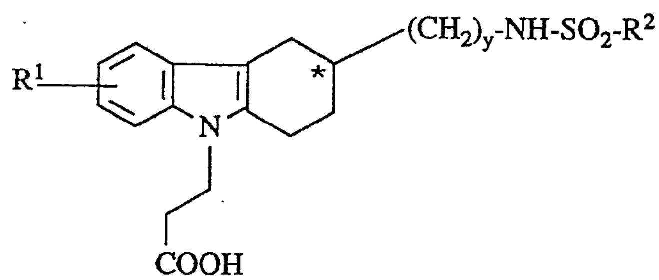

(XII)

in der
$R^1$ – für Wasserstoff, Fluor, Methyl, Methoxy, Benzyloxy oder Hydroxy steht,
$R^2$ – für Phenyl steht, das durch Fluor, Chlor, Trifluormethyl, Methyl, Ethyl, Propyl, Isopropyl, oder Methoxy substituiert ist, und
y – für die Zahl 0 oder 1 steht, oder deren Salze.

5. (+)-3-(4-Fluor-phenyl-sulfonamido)-9-(2-carboxy-ethyl)-1,2,3,4-tetrahydrocarbazol.

6. (–)-3-(4-Fluor-phenyl-sulfonamido)-9-(2-carboxy-ethyl)-1,2,3,4-tetrahydrocarbazol.

7. Verfahren zur Herstellung von Cycloalkano[1.2-b]indol-sulfonamide der Formel

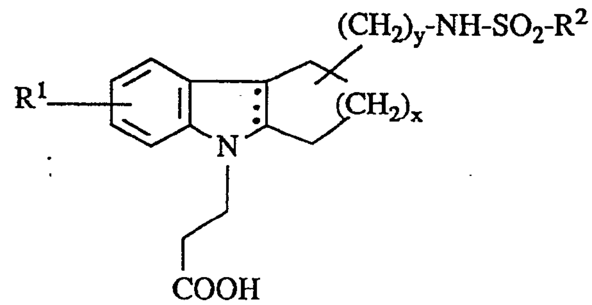

(I)

worin

$R^1$ – für Wasserstoff, Fluor, Chlor, Brom, Trifluormethyl, Carboxy, $C_1$-$C_6$-Alkoxycarbonyl steht,

– für eine Gruppe der Formel

$-S(O)_mR^3$ steht,

worin

$R^3$ – $C_1$-$C_6$-Alkyl oder Phenyl bedeutet und

m – eine Zahl 0 oder 2 bedeutet,

– für eine Gruppe der Formel

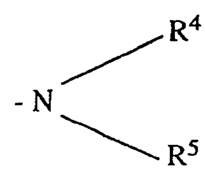

steht, worin

$R^4$ und $R^5$ gleich oder verschieden sind und Wasserstoff, $C_1$-$C_6$-Alkyl, Phenyl oder Acetyl bedeuten,

– oder für eine Gruppe der Formel

– $OR^6$ steht, worin

$R^6$ – Wasserstoff, $C_1$-$C_6$-Alkyl oder Phenyl bedeutet,

– oder für gegebenenfalls durch Carboxy, Methoxycarbonyl, Ethoxycarbonyl, Fluor, Chlor, Brom, Hydroxy, $C_1$-$C_6$-Alkoxy oder Cyano substituiertes $C_1$-$C_6$-Alkyl steht,

$R^2$ – für Phenyl steht, das gegebenenfalls bis zu dreifach durch Fluor, Chlor, Brom, Cyano, Trifluormethyl, Trifluormethoxy, Trifluormethylthio, $C_1$-$C_6$-Alkyl, Carboxymethyl, Carboxyethyl, Methoxymethyl, Ethoxymethyl, Methoxyethyl, Ethoxyethyl, $C_1$-$C_6$-Alkoxy, $C_1$-$C_6$-Alkylthio, Hydroxy, Carboxy, $C_1$-$C_6$-Alkoxycarbonyl, Phenyl, Phenoxy, Benzyloxy, Benzylthio oder durch die Gruppe

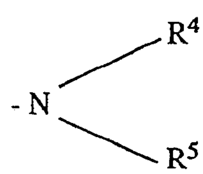

substituiert ist, worin

$R^4$ und $R^5$ die bereits angegebene Bedeutung haben,

x – für die Zahl 1 oder 2 steht, und

y – für die Zahl 0 oder 1 steht, gegebenenfalls in einer isomeren Form, oder deren Salze, dadurch gekennzeichnet, daß man [Benzolsulfonamidoalkyl]-cycloalkano[1.2-b]indole der allgemeinen Formel

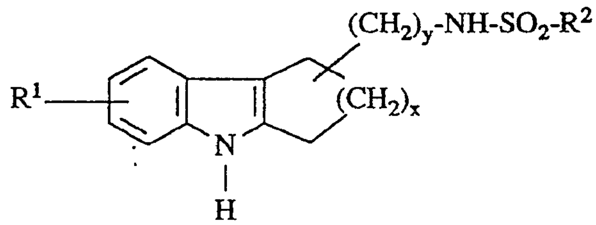

(XIII)

in welcher

$R^1$, $R^2$, x und y die obengenannte Bedeutung haben, mit Acrylnitril in Gegenwart eines inerten Lösungsmittels, gegebenenfalls in Gegenwart einer Base, umsetzt, dann die N,N'-Biscyanoethylverbindungen in Gegenwart von Basen in einem inerten Lösemittel verseift, dann im Fall der Herstellung der Cyclo-alkano[1.2-b]dihydroindol-sulfonamide die Cyclo-alkano[1.2-b]indol-sulfonamide gegebenenfalls in Gegenwart eines inerten Lösungsmittels in Gegenwart einer Säure und eines Reduktionsmittels hydriert, gegebenenfalls die Isomeren in üblicher Weise trennt, und dann gegebenenfalls im Fall der Herstellung der Salze mit einer entsprechenden Base umsetzt.

8. Verfahren nach Anspruch 7, dadurch gekennzeichnet, daß man die Umsetzung des [Benzolsulfonamidoalkyl]cycloalkano-[1.2-b]indols mit Acrylnitril im Temperaturbereich von 0 bis 150°C durchführt.

9. Verfahren nach den Ansprüchen 7 und 8, dadurch gekennzeichnet, daß man Acrylnitril und [Benzolsulfonamidoalkyl]cycloalkano[1.2-b]indol im Verhältnis 1 bis 20 Mol zu 1 Mol einsetzt.

10. [Benzolsulfonamidoalkyl]cycloalkano[1.2-b]indole der Formel

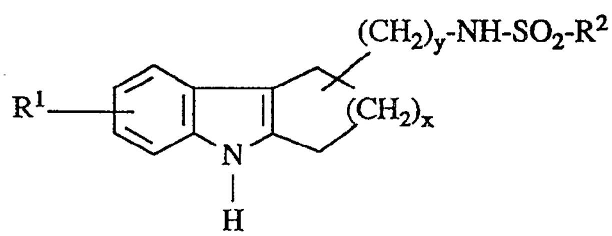

(XIII)

in der

$R^1$ – für Wasserstoff, Fluor, Chlor, Brom, Trifluormethyl, Carboxy, $C_1$-$C_6$-Alkoxycarbonyl steht,

– für eine Gruppe der Formel

$$-S(O)_mR^3 \text{ steht,}$$

worin

$R^3$ – $C_1$-$C_6$-Alkyl oder Phenyl bedeutet und

m – eine Zahl 0 oder 2 bedeutet,

– für eine Gruppe der Formel

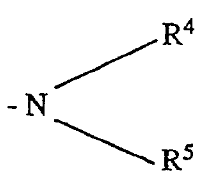

steht,

worin

$R^4$ und $R^5$ gleich oder verschieden sind und Wasserstoff, $C_1$-$C_6$-Alkyl, Phenyl oder Acetyl bedeuten,

– oder für eine Gruppe der Formel

$$-OR^6 \text{ steht,}$$

worin

$R^6$ – Wasserstoff, $C_1$-$C_6$-Alkyl oder Phenyl bedeutet,

– oder für gegebenenfalls durch Carboxy, Methoxycarbonyl, Ethoxycarbonyl, Fluor, Chlor, Brom, Hydroxy, $C_1$-$C_6$-Alkoxy oder Cyano substituiertes $C_1$-$C_6$-Alkyl steht,

$R^2$ – für Phenyl steht, das gegebenenfalls bis zu dreifach durch Fluor, Chlor, Brom, Cyano, Trifluormethyl, Trifluormethoxy, Trifluormethylthio, $C_1$-$C_6$-Alkyl, Carboxymethyl, Carboxyethyl, Methoxymethyl, Ethoxymethyl,

Methoxyethyl, Ethoxyethyl, $C_1$-$C_6$-Alkoxy, $C_1$-$C_6$-Alkylthio, Hydroxy, Carboxy, $C_1$-$C_6$-Alkoxycarbonyl, Phenyl, Phenoxy, Benzyloxy, Benzylthio oder durch die Gruppe

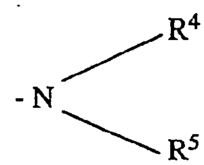

substituiert ist, worin

$R^4$ und $R^5$ die bereits angegebene Bedeutung haben,

x – für die Zahl 1 oder 2 steht, und

y – für die Zahl 0 oder 1 steht, gegebenenfalls in einer isomeren Form.

11. Verfahren zur Herstellung von [Benzolsulfonamidoalkyl]cycloalkano[1.2-b]indolen der Formel

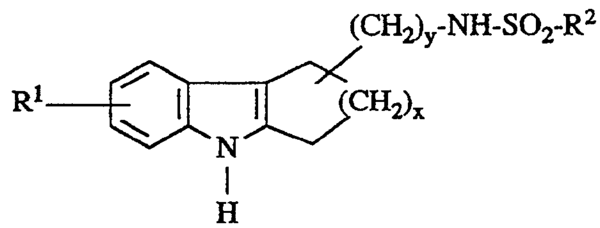

(XIII)

in der

$R^1$ – für Wasserstoff, Fluor, Chlor, Brom, Trifluormethyl, Carboxy, $C_1$-$C_6$-Alkoxycarbonyl steht,

– für eine Gruppe der Formel

$-S(O)_mR^3$ steht,

worin

$R^3$ – $C_1$-$C_6$-Alkyl oder Phenyl bedeutet und

m – eine Zahl 0 oder 2 bedeutet,

– für eine Gruppe der Formel

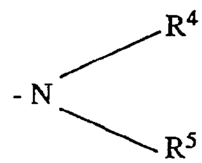

steht, worin

$R^4$ und $R^5$ gleich oder verschieden sind und Wasserstoff, $C_1$-$C_6$-Alkyl, Phenyl oder Acetyl bedeuten,

– oder für eine Gruppe der Formel

$-OR^6$ steht,

worin

$R^6$ – Wasserstoff, $C_1$-$C_6$-Alkyl oder Phenyl bedeutet,

– oder für gegebenenfalls durch Carboxy, Methoxycarbonyl, Ethoxycarbonyl, Fluor, Chlor, Brom, Hydroxy, $C_1$-$C_6$-Alkoxy oder Cyano substituiertes $C_1$-$C_6$-Alkyl steht,

$R^2$ – für Phenyl steht, das gegebenenfalls bis zu dreifach durch Fluor, Chlor, Brom, Cyano, Trifluormethyl, Trifluormethoxy, Trifluormethylthio, $C_1$-$C_6$-Alkyl, Carboxymethyl, Carboxyethyl, Methoxymethyl, Ethoxymethyl, Methoxyethyl, Ethoxyethyl, $C_1$-$C_6$-Alkoxy, $C_1$-$C_6$-Alkylthio, Hydroxy, Carboxy, $C_1$-$C_6$-Alkoxycarbonyl, Phenyl, Phenoxy, Benzyloxy, Benzylthio oder durch die Gruppe

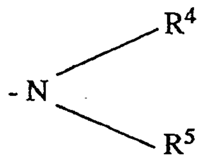

substituiert ist, worin

$R^4$ und $R^5$ die bereits angegebene Bedeutung haben,

x – für die Zahl 1 oder 2 steht, und

y – für die Zahl 0 oder 1 steht, gegebenenfalls in einer isomeren Form, dadurch gekennzeichnet, daß man Phenylhydrazine der allgemeinen Formel

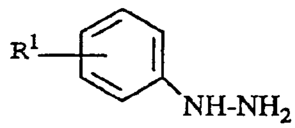

(XIV)

in welcher

$R^1$ – die oben angegebene Bedeutung hat, mit Cycloalkanonsulfonamiden der allgemeinen Formel

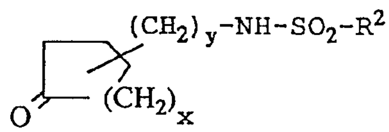

(XV)

in welcher

$R^2$, x und y die oben angegebene Bedeutung haben, in Gegenwart von inerten Lösungsmitteln, gegebenenfalls in Anwesenheit eines Katalysators umsetzt.

12. Ketone der Formel

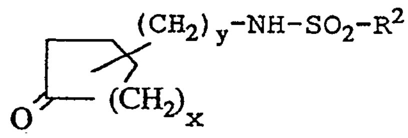

(XVb)

in der

$R^2$ – für Phenyl steht, das gegebenenfalls bis zu dreifach durch Fluor, Chlor, Brom, Cyano, Trifluormethyl, Trifluormethoxy, Trifluormethylthio, $C_1$-$C_6$-Alkyl, Carboxymethyl, Carboxyethyl, Methoxymethyl, Ethoxymethyl, Methoxyethyl, Ethoxyethyl, $C_1$-$C_6$-Alkoxy, $C_1$-$C_6$-Alkylthio, Hydroxy, Carboxy, $C_1$-$C_6$-Alkoxycarbonyl, Phenyl, Phenoxy, Benzyloxy, Benzylthio oder durch die Gruppe

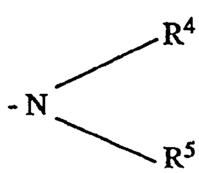

substituiert ist, worin

$R^4$ und $R^5$ gleich oder verschieden sind und Wasserstoff, $C_1$-$C_6$-Alkyl, Phenyl oder Acetyl bedeuten,

x – für die Zahl 1 steht, und

y – für die Zahl 0 oder 1 steht.

13. Verfahren zur Herstellung von Cycloalkanosulfonamiden der Formel

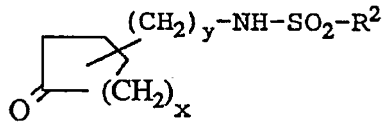

(XVb)

in der

$R^2$ – für Phenyl steht, das gegebenenfalls bis zu dreifach durch Fluor, Chlor, Brom, Cyano, Trifluormethyl, Trifluormethoxy, Trifluormethylthio, $C_1$-$C_6$-Alkyl, Carboxymethyl, Carboxyethyl, Methoxymethyl, Ethoxymethyl, Methoxyethyl, Ethoxyethyl, $C_1$-$C_6$-Alkoxy, $C_1$-$C_6$-Alkylthio, Hydroxy, Carboxy, $C_1$-$C_6$-Alkoxycarbonyl, Phenyl, Phenoxy, Benzyloxy, Benzylthio oder durch die Gruppe

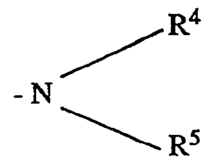

substituiert ist, worin

$R^4$ und $R^5$ gleich oder verschieden sind und Wasserstoff, $C_1$-$C_6$-Alkyl, Phenyl oder Acetyl bedeuten,

x – für die Zahl 1 steht, und

y – für die Zahl 0 oder 1 steht, dadurch gekennzeichnet, daß man Cycloalkanole der Formel

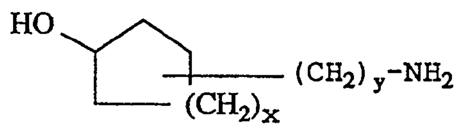

(XVI)

in der

x und y die oben angegebene Bedeutung haben, mit Sulfonsäurehalogeniden der Formel

$$Hal - SO_2 - R^2 \qquad (XVII)$$

in der

$R^2$ die oben angegebene Bedeutung hat und Hal für Fluor, Chlor, Brom oder Iod steht, in inertem organischen Lösungsmittel, gegebenenfalls in Gegenwart von Basen, umsetzt und anschließend in inertem Lösungsmittel mit Chrom(VI)-Verbindungen oxidiert.

14. Arzneimittel, enthaltend Cycloalkano[1.2-b]indol-sulfonamide nach Anspruch 1.

15. Verwendung von Cycloalkano[1.2-b]indol-sulfonamiden nach Anspruch 1 zur Herstellung von Arzneimitteln.

16. Arzneimittel nach Anspruch 14 zur Behandlung von Thrombosen, Thromboembolien, Ischämien, Asthma und Allergien.

17. Cycloalkano[1.2-b]indol-sulfonamide nach Anspruch 1 zur Bekämpfung von Krankheiten.

**Patentansprüche für den Vertragsstaat : ES**

1. Verfahren zur Herstellung von Cycloalkano[1.2-b]indol-sulfonamide der Formel

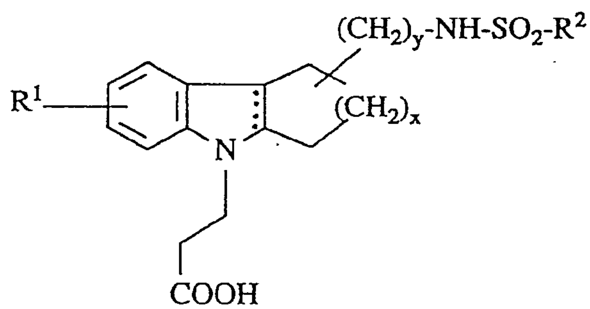

(I)

worin

$R^1$ – für Wasserstoff, Fluor, Chlor, Brom, Trifluormethyl, Carboxy, $C_1$-$C_6$-Alkoxycarbonyl steht,

– für eine Gruppe der Formel

$$-S(O)_mR^3 \text{ steht,}$$

worin

$R^3$ – $C_1$-$C_6$-Alkyl oder Phenyl bedeutet und

m – eine Zahl 0 oder 2 bedeutet,

– für eine Gruppe der Formel

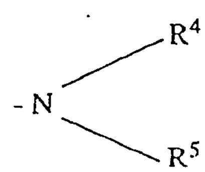

steht, worin

$R^4$ und $R^5$ gleich oder verschieden sind und Wasserstoff, $C_1$-$C_6$-Alkyl, Phenyl oder Acetyl bedeuten,

– oder für eine Gruppe der Formel

– $OR^6$ steht, worin

$R^6$ – Wasserstoff, $C_1$-$C_6$-Alkyl oder Phenyl bedeutet,

– oder für gegebenenfalls durch Carboxy, Methoxycarbonyl, Ethoxycarbonyl, Fluor, Chlor, Brom, Hydroxy, $C_1$-$C_6$-Alkoxy oder Cyano substituiertes $C_1$-$C_6$-Alkyl steht,

$R^2$ – für Phenyl steht, das gegebenenfalls bis zu dreifach durch Fluor, Chlor, Brom, Cyano, Trifluormethyl, Trifluormethoxy, Trifluormethylthio, $C_1$-$C_6$-Alkyl, Carboxymethyl, Carboxyethyl, Methoxymethyl, Ethoxymethyl, Methoxyethyl, Ethoxyethyl, $C_1$-$C_6$-Alkoxy, $C_1$-$C_6$-Alkylthio, Hydroxy, Carboxy, $C_1$-$C_6$-Alkoxycarbonyl, Phenyl, Phenoxy, Benzyloxy, Benzylthio oder durch die Gruppe

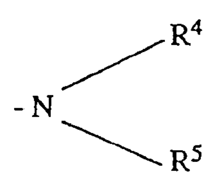

substituiert ist, worin

$R^4$ und $R^5$ die bereits angegebene Bedeutung haben,

x – für die Zahl 1 oder 2 steht, und

y – für die Zahl 0 oder 1 steht, gegebenenfalls in einer isomeren Form, oder deren Salze, dadurch gekennzeichnet, daß man [Benzolsulfonamidoalkyl]-cycloalkano[1.2-b]indole der allgemeinen Formel

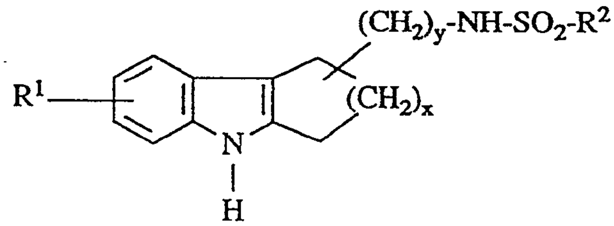

(XIII)

in welcher

$R^1$, $R^2$, x und y die obengenannte Bedeutung haben, mit Acrylnitril in Gegenwart eines inerten Lösungsmittels, gegebenenfalls in Gegenwart einer Base, umsetzt, dann die N,N'-Biscyanoethylverbindungen in Gegenwart von Basen in einem inerten Lösemittel verseift, dann im Fall der Herstellung der Cyclo-alkano[1.2-b]dihydroindol-sulfonamide die Cyclo-alkano[1.2-b]indol-sulfonamide gegebenenfalls in Gegenwart eines inerten Lösungsmittels in Gegenwart einer Säure und eines Reduktionsmittels hydriert, gegebenenfalls die Isomeren in üblicher Weise trennt, und dann gegebenenfalls im Fall der Herstellung der Salze mit einer entsprechenden Base umsetzt.

2. Verfahren nach Anspruch 1 zur Herstellung von Cycloalkano[1,2-b]indol-sulfonamiden der Formel

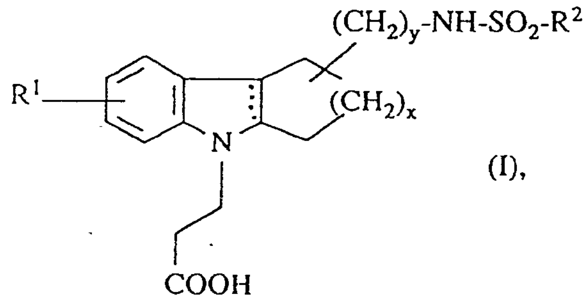

(I),

worin

$R^1$ – für Wasserstoff, Fluor, Chlor, Brom, Trifluormethyl, Methylthio, Ethylthio, Methylsulfonyl, Phenylthio, Phenylsulfonyl, Amino, Dimethylamino, Diethylamino, Acetylamino steht, oder

– für eine Gruppe der Formel

$-OR^6$ steht,

worin

$R^6$ – Wasserstoff, $C_1$-$C_4$-Alkyl oder Phenyl bedeutet,

– oder für $C_1$-$C_4$-Alkyl steht,

$R^2$ – für Phenyl steht, das bis zu zweifach gleich oder verschieden durch Fluor, Chlor, Brom, Cyano, Trifluormethyl, Trifluormethoxy, $C_1$-$C_4$-Alkyl, $C_1$-$C_4$-Alkoxy, Methylthio, Hydroxy, Methoxycarbonyl, Ethoxycarbonyl, Dimethylamino, Acetylamino, Diethylamino substituiert ist,

x – für die Zahl 1 oder 2 steht, und

y – für die Zahl 0 oder 1 steht, gegebenenfalls in einer isomeren Form, oder deren Salze.

3. Verfahren nach Anspruch 1 zur Herstellung von Cycloalkano[1,2-b]indol-sulfonamiden der Formel

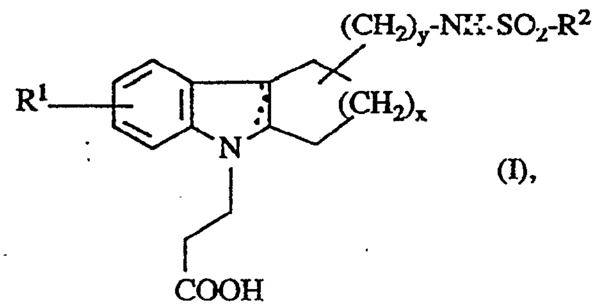

(I),

worin

R¹ – für Wasserstoff, Fluor, Methyl, Methoxy, Benzyloxy oder Hydroxy steht,

R² – für Phenyl steht, das durch Fluor, Chlor, Trifluormethyl, Methyl, Ethyl, Propyl, Isopropyl oder Methoxy substituiert ist,

x – für die Zahl 1 oder 2 steht, und

y – für die Zahl 0 oder 1 steht, gegebenenfalls in einer isomeren Form, oder deren Salze.

4. Verfahren nach Anspruch 1 zur Herstellung von (+) oder (–) isomeren Cycloalkano[1.2-b]indol-sulfonamiden der Formel

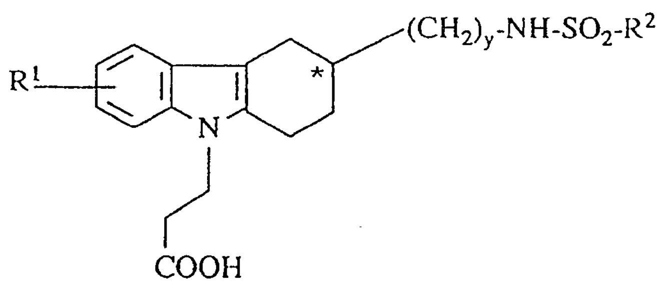

(XII)

in der

R¹ – für Wasserstoff, fluor, Methyl, Methoxy, Benzyloxy oder Hydroxy steht,

R² – für Phenyl steht, das durch Fluor, Chlor, Trifluormethyl, Methyl, Ethyl, Propyl, Isopropyl, oder Methoxy substituiert ist, und

y – für die Zahl 0 oder 1 steht, oder deren Salze.

5. Verfahren nach Anspruch 1 zur Herstellung von (+)-3-(4-Fluor-phenyl-sulfonamido)-9-(2-carboxy-ethyl)-1,2,3,4-tetrahydrocarbazol.

6. Verfahren nach Anspruch 1 zur Herstellung von (–)-3-(4-Fluor-phenyl-sulfonamido)-9-(2-carboxy-ethyl)-1,2,3,4-tetrahydrocarbazol.

7. Verfahren nach den Ansprüchen 1 bis 6, dadurch gekennzeichnet, daß man die Umsetzung des [Benzolsulfonamidoalkyl]cycloalkano[1,2-b]indols mit Acrylnitril im Temperaturbereich von 0°C bis +150°C durchführt.

8. Verfahren nach den Ansprüchen 1 bis 7, dadurch gekennzeichnet, daß man Acrylnitril und [Benzolsulfonamidoalkyl]cycloalkano[1,2-b]indol im Verhältnis 1 bis 20 Mol zu 1 Mol einsetzt.

9. Verfahren zur Herstellung von [Benzolsulfonamidoalkyl]cycloalkano[1.2-b]indolen der Formel

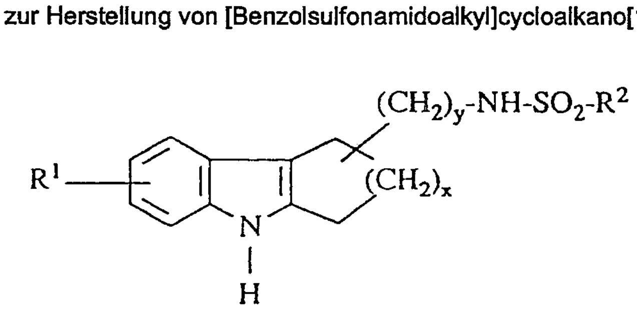

(XIII)

in der

R¹ – für Wasserstoff, Fluor, Chlor, Brom, Trifluormethyl, Carboxy, $C_1$-$C_6$-Alkoxycarbonyl steht,

– für eine Gruppe der Formel

$-S(O)_mR^3$ steht,

worin

$R^3$ – $C_1$-$C_6$-Alkyl oder Phenyl bedeutet und

m – eine Zahl 0 oder 2 bedeutet,

– für eine Gruppe der Formel

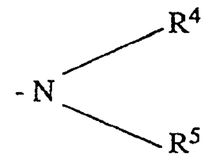

steht, worin

$R^4$ und $R^5$ gleich oder verschieden sind und Wasserstoff, $C_1$-$C_6$-Alkyl, Phenyl oder Acetyl bedeuten,

– oder für eine Gruppe der Formel

$-OR^6$ steht,

worin

$R^6$ – Wasserstoff, $C_1$-$C_6$-Alkyl oder Phenyl bedeutet,

– oder für gegebenenfalls durch Carboxy, Methoxycarbonyl, Ethoxycarbonyl, Fluor, Chlor, Brom, Hydroxy, $C_1$-$C_6$,-Alkoxy oder Cyano substituiertes $C_1$-$C_6$-Alkyl steht,

$R^2$ – für Phenyl steht, das gegebenenfalls bis zu dreifach durch Fluor, Chlor, Brom, Cyano, Trifluormethyl, Trifluormethoxy, Trifluormethylthio, $C_1$-$C_6$-Alkyl, Carboxymethyl, Carboxyethyl, Methoxymethyl, Ethoxymethyl, Methoxyethyl, Ethoxyethyl, $C_1$-$C_6$-Alkoxy, $C_1$-$C_6$-Alkylthio, Hydroxy, Carboxy, $C_1$-$C_6$-Alkoxycarbonyl, Phenyl, Phenoxy, Benzyloxy, Benzylthio oder durch die Gruppe

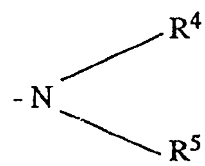

substituiert ist, worin

$R^4$ und $R^5$ die bereits angegebene Bedeutung haben,

x – für die Zahl 1 oder 2 steht, und

y – für die Zahl 0 oder 1 steht, gegebenenfalls in einer isomeren Form, dadurch gekennzeichnet, daß man Phenylhydrazine der allgemeinen Formel

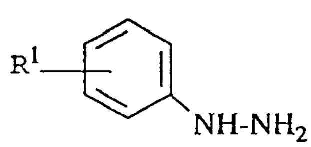

(XIV)

in welcher

$R^1$ – die oben angegebene Bedeutung hat, mit Cycloalkanonsulfonamiden der allgemeinen Formel

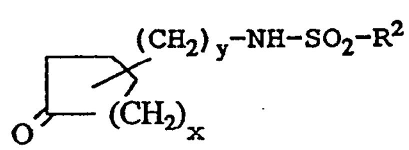

(XV)

in welcher

$R^2$, x und y die oben angegebene Bedeutung haben, in Gegenwart von inerten Lösungsmitteln, gegebenenfalls in Anwesenheit eines Katalysators umsetzt.

10. Verfahren zur Herstellung von Cycloalkanosulfonamiden der Formel

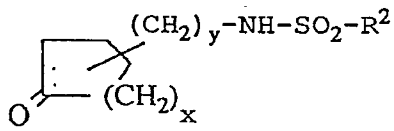

(XVb)

in der

$R^2$ – für Phenyl steht, das gegebenenfalls bis zu dreifach durch Fluor, Chlor, Brom, Cyano, Trifluormethyl, Trifluormethoxy, Trifluormethylthio, $C_1$-$C_6$-Alkyl, Carboxymethyl, Carboxyethyl, Methoxymethyl, Ethoxymethyl, Methoxyethyl, Ethoxyethyl, $C_1$-$C_6$-Alkoxy, $C_1$-$C_6$-Alkylthio, Hydroxy, Carboxy, $C_1$-$C_6$-Alkoxycarbonyl, Phenyl, Phenoxy, Benzyloxy, Benzylthio oder durch die Gruppe

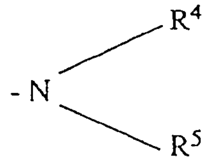

substituiert ist, worin

$R^4$ und $R^5$ gleich oder verschieden sind und Wasserstoff, $C_1$-$C_6$-Alkyl, Phenyl oder Acetyl bedeuten,

x – für die Zahl 1 steht, und

y – für die Zahl 0 oder 1 steht, dadurch gekennzeichnet, daß man Cycloalkanole der Formel

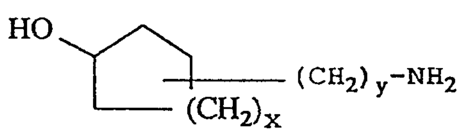

(XVI)

in der

x und y die oben angegebene Bedeutung haben, mit Sulfonsäurehalogeniden der Formel

$$Hal - SO_2 - R^2 \qquad (XVII)$$

in der

$R^2$ die oben angegebene Bedeutung hat und

Hal für Fluor, Chlor, Brom oder Iod steht, in inertem organischen Lösungsmittel, gegebenenfalls in Gegenwart von Basen, umsetzt und anschließend in inertem Lösungsmittel mit Chrom(VI)-Verbindungen oxidiert.

## Claims

**Claims for the Contracting States : AT, BE, CH, DE, FR, GB, GR, IT, LI, LU, NL, SE**

1. Cycloalkano[1.2-b]indole-sulphonamides of the formula

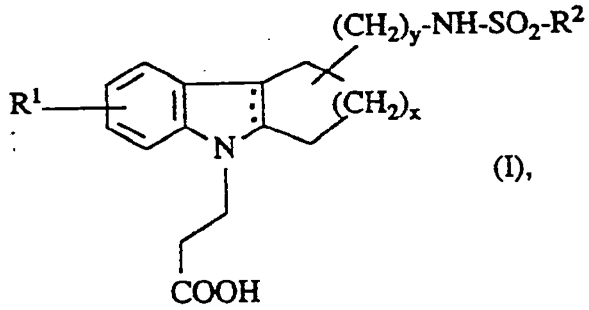

(I),

in which

$R^1$ represents hydrogen, fluorine, chlorine, bromine, triflouromethyl, carboxyl or $C_1$-$C_6$-alkoxycarbonyl, represents a group of the formula

$$-S(O)_mR^3$$

in which

$R^3$ denotes $C_1$-$C_6$-alkyl or phenyl, and

m denotes a number 0 or 2, represents a group of the formula

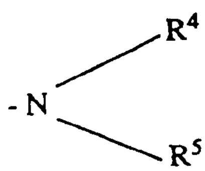

in which

$R^4$ and $R^5$ are identical or different and denote hydrogen, $C_1$-$C_6$-alkyl, phenyl, or acetyl, or represents a group of the formula

$$-OR^6,$$

in which

$R^6$ denotes hydrogen, $C_1$-$C_6$-alkyl, or phenyl, or represents $C_1$-$C_6$-alkyl which is optionally substituted by carboxyl, methoxycarbonyl, ethoxycarbonyl, fluorine, chlorine, bromine, hydroxyl, $C_1$-$C_6$-alkoxy or cyano,

$R^2$ represents phenyl which is optionally substituted up to three times by fluorine, chlorine, bromine, cyano, trifluoromethyl, trifluoromethoxy, trifluoromethylthio, $C_1$-$C_6$-alkyl, carboxymethyl, carboxyethyl, methoxymethyl, ethoxymethyl, methoxyethyl, ethoxyethyl, $C_1$-$C_6$-alkoxy, $C_1$-$C_6$-alkylthio, hydroxyl, carboxyl, $C_1$-$C_6$-alkoxycarbonyl, phenyl, phenoxy, benzyloxy, benzylthio or by the group

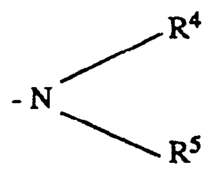

in which

$R^4$ and $R^5$ have the meaning already indicated,

x represents the number 1 or 2, and

y represents the number 0 or 1, if appropriate in an isomeric form, or their salts.

2. Cycloalkano[1.2-b]indole-sulphonamides according to Claim 1, in which

$R^1$ represents hydrogen, fluorine, chlorine, bromine, trifluoromethyl, methylthio, ethylthio, methylsulphonyl, phenylthio, phenylsulphonyl, amino, dimethylamino, diethylamino or acetylamino, or represents a group of the formula

$$-OR^6,$$

in which

$R^6$ denotes hydrogen, $C_1$-$C_4$-alkyl or phenyl

or represents $C_1$-$C_4$-alkyl,

$R^2$ represents phenyl which is substituted up to twice, identically or differently, by fluorine, chlorine, bromine, cyano, trifluoromethyl, trifluoromethoxy, $C_1$-$C_4$-alkyl, $C_1$-$C_4$-alkoxy, methylthio, hydroxyl, methoxycarbonyl, ethoxycarbonyl, dimethylamino, acetylamino, or diethylamino

x represents the number 1 or 2, and

y represents the number 0 or 1, if appropriate in an isomeric form, or their salts.

3. Cycloalkano[1.2-b]indole-sulphonamides according to Claim 1 in which

$R^1$ represents hydrogen, fluorine, methyl, methoxy benzyloxy or hydroxyl,

$R^2$ represents phenyl which is substituted by fluorine, chlorine, trifluoromethyl, methyl, ethyl, propyl, isopropyl or methoxy,

x represents the number 1 or 2, and

y represents the number 0 or 1, if appropriate in an isomeric form, or their salts.

4. (+) or (–) isomeric cycloalkano[1.2-b]indole-sulphonamides according to Claims 1 to 3 of the formula

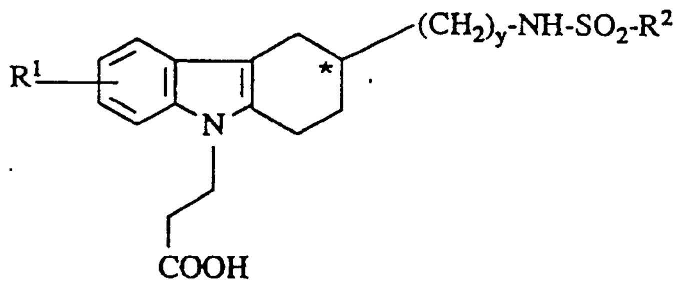

(XII)

in which

$R^1$ represents hydrogen, fluorine, methyl, methoxy, benzyloxy or hydroxyl,

$R^2$ represents phenyl which is substituted by fluorine, chlorine, trifluoromethyl, methyl, ethyl, propyl, isopropyl or methoxy, and

y represents the number 0 or 1, or their salts.

5. (+)-3-(4-Fluoro-phenyl-sulphonamido)-9-(2-carboxy-ethyl)-1,2,3,4-tetrahydrocarbazole.

6. (–)-3-(4-Fluoro-phenyl-sulphonamido)-9-(2-carboxy-ethyl)-1,2,3,4-tetrahydrocarbazole.

7. Process for the preparation of cycloalkano[1.2-b]indole-sulphonamides of the formula

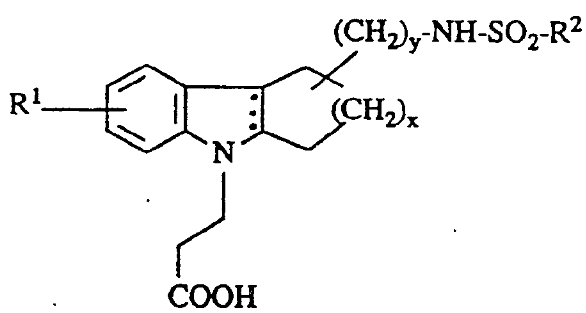

(I)

in which

$R^1$ represents hydrogen, fluorine, chlorine, bromine, trifluoromethyl, carboxyl or $C_1$-$C_6$-alkoxycarbonyl, represents a group of the formula

$$-S(O)_mR^3,$$

in which

$R^3$ denotes $C_1$-$C_6$-alkyl or phenyl, and

m denotes a number 0 or 2, represents a group of the formula

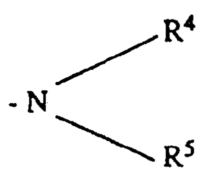

in which

$R^4$ and $R^5$ are identical or different and denote hydrogen, $C_1$-$C_6$-alkyl, phenyl or acetyl, or represents a group of the formula

$$-OR^6,$$

in which

$R^6$ denotes hydrogen, $C_1$-$C_6$-alkyl or phenyl, or represents $C_1$-$C_6$-alkyl, which is optionally substituted by carboxyl, methoxycarbonyl, ethoxycarbonyl, fluorine, chlorine, bromine, hydroxyl, $C_1$-$C_6$-alkoxy or cyano,

$R^2$ represents phenyl which is optionally substituted up to 3 times by fluorine, chlorine, bromine, cyano, trifluoromethyl, trifluoromethoxy, trifluoromethylthio, $C_1$-$C_6$-alkyl, carboxymethyl, carboxyethyl, methoxymethyl, ethoxymethyl, methoxyethyl, ethoxyethyl, $C_1$-$C_6$-alkoxy, $C_1$-$C_6$-alkylthio, hydroxyl, carboxyl, $C_1$-$C_6$-alkoxycarbonyl, phenyl, phenoxy, benzyloxy, benzylthio or by the group

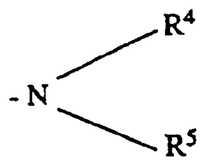

in which

$R^4$ and $R^5$ have the meaning already indicated,

x represents the number 1 or 2, and

y represents the number 0 or 1, if appropriate in an isomeric form, or their salts, characterized in that [benzenesulphonamidoalkyl]cycloalkano[1.2-b]indoles of the general formula

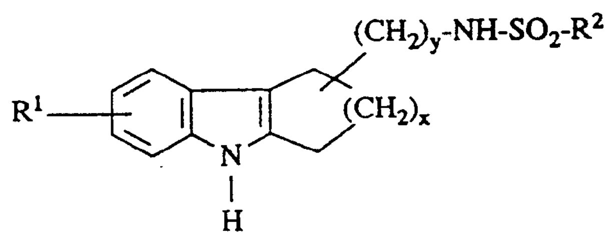

(XIII)

in which

$R^1$, $R^2$, x and y have the abovementioned meaning, are reacted with acrylonitrile in the presence of an inert solvent, where appropriate in the presence of a base, then the N,N'-biscyanoethyl compounds are hydrolysed in the presence of bases in an inert solvent, then, in the case where the cyclo-alkano[1.2-b]dihydroindole-sulphonamides are being prepared the cyclo-alkano[1.2-b]indole-sulphonamides are hydrogenated, where appropriate in the presence of an inert solvent, in the presence of an acid and of a reducing agent, where appropriate the isomers are separated in a customary manner, and then, where appropriate, in the case where the salts are being prepared reaction with an appropriate base is carried out.

8. Process according to Claim 7, characterized in that the reaction of the [benzenesulphonamidoalkyl]-cycloalkano[1.2-b]indole with acrylonitrile is carried out in the temperature range from 0 to 150°C.

9. Process according to Claims 7 and 8, characterized in that acrylonitrile and [benzenesulphonamidoalkyl]-cycloalkano[1.2-b]indole are used in the ratio 1 to 20 mol to 1 mol.

10. [Benzenesulphonamidoalkyl]cycloalkano[1.2-b]-indoles of the formula

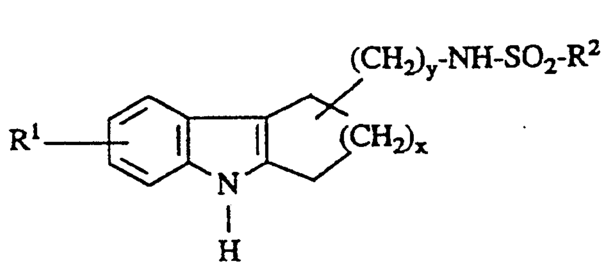

(XIII)

in which

$R^1$ represents hydrogen, fluorine, chlorine, bromine, trifluoromethyl, carboxyl or $C_1$-$C_6$-alkoxycarbonyl, rep-

resents a group of the formula

$$-S(O)_mR^3$$

in which

$R^3$ denotes $C_1$-$C_6$-alkyl or phenyl, and

m denotes a number 0 or 2, represents a group of the formula

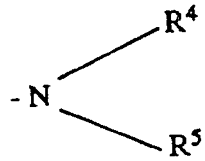

in which

$R^4$ and $R^5$ are identical or different and denote hydrogen, $C_1$-$C_6$-alkyl, phenyl or acetyl, or represents a group of the formula

$$-OR^6,$$

in which

$R^6$ denotes hydrogen, $C_1$-$C_6$-alkyl or phenyl, or represents $C_1$-$C_6$-alkyl, which is optionally substituted by carboxyl, methoxycarbonyl, ethoxycarbonyl, fluorine, chlorine, bromine, hydroxyl, $C_1$-$C_6$-alkoxy or cyano,

$R^2$ represents phenyl which is optionally substituted up to three times by fluorine, chlorine, bromine, cyano, trifluoromethyl, trifluoromethoxy, trifluoromethylthio, $C_1$-$C_6$-alkyl, carboxymethyl, carboxyethyl, methoxymethyl, ethoxymethyl, methoxyethyl, ethoxyethyl, $C_1$-$C_6$-alkoxy, $C_1$-$C_6$-alkylthio, hydroxyl, carboxyl, $C_1$-$C_6$-alkoxycarbonyl, phenyl, phenoxy, benzyloxy, benzylthio or by the group

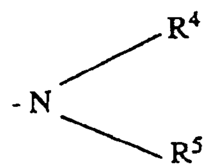

in which

$R^4$ and $R^5$ have the meaning already indicated,

x represents the number 1 or 2, and

y represents the number 0 or 1, if appropriate in an isomeric form.

11. Process for the preparation of [benzenesulphonamidoalkyl]cycloalkano[1.2-b]indoles of the formula

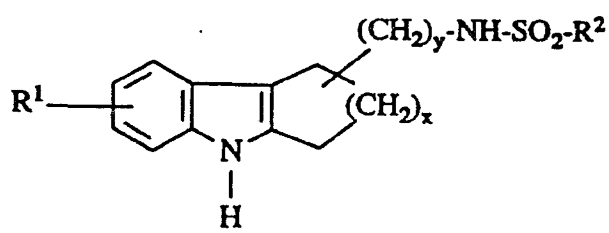

(XIII)

in which

$R^1$ represents hydrogen, fluorine, chlorine, bromine, trifluoromethyl, carboxyl or $C_1$-$C_6$-alkoxycarbonyl, represents a group of the formula

$$-S(O)_mR^3$$

in which

$R^3$ denotes $C_1$-$C_6$-alkyl or phenyl, and

m denotes a number 0 to 2, represents a group of the formula

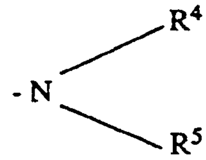

in which

$R^4$ and $R^5$ are identical or different and denote hydrogen, $C_1$-$C_6$-alkyl, phenyl or acetyl, or represents a group of the formula

$$-OR_6,$$

in which

$R^6$ denotes hydrogen, $C_1$-$C_6$-alkyl or phenyl, or represents $C_1$-$C_6$-alkyl which is optionally substituted by carboxyl, methoxycarbonyl, ethoxycarbonyl, fluorine, chlorine, bromine, hydroxyl, $C_1$-$C_6$-alkoxy or cyano,

$R^2$ represents phenyl which is optionally substituted up to three times by fluorine, chlorine, bromine, cyano, trifluoromethyl, trifluoromethoxy, trifluoromethylthio, $C_1$-$C_6$-alkyl, carboxymethyl, carboxyethyl, methoxymethyl, ethoxymethyl, methoxyethyl, ethoxyethyl, $C_1$-$C_6$-alkoxy, $C_1$-$C_6$-alkylthio, hydroxyl, carboxyl, $C_1$-$C_6$-alkoxycarbonyl, phenyl, phenoxy, benzyloxy, benzylthio or by the group

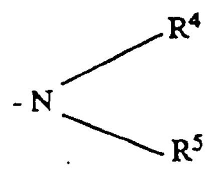

in which

$R^4$ and $R^5$ have the meaning already indicated,

x represents the number 1 or 2, and

y represents the number 0 or 1, if appropriate in an isomeric form, characterized in that phenylhydrazines of the general formula

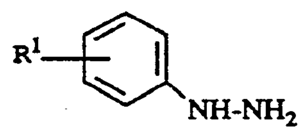

(XIV)

in which

$R^1$ has the abovementioned meaning, are reacted with cycloalkanonesulphonamides of the general formula

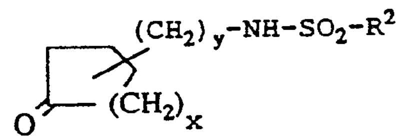

(XV)

in which

$R^2$, x and y have the abovementioned meaning, in the presence of inert solvents and, where appropriate, in the presence of a catalyst.

12. Ketones of the formula

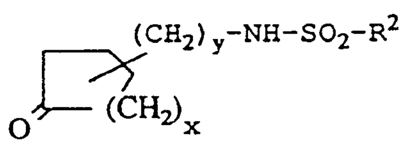

(XVb)

in which

$R^2$ represents phenyl which is optionally substituted up to three times by fluorine, chlorine, bromine, cyano, trifluoromethyl, trifluoromethoxy, trifluormethylthio, $C_1$-$C_6$-alkyl,carboxymethyl, carboxyethyl, methoxymethyl, ethoxymethyl, methoxyethyl, ethoxyethyl, $C_1$-$C_6$-alkoxy, $C_1$-$C_6$-alkylthio, hydroxyl, carboxyl, $C_1$-$C_6$-alkoxycarbonyl, phenyl, phenoxy, benzyloxy, benzylthio or by the group

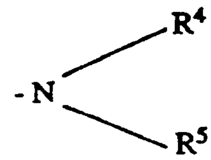

in which

$R^4$ and $R^5$ are identical or different and denote hydrogen, $C_1$-$C_6$-alkyl, phenyl or acetyl,
x represents the number 1, and
y represents the number 0 or 1.

13. Process for the preparation of cycloalkanonesulphonamides of the formula

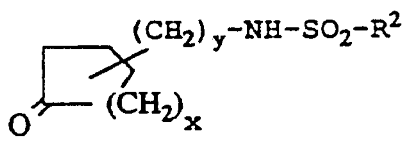

(XVb)

in which

$R^2$ represents phenyl which is optionally substituted up to three times by fluorine, chlorine, bromine, cyano, trifluoromethyl, trifluoromethoxy, trifluoromethylthio, $C_1$-$C_6$-alkyl, carboxymethyl, carboxyethyl, methoxymethyl, ethoxymethyl, methoxyethyl, ethoxyethyl, $C_1$-$C_6$-alkoxy, $C_1$-$C_6$-alkylthio, hydroxyl, carboxyl, $C_1$-$C_6$-alkoxycarbonyl, phenyl, phenoxy, benzyloxy, benzylthio or by the group

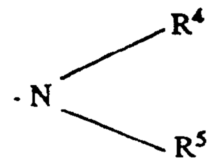

in which

$R^4$ and $R^5$ are identical or different and denote hydrogen, $C_1$-$C_6$-alkyl, phenyl or acetyl,
x represents the number 1, and
y represents the number 0 or 1, characterized in that cycloalkanols of the formula

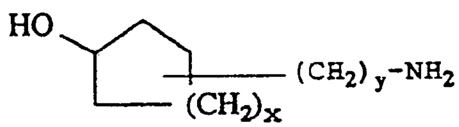

(XVI)

in which

x and y have the abovementioned meaning, are reacted with sulphonyl halides of the formula

$$Hal\text{-}SO_2\text{-}R^2 \qquad (XVII)$$

in which

$R^2$ has the abovementioned meaning, and Hal represents fluorine, chlorine, bromine or iodine, in an inert organic solvent, where appropriate in the presence of bases, and then oxidation with chromium (VI) compounds

in an inert solvent is carried out.

14. Medicament containing cycloalkano[1.2-b]indole-sulphonamides according to Claim 1.

15. Use of cycloalkano[1.2-b]indole-sulphonamides according to Claim 1 for the preparation of medicaments.

16. Medicament according to Claim 14 for treating thromboses, thromboembolisms, ischaemias, asthma and allergies.

17. Cycloalkano[1.2-b]indole-sulphonamides according to Claim 1 for controlling diseases.

**Claims for the Contracting State : ES**

1. Process for the preparation of cycloalkano[1.2-b]indole-sulphonamides of the formula

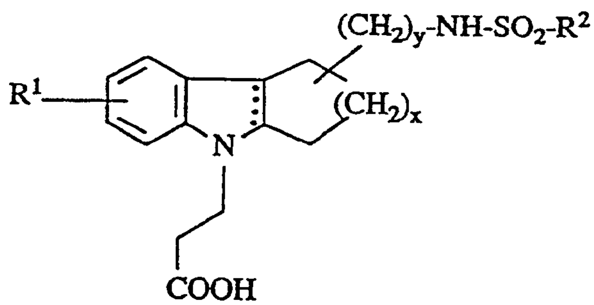

(I)

in which

$R^1$ represents hydrogen, fluorine, chlorine, bromine, trifluoromethyl, carboxyl or $C_1$-$C_6$-alkoxycarbonyl, represents a group of the formula

$$-S(O)_mR^3$$

in which

$R^3$ denotes $C_1$-$C_6$-alkyl or phenyl, and

m denotes a number 0 or 2, represents a group of the formula

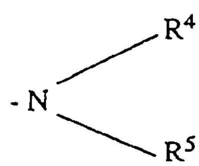

in which

$R^4$ and $R^5$ are identical or different and denote hydrogen, $C_1$-$C_6$-alkyl, phenyl or acetyl, or represents a group of the formula

$$-OR_6,$$

in which

$R^6$ denotes hydrogen, $C_1$-$C_6$-alkyl or phenyl, or represents $C_1$-$C_6$-alkyl which is optionally substituted by carboxyl, methoxycarbonyl, ethoxycarbonyl, fluorine, chlorine, bromine, hydroxyl, $C_1$-$C_6$-alkoxy or cyano,

$R^2$ represents phenyl which is optionally substituted up to three times by fluorine, chlorine, bromine, cyano, trifluoromethyl, trifluoromethoxy, trifluoromethylthio, $C_1$-$C_6$-alkyl, carboxymethyl, carboxyethyl, methoxymethyl, ethoxymethyl, methoxyethyl, ethoxyethyl, $C_1$-$C_6$-alkoxy, $C_1$-$C_6$-alkylthio, hydroxyl, carboxyl, $C_1$-$C_6$-alkoxycarbonyl, phenyl, phenoxy, benzyloxy, benzylthio or by the group

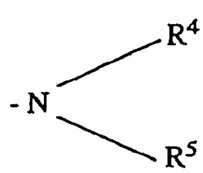

in which

$R^4$ and $R^5$ have the meaning already indicated,

x represents the number 1 or 2, and

y represents the number 0 or 1, if appropriate in an isomeric form, or their salts, characterized in that [benzenesulphonamidoalkyl]cycloalkano[1.2-b]indoles of the general formula

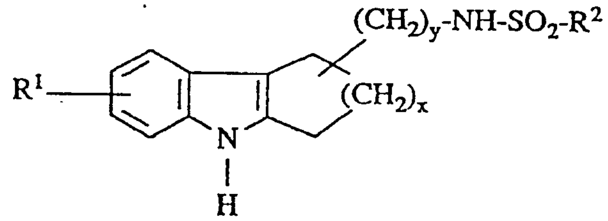

(XIII)

in which

$R^1$, $R^2$, x and y have the abovementioned meaning, are reacted with acrylonitrile in the presence of an inert solvent, where appropriate in the presence of a base, then the N,N'-biscyanoethyl compounds are hydrolysed in the presence of bases in an inert solvent, then, in the case where the cyclo-alkano[1.2-b]dihydroindolesulphonamides are being prepared the cyclo-alkano[1.2-b]indole-sulphonamides are hydrogenated, where appropriate in the presence of an inert solvent, in the presence of an acid and of a reducing agent, where appropriate the isomers are separated in a customary manner, and then, where appropriate, in the case where the salts are being prepared reaction with an appropriate base is carried out.

2. Process according to Claim 1 for the preparation of cycloalkano[1.2-b]indole-sulphonamides of the formula

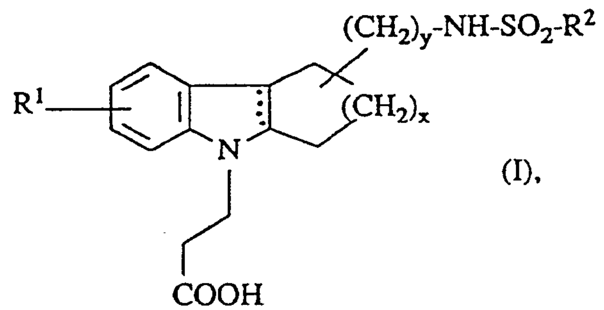

(I),

in which

$R^1$ represents hydrogen, fluorine, chlorine, bromine, trifluoromethyl, methylthio, ethylthio, methylsulphonyl, phenylthio, phenylsulphonyl, amino, dimethylamino, diethylamino or acetylamino, or represents a group of the formula

$$-OR^6,$$

in which

$R^6$ denotes hydrogen, $C_1$-$C_4$-alkyl or phenyl or represents $C_1$-$C_4$-alkyl,

$R^2$ represents phenyl which is substituted up to twice, identically or differently, by fluorine, chlorine, bromine, cyano, trifluoromethyl, trifluoromethoxy, $C_1$-$C_4$-alkyl, $C_1$-$C_4$-alkoxy, methylthio, hydroxyl, methoxycarbonyl, ethoxycarbonyl, dimethylamino, acetylamino, or diethylamino,

x represents the number 1 or 2, and

y represents the number 0 or 1, if appropriate in an isomeric form, or their salts.

3. Process according to Claim 1 for the preparation of cycloalkano[1.2-b]indole-sulphonamides of the formula

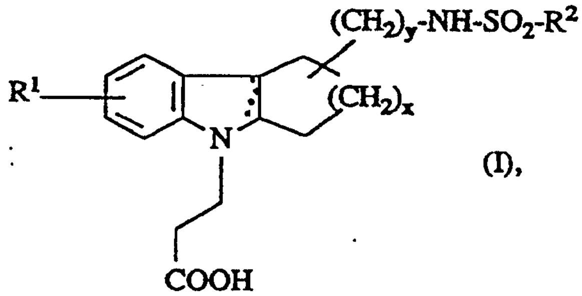

(I),

in which

$R^1$ represents hydrogen, fluorine, methyl, methoxy, benzyloxy or hydroxyl,

$R^2$ represents phenyl which is substituted by fluorine, chlorine, trifluoromethyl, methyl, ethyl, propyl, isopropyl or methoxy,

x represents the number 1 or 2, and

y represents the number 0 or 1, if appropriate in an isomeric form, or their salts.

4. Process according to Claim 1 for the preparation of (+) or (–) isomeric cycloalkano[1.2-b]indole-sulphonamides of the formula

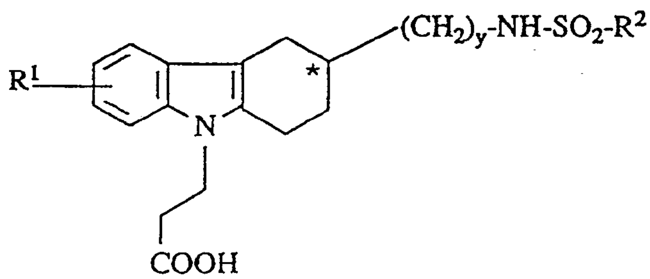

(XII)

in which

$R^1$ represents hydrogen, fluorine, methyl, methoxy, benzyloxy or hydroxyl,

$R^2$ represents phenyl which is substituted by fluorine, chlorine, trifluoromethoxy, methyl, ethyl, propyl, isopropyl or methoxy, and

y represents the number 0 or 1, or their salts.

5. Process according to Claim 1 for preparation of (+)-3-(4-fluoro-phenyl-sulphonamido)-9-(2-carboxyethyl)-1,2,3,4-tetrahydrocarbazole.

6. Process according to Claim 1 for the preparation of (–)-3-(4-fluoro-phenyl-sulphonamido)-9-(2-carboxyethyl)-1,2,3,4-tetrahydrocarbazole.

7. Process according to Claims 1 to 6, characterized in that the reaction of the [benzenesulphonamidoalkyl]-cycloalkano[1.2-b]indole with acrylonitrile is carried out in the temperature range from 0°C to +150°C.

8. Process according to Claims 1 to 7, characterized in that acrylonitrile and [benzenesulphonamidoalkyl]-cycloalkano[1.2-b]indole are used in the ratio 1 to 20 mol to 1 mol.

9. Process for the preparation of [benzenesulphonamidoalkyl]cycloalkano[1.2-b]indoles of the formula

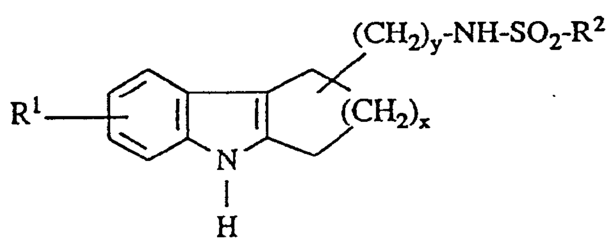

(XIII)

in which

$R^1$ represents hydrogen, fluorine, chlorine, bromine, trifluoromethyl, carboxyl or $C_1$-$C_6$-alkoxycarbonyl, represents a group of the formula

$$-S(O)_mR^3$$

in which

$R^3$ denotes $C_1$-$C_6$-alkyl or phenyl, and

m denotes a number 0 or 2, represents a group of the formula

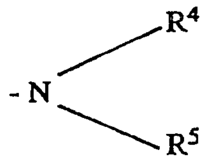

in which

$R^4$ and $R^5$ are identical or different and denote hydrogen, $C_1$-$C_6$-alkyl, phenyl or acetyl, or represents a group of the formula

$$-OR^6,$$

in which

$R^6$ denotes hydrogen, $C_1$-$C_6$-alkyl, or phenyl, or represents $C_1$-$C_6$-alkyl which is optionally substituted by carboxyl, methoxycarbonyl, ethoxycarbonyl, fluorine, chlorine, bromine, hydroxyl, $C_1$-$C_6$-alkoxy or cyano,

$R^2$ represents phenyl which is optionally substituted up to three times by fluorine, chlorine, bromine, cyano, trifluoromethyl, trifluoromethoxy, trifluoromethylthio, $C_1$-$C_6$-alkyl, carboxymethyl, carboxyethyl, methoxymethyl, ethoxymethyl, methoxyethyl, ethoxyethyl, $C_1$-$C_6$-alkoxy, $C_1$-$C_6$-alkylthio, hydroxyl, carboxyl, $C_1$-$C_6$-alkoxy-carbonyl, phenyl, phenoxy, benzyloxy, benzylthio or by the group

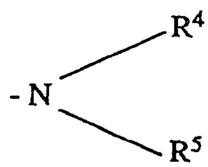

in which

$R^4$ and $R^5$ have the meaning already indicated,

x represents the number 1 or 2, and

y represents the number 0 or 1, if appropriate in an isomeric form, characterized in that phenylhydrazines of the general formula

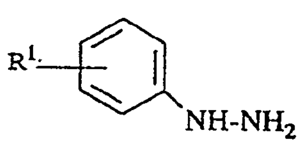

(XIV)

in which

$R^1$ has the abovementioned meaning, are reacted with cycloalkanonesulphonamides of the general formula

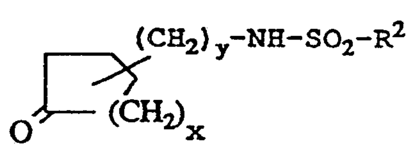

(XV)

in which

$R^2$, x and y have the abovementioned meaning, in the presence of inert solvents and, where appropriate, in the presence of a catalyst.

10. Process for the preparation of cycloalkanonesulphonamides of the formula

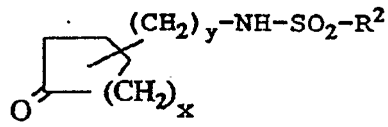

(XVb)

in which

$R^2$ represents phenyl which is optionally substituted up to three times by fluorine, chlorine, bromine, cyano, trifluoromethyl, trifluoromethoxy, trifluoromethylthio, $C_1$-$C_6$-alkyl, carboxymethyl, carboxyethyl, methoxymethyl, ethoxymethyl, methoxyethyl, ethoxyethyl, $C_1$-$C_6$-alkoxy, $C_1$-$C_6$-alkylthio, hydroxyl, carboxyl, $C_1$-$C_6$-alkoxycarbonyl, phenyl, phenoxy, benzyloxy, benzylthio or by the group

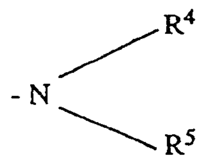

in which

$R^4$ and $R^5$ are identical or different and denote hydrogen, $C_1$-$C_6$-alkyl, phenyl or acetyl,

x represents the number 1, and

y represents the number 0 or 1, characterized in that cycloalkanols of the formula

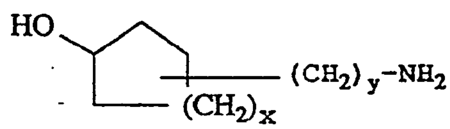

(XVI)

in which

x and y have the abovementioned meaning, are reacted with sulphonyl halides of the formula

$$Hal\text{-}SO_2\text{-}R^2 \qquad (XVII)$$

in which

$R^2$ has the abovementioned meaning, and

Hal represents fluorine, chlorine, bromine or iodine, in an inert organic solvent, where appropriate in the presence of bases, and then oxidation with chromium (VI) compounds in an inert solvent is carried out.

## Revendications

### Revendications pour les Etats contractants : AT, BE, CH, DE, FR, GB, GR, IT, LI, LU, NL, SE

1. Cycloalcano[1.2-b]indole-sulfonamides de formule

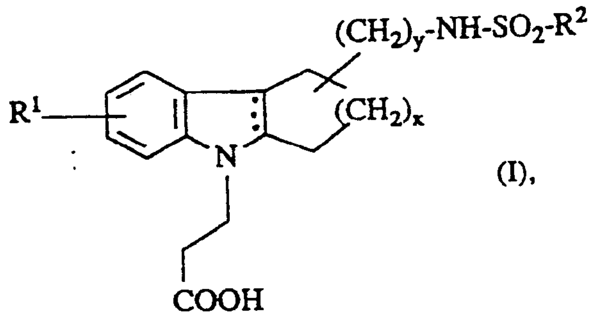

(I),

dans laquelle

$R^1$ représente un atome d'hydrogène, un atome de fluor, un atome de chlore, un atome de brome, un groupe trifluorométhyle, un groupe carboxyle, un groupe alcoxy(en $C_1$-$C_6$)carbonyle, un groupe de formule

$$-S(O)_mR^3$$

dans laquelle

$R^3$ représente un groupe alkyle en $C_1$-$C_6$ ou un groupe phényle et

m représente le chiffre 0 ou 2, un groupe de formule

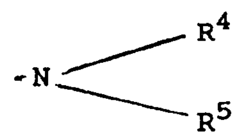

dans laquelle

$R^4$ et $R^5$ sont identiques ou différents et représentent un atome d'hydrogène, un groupe alkyle en $C_1$-$C_6$, un groupe phényle ou un groupe acétyle, ou encore un groupe de formule

$$-OR^6$$

dans laquelle

$R^6$ représente un atome d'hydrogène, un groupe alkyle en $C_1$-$C_6$ ou un groupe phényle, ou encore un groupe alkyle en $C_1$-$C_6$ éventuellement substitué par un groupe carboxyle, un groupe méthoxycarbonyle, un groupe éthoxycarbonyle, un atome de fluor, un atome de chlore, un atome de brome, un groupe hydroxyle, un groupe alcoxy en $C_1$-$C_6$ ou un groupe alkyle en $C_1$-$C_6$ substitué par un groupe cyano ;

$R^2$ représente un groupe phényle éventuellement substitué, jusqu'à trois fois, par un atome de fluor, un atome de chlore, un atome de brome, un groupe cyano, un groupe trifluorométhyle, un groupe trifluorométhoxy, un groupe trifluorométhylthio, un groupe alkyle en $C_1$-$C_6$, un groupe carboxyméthyle, un groupe carboxyéthyle, un groupe méthoxyméthyle, un groupe éthoxyméthyle, un groupe méthoxyéthyle, un groupe éthoxyéthyle, un groupe alcoxy en $C_1$-$C_6$, un groupe alkyl(en $C_1$-$C_6$)-thio, un groupe hydroxyle, un groupe carboxyle, un groupe alcoxy(en $C_1$-$C_6$)-carbonyle, un groupe phényle, un groupe phénoxy, un groupe benzyloxy, un groupe benzylthio ou par le groupe

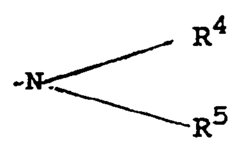

dans lequel

$R^4$ et $R^5$ ont la signification déjà mentionnée ci-dessus ;

x représente le chiffre 1 ou 2, et

y représente le chiffre 0 ou 1, éventuellement sous une forme isomère ou leurs sels.

2. Cycloalcano[1.2-b]indole-sulfonamides selon la revendication 1, dans lesquels

$R^1$ représente un atome d'hydrogène, un atome de fluor, un atome de chlore, un atome de brome, un groupe trifluorométhyle, un groupe méthylthio, un groupe éthylthio, un groupe méthylsulfonyle, un groupe phénylthio,

un groupe phénylsulfonyle, un groupe amino, un groupe diméthylamino, un groupe diéthylamino, un groupe acétylamino ou un groupe de formule

$$-OR^6$$

dans laquelle

$R^6$ représente un atome d'hydrogène, un groupe alkyle en $C_1$-$C_4$ ou un groupe phényle, ou un groupe alkyle en $C_1$-$C_4$,

$R^2$ représente un groupe phényle substitué jusqu'à deux fois, de manière identique ou différente, par un atome de fluor, un atome de chlore, un atome de brome, un groupe cyano, un groupe trifluorométhyle, un groupe trifluorométhoxy, un groupe alkyle en $C_1$-$C_4$, un groupe alcoxy en $C_1$-$C_4$, un groupe méthylthio, un groupe hydroxyle, un groupe méthoxycarbonyle, un groupe éthoxycarbonyle, un groupe diméthylamino, un groupe acétylamino, un groupe diéthylamino ;

x représente le chiffre 1 ou 2, et

y représente le chiffre 0 ou 1, éventuellement sous une forme isomère ou leurs sels.

3. Cycloalcano[1.2-b]indole-sulfonamides selon la revendication 1, dans lesquels

$R^1$ représente un atome d'hydrogène, un atome de fluor, un groupe méthyle, un groupe méthoxy, un groupe benzyloxy ou un groupe hydroxyle ;

$R^2$ représente un groupe phényle substitué par un atome de fluor, un atome de chlore, un groupe trifluorométhyle, un groupe méthyle, un groupe éthyle, un groupe propyle, un groupe isopropyle ou un groupe méthoxy ;

x représente le chiffre 1 ou 2 et

y représente le chiffre 0 ou 1, éventuellement sous une forme isomère ou leurs sels.

4. Cycloalcano[1.2-b]indole-sulfonamides isomères (+) ou (–) selon les revendications 1 à 3, répondant à la formule

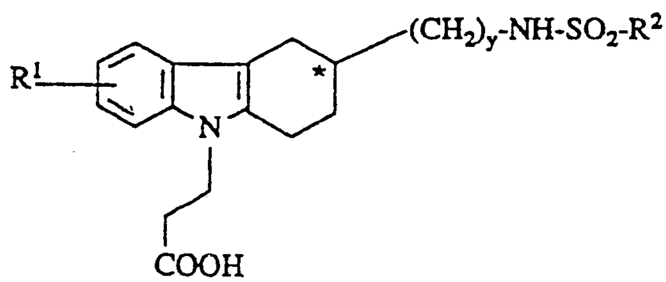

(XII)

dans laquelle

$R^1$ représente un atome d'hydrogène, un atome de fluor, un groupe méthyle, un groupe méthoxy, un groupe benzyloxy ou un groupe hydroxyle ;

$R^2$ représente un groupe phényle substitué par un atome de fluor, un atome de chlore, un groupe trifluorométhyle, un groupe méthyle; un groupe éthyle, un groupe propyle, un groupe isopropyle, ou un groupe méthoxy ; et

y représente le chiffre 0 ou 1, ou leurs sels.

5. (+)-3-(4-fluorophénylsulfonamido)-9-(2-carboxyéthyl)-1,2,3,4-tétrahydrocarbazol.

6. (–)-3-(4-fluorophénylsulfonamido)-9-(2-carboxy-éthyl)-1,2,3,4-tétrahydrocarbazol.

7. Procédé pour la préparation de cycloalcano[1.2-b]indole-sulfonamides de formule

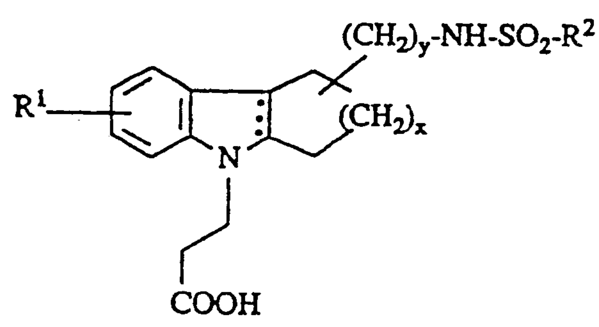

(I)

dans laquelle

$R^1$ représente un atome d'hydrogène, un atome de fluor, un atome de chlore, un atome de brome, un groupe trifluorométhyle, un groupe carboxyle, un groupe alcoxy(en $C_1$-$C_6$)carbonyle, un groupe de formule

$$-S(O)_mR^3$$

dans laquelle

$R^3$ représente un groupe alkyle en $C_1$-$C_6$ ou un groupe phényle et

m représente le chiffre 0 ou 2, un groupe de formule

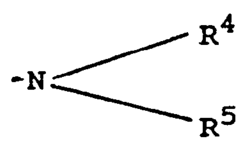

dans laquelle

$R^4$ et $R^5$ sont identiques ou différents et représentent un atome d'hydrogène, un groupe alkyle en $C_1$-$C_6$, un groupe phényle ou un groupe acétyle, ou encore un groupe de formule

$$-OR^6$$

dans laquelle

$R^6$ représente un atome d'hydrogène, un groupe alkyle en $C_1$-$C_6$ ou un groupe phényle, ou encore un groupe alkyle en $C_1$-$C_6$ éventuellement substitué par un groupe carboxyle, un groupe méthoxycarbonyle, un groupe éthoxycarbonyle, un atome de fluor, un atome de chlore, un atome de brome, un groupe hydroxyle, un groupe alcoxy en $C_1$-$C_6$ ou un groupe alkyle en $C_1$-$C_6$ substitué par un groupe cyano ;

$R^2$ représente un groupe phényle éventuellement substitué, jusqu'à trois fois, par un atome de fluor, un atome de chlore, un atome de brome, un groupe cyano, un groupe trifluorométhyle, un groupe trifluorométhoxy, un groupe trifluorométhylthio, un groupe alkyle en $C_1$-$C_6$, un groupe carboxyméthyle, un groupe carboxyéthyle, un groupe méthoxyméthyle, un groupe éthoxyméthyle, un groupe méthoxyéthyle, un groupe éthoxyéthyle, un groupe alcoxy en $C_1$-$C_6$, un groupe alkyl(en $C_1$-$C_6$)-thio, un groupe hydroxyle, un groupe carboxyle, un groupe alcoxy(en $C_1$-$C_6$)-carbonyle, un groupe phényle, un groupe phénoxy, un groupe benzyloxy, un groupe benzylthio ou par le groupe

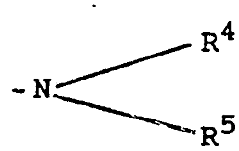

dans lequel

$R^4$ et $R^5$ ont la signification déjà mentionnée ci-dessus ;

x représente le chiffre 1 ou 2, et

y représente le chiffre 0 ou 1, éventuellement sous une forme isomère ou leurs sels, **caractérisé en ce qu'**on fait réagir des [benzènesulfonamidoalkyl]cycloalcano[1.2-b]-indoles répondant à la formule générale

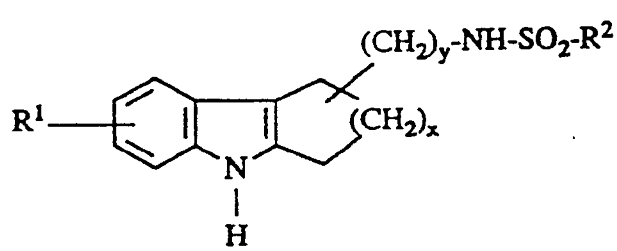

(XIII)

dans laquelle

$R^1$, $R^2$, x et y ont la signification mentionnée ci-dessus, avec de l'acrylonitrile en présence d'un solvant inerte, éventuellement en présence d'une base ; ensuite, on saponifie les composés N,N'-biscyanoéthyle en

présence de bases dans un solvant inerte ; ensuite, dans le cas de la préparation des cycloalcano[1.2-b]dihydroindole-sulfonamides, on soumet les cycloalcano[1.2-b]indole-sulfonamides à une hydrogénation, éventuellement en présence d'un solvant inerte, en présence d'un acide et d'un agent de réduction ; on sépare éventuellement les isomères de façon habituelle ; et ensuite, éventuellement, dans le cas de la préparation des sels, on les fait réagir avec une base correspondante.

8. Procédé selon la revendication 7, **caractérisé en ce qu'**on effectue la réaction du [benzènesulfonamidoalkyl]cycloalcano[1.2-b]indole avec de l'acrylonitrile dans un domaine de température allant de 0 à 150°C.

9. Procédé selon les revendications 7 et 8, **caractérisé en ce qu'**on met en oeuvre l'acrylonitrile et le [benzènesulfonamidoalkyl]-cycloalcano[1.2-b]indole dans un rapport de 1 à 20 moles pour une mole.

10. [Benzènesulfonamidoalkyl]cycloalcano[1.2b]-indoles de formule

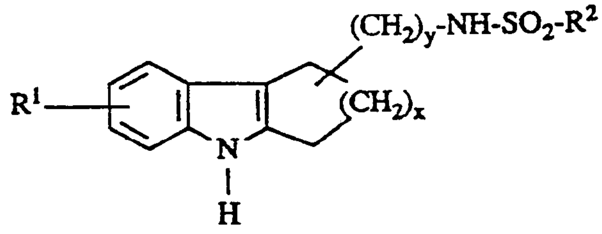

(XIII)

dans laquelle

$R^1$ représente un atome d'hydrogène, un atome de fluor, un atome de chlore, un atome de brome, un groupe trifluorométhyle, un groupe carboxyle, un groupe alcoxy(en $C_1$-$C_6$)carbonyle, un groupe de formule

$$-S(O)_mR^3$$

dans laquelle

$R^3$ représente un groupe alkyle en $C_1$-$C_6$ ou un groupe phényle et
m représente le chiffre 0 ou 2, un groupe de formule

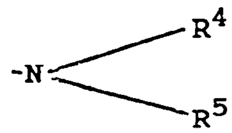

dans laquelle

$R^4$ et $R^5$ sont identiques ou différents et représentent un atome d'hydrogène, un groupe alkyle en $C_1$-$C_6$, un groupe phényle ou un groupe acétyle, ou encore un groupe de formule

$$-OR^6$$

dans laquelle

$R^6$ représente un atome d'hydrogène, un groupe alkyle en $C_1$-$C_8$ ou un groupe phényle, ou encore un groupe alkyle en $C_1$-$C_8$ éventuellement substitué par un groupe carboxyle, un groupe méthoxycarbonyle, un groupe éthoxycarbonyle, un atome de fluor, un atome de chlore, un atome de brome, un groupe hydroxyle, un groupe alcoxy en $C_1$-$C_6$ ou un groupe alkyle en $C_1$-$C_6$ substitué par un groupe cyano ;

$R^2$ représente un groupe phényle éventuellement substitué, jusqu'à trois fois, par un atome de fluor, un atome de chlore, un atome de brome, un groupe cyano, un groupe trifluorométhyle, un groupe trifluorométhoxy, un groupe trifluorométhylthio, un groupe alkyle en $C_1$-$C_6$, un groupe carboxyméthyle, un groupe carboxyéthyle, un groupe méthoxyméthyle, un groupe éthoxyméthyle, un groupe méthoxyéthyle, un groupe éthoxyéthyle, un groupe alcoxy en $C_1$-$C_6$, un groupe alkyl(en $C_1$-$C_6$)-thio, un groupe hydroxyle, un groupe carboxyle, un groupe alcoxy(en $C_1$-$C_6$)-carbonyle, un groupe phényle, un groupe phénoxy, un groupe benzyloxy, un groupe benzylthio ou par le groupe

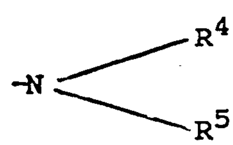

dans lequel

$R^4$ et $R^5$ ont la signification déjà mentionnée ci-dessus ;

x représente le chiffre 1 ou 2, et

y représente le chiffre 0 ou 1, éventuellement sous une forme isomère.

11. Procédé pour la préparation de [benzènesulfonamidoalkyl]cycloalcano[1.2-b]indoles de formule

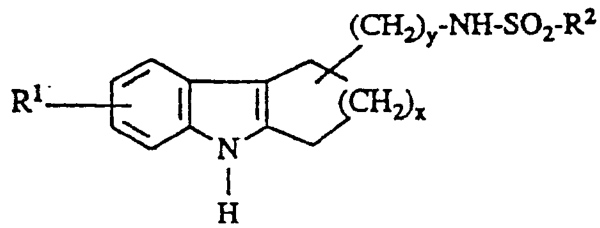

(XIII)

dans laquelle

$R^1$ représente un atome d'hydrogène, un atome de fluor, un atome de chlore, un atome de brome, un groupe trifluorométhyle, un groupe carboxyle, un groupe alcoxy(en $C_1$-$C_6$)carbonyle, un groupe de formule

$$-S(O)_mR^3$$

dans laquelle

$R^3$ représente un groupe alkyle en $C_1$-$C_6$ ou un groupe phényle et

m représente le chiffre 0 ou 2, un groupe de formule

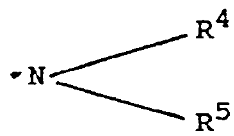

dans laquelle

$R^4$ et $R^5$ sont identiques ou différents et représentent un atome d'hydrogène, un groupe alkyle en $C_1$-$C_6$, un groupe phényle ou un groupe acétyle, ou encore un groupe de formule

$$-OR^6$$

dans laquelle

$R^6$ représente un atome d'hydrogène, un groupe alkyle en $C_1$-$C_6$ ou un groupe phényle, ou encore un groupe alkyle en $C_1$-$C_6$ éventuellement substitué par un groupe carboxyle, un groupe méthoxycarbonyle, un groupe éthoxycarbonyle, un atome de fluor, un atome de chlore, un atome de brome, un groupe hydroxyle, un groupe alcoxy en $C_1$-$C_6$ ou un groupe alkyle en $C_1$-$C_6$ substitué par un groupe cyano ;

$R^2$ représente un groupe phényle éventuellement substitué, jusqu'à trois fois, par un atome de fluor, un atome de chlore, un atome de brome, un groupe cyano, un groupe trifluorométhyle, un groupe trifluorométhoxy, un groupe trifluorométhylthio, un groupe alkyle en $C_1$-$C_6$, un groupe carboxyméthyle, un groupe carboxyéthyle, un groupe méthoxyméthyle, un groupe éthoxyméthyle, un groupe méthoxyéthyle, un groupe éthoxyéthyle, un groupe alcoxy en $C_1$-$C_6$, un groupe alkyl(en $C_1$-$C_6$)-thio, un groupe hydroxyle, un groupe carboxyle, un groupe alcoxy(en $C_1$-$C_6$)-carbonyle, un groupe phényle, un groupe phénoxy, un groupe benzyloxy, un groupe benzylthio ou par le groupe

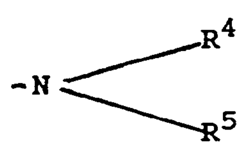

dans lequel

$R^4$ et $R^5$ ont la signification déjà mentionnée ci-dessus ;

x représente le chiffre 1 ou 2, et

y représente le chiffre 0 ou 1, éventuellement sous une forme isomère, **caractérisé en ce qu'**on fait réagir

des phénylhydrazines de formule générale

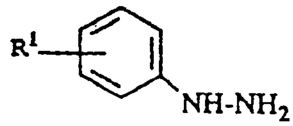

(XIV)

dans laquelle

$R^1$ a la signification définie ci-dessus, avec des cycloalcanonesulfonamides de formule générale

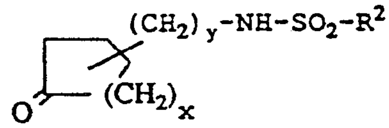

(XV)

dans laquelle

$R^2$, x et y ont la signification mentionnée ci-dessus, en présence de solvants inertes, éventuellement en présence d'un catalyseur.

12. Cétones de formule

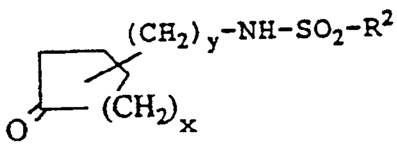

(XVb)

dans laquelle

$R^2$ représente un groupe phényle éventuellement substitué, jusqu'à trois fois, par un atome de fluor, un atome de chlore, un atome de brome, un groupe cyano, un groupe trifluorométhyle, un groupe trifluorométhoxy, un groupe trifluorométhylthio, un groupe alkyle en $C_1$-$C_6$, un groupe carboxyméthyle, un groupe carboxyéthyle, un groupe méthoxyméthyle, un groupe éthoxyméthyle, un groupe méthoxyéthyle, un groupe éthoxyéthyle, un groupe alcoxy en $C_1$-$C_6$, un groupe alkyl(en $C_1$-$C_6$)-thio, un groupe hydroxyle, un groupe carboxyle, un groupe alcoxy(en $C_1$-$C_6$)-carbonyle, un groupe phényle, un groupe phénoxy, un groupe benzyloxy, un groupe benzylthio ou par le groupe

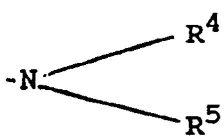

dans lequel

$R^4$ et $R^5$ ont la signification déjà mentionnée ci-dessus ;

x représente le chiffre 1, et

y représente le chiffre 0 ou 1.

13. Procédé pour la préparation de cycloalcanosulfonamides de formule

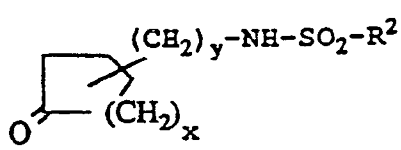

(XVb)

dans laquelle

$R^2$ représente un groupe phényle éventuellement substitué, jusqu'à trois fois, par un atome de fluor, un atome de chlore, un atome de brome, un groupe cyano, un groupe trifluorométhyle, un groupe trifluorométhoxy,

un groupe trifluorométhylthio, un groupe alkyle en $C_1$-$C_6$, un groupe carboxyméthyle, un groupe carboxyéthyle, un groupe méthoxyméthyle, un groupe éthoxyméthyle, un groupe méthoxyéthyle, un groupe éthoxyéthyle, un groupe alcoxy en $C_1$-$C_6$, un groupe alkyl(en $C_1$-$C_6$)-thio, un groupe hydroxyle, un groupe carboxyle, un groupe alcoxy(en $C_1$-$C_6$)-carbonyle, un groupe phényle, un groupe phénoxy, un groupe benzyloxy, un groupe benzylthio ou par le groupe

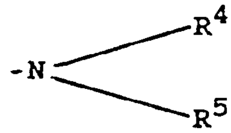

dans lequel

$R^4$ et $R^5$ ont la signification déjà mentionnée ci-dessus ;

x représente le chiffre 1, et

y représente le chiffre 0 ou 1, **caractérisé en ce qu'**on fait réagir des cycloalcanols de formule

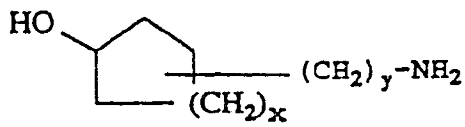 (XVI)

dans laquelle

x et y ont la signification mentionnée ci-dessus, avec des halogénures d'acide sulfonique de formule

$$Hal-SO_2-R^2 \qquad (XVII)$$

dans laquelle

$R^2$ a la signification définie ci-dessus et

Hal représente un atome de fluor, un atome de chlore, un atome de brome ou un atome d'iode, dans un solvant organique inerte, éventuellement en présence de bases ; ensuite, on les oxyde dans un solvant inerte avec des composés de chrome(VI).

14. Médicaments contenant des cycloalcano[1.2-b]indole-sulfonamides selon la revendication 1.

15. Utilisation de cycloalcano[1.2-b]indole-sulfonamides selon la revendication 1, pour la préparation de médicaments.

16. Médicaments selon la revendication 14, pour le traitement de thromboses, de thrombo-embolies, d'ischémies, d'asthme et d'allergies.

17. Cycloalcano[1.2-b]indole-sulfonamides selon la revendication 1, destinés à combattre des maladies.

**Revendications pour l'Etat contractant : ES**

1. Procédé pour la préparation de cycloalcano[1.2-b]indole-sulfonamides de formule

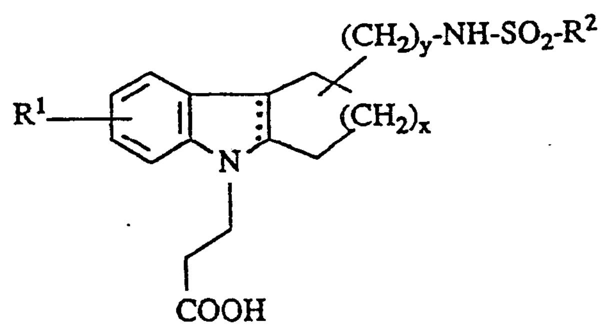 (I)

dans laquelle

$R^1$ représente un atome d'hydrogène, un atome de fluor, un atome de chlore, un atome de brome, un groupe trifluorométhyle, un groupe carboxyle, un groupe alcoxy(en $C_1$-$C_6$)carbonyle, un groupe de formule

$$-S(O)_mR^3$$

dans laquelle

$R^3$ représente un groupe alkyle en $C_1$-$C_6$ ou un groupe phényle et

m représente le chiffre 0 ou 2, un groupe de formule

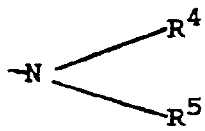

dans laquelle

$R^4$ et $R^5$ sont identiques ou différents et représentent un atome d'hydrogène, un groupe alkyle en $C_1$-$C_6$, un groupe phényle ou un groupe acétyle, ou encore un groupe de formule

$$-OR^6$$

dans laquelle

$R^6$ représente un atome d'hydrogène, un groupe alkyle en $C_1$-$C_6$ ou un groupe phényle, ou encore un groupe alkyle en $C_1$-$C_6$ éventuellement substitué par un groupe carboxyle, un groupe méthoxycarbonyle, un groupe éthoxycarbonyle, un atome de fluor, un atome de chlore, un atome de brome, un groupe hydroxyle, un groupe alcoxy en $C_1$-$C_6$ ou un groupe alkyle en $C_1$-$C_6$ substitué par un groupe cyano ;

$R^2$ représente un groupe phényle éventuellement substitué, jusqu'à trois fois, par un atome de fluor, un atome de chlore, un atome de brome, un groupe cyano, un groupe trifluorométhyle, un groupe trifluorométhoxy, un groupe trifluorométhylthio, un groupe alkyle en $C_1$-$C_6$, un groupe carboxyméthyle, un groupe carboxyéthyle, un groupe méthoxyméthyle, un groupe éthoxyméthyle, un groupe méthoxyéthyle, un groupe éthoxyéthyle, un groupe alcoxy en $C_1$-$C_6$, un groupe alkyl(en $C_1$-$C_6$)-thio, un groupe hydroxyle, un groupe carboxyle, un groupe alcoxy(en $C_1$-$C_6$)-carbonyle, un groupe phényle, un groupe phénoxy, un groupe benzyloxy, un groupe benzylthio ou par le groupe

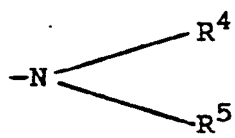

dans lequel

$R^4$ et $R^5$ ont la signification déjà mentionnée ci-dessus ;

x représente le chiffre 1 ou 2, et

y représente le chiffre 0 ou 1, éventuellement sous une forme isomère ou leurs sels, **caractérisé en ce qu'**on fait réagir des [benzènesulfonamidoalkyl]cycloalcano[1.2-b]-indoles répondant à la formule générale

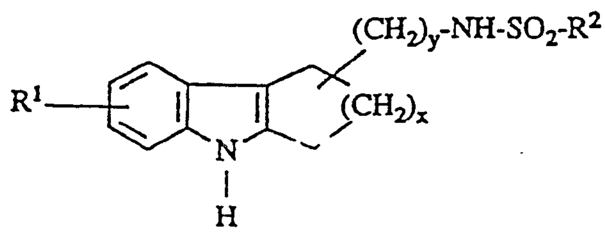

(XIII)

dans laquelle

$R^1$, $R^2$, x et y ont la signification mentionnée ci-dessus, avec de l'acrylonitrile en présence d'un solvant inerte, éventuellement en présence d'une base ; ensuite, on saponifie les composés N,N'-biscyanoéthyle en présence de bases dans un solvant inerte ; ensuite, dans le cas de la préparation des cycloalcano[1.2-b]dihy-

droindole-sulfonamides, on soumet les cycloalcano[1.2-b]indole-sulfonamides à une hydrogénation, éventuellement en présence d'un solvant inerte, en présence d'un acide et d'un agent de réduction ; on sépare éventuellement les isomères de façon habituelle ; et ensuite, éventuellement, dans le cas de la préparation des sels, on les fait réagir avec une base correspondante.

2. Procédé selon la revendication 1, pour la préparation de cycloalcano[1.2-b]indole-sulfonamides de formule

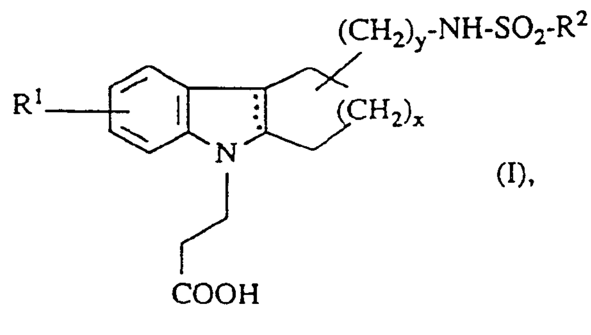

(I),

dans laquelle

$R^1$ représente un atome d'hydrogène, un atome de fluor, un atome de chlore, un atome de brome, un groupe trifluorométhyle, un groupe méthylthio, un groupe éthylthio, un groupe méthylsulfonyle, un groupe phénylthio, un groupe phénylsulfonyle, un groupe amino, un groupe diméthylamino, un groupe diéthylamino, un groupe acétylamino ou un groupe de formule

$$-OR^6$$

dans laquelle $R^6$ représente un atome d'hydrogène, un groupe alkyle en $C_1$-$C_4$ ou un groupe phényle, ou un groupe alkyle en $C_1$-$C_4$,

$R^2$ représente un groupe phényle substitué jusqu'à deux fois, de manière identique ou différente, par un atome de fluor, un atome de chlore, un atome de brome, un groupe cyano, un groupe trifluorométhyle, un groupe trifluorométhoxy, un groupe alkyle en $C_1$-$C_4$, un groupe alcoxy en $C_1$-$C_4$, un groupe méthylthio, un groupe hydroxyle, un groupe méthoxycarbonyle, un groupe éthoxycarbonyle, un groupe diméthylamino, un groupe acétylamino, un groupe diéthylamino ;

x représente le chiffre 1 ou 2, et

y représente le chiffre 0 ou 1, éventuellement sous une forme isomère ou leurs sels.

3. Procédé selon la revendication 1, pour la préparation de cycloalcano[1.2-b]indole-sulfonamides de formule

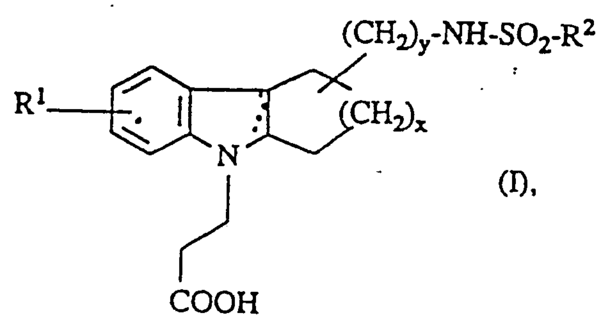

(I),

dans laquelle

$R^1$ représente un atome d'hydrogène, un atome de fluor, un groupe méthyle, un groupe méthoxy, un groupe benzyloxy ou un groupe hydroxyle ;

$R^2$ représente un groupe phényle substitué par un atome de fluor, un atome de chlore, un groupe trifluorométhyle, un groupe méthyle, un groupe éthyle, un groupe propyle, un groupe isopropyle ou un groupe méthoxy ;

x représente le chiffre 1 ou 2 et

y représente le chiffre 0 ou 1, éventuellement sous une forme isomère ou leurs sels.

4. Procédé selon la revendication 1, pour la préparation de cycloalcano[1.2-b]indole sulfonamides isomères (+) ou (–) répondant à la formule

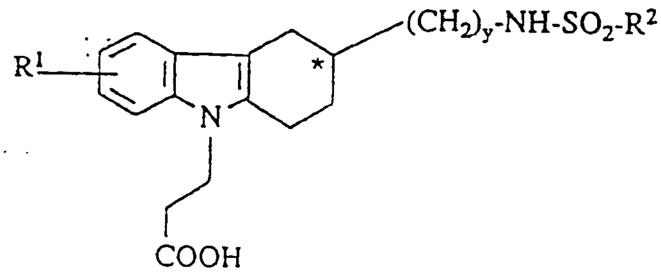

(XII)

dans laquelle

$R^1$ représente un atome d'hydrogène, un atome de fluor, un groupe méthyle, un groupe méthoxy, un groupe benzyloxy ou un groupe hydroxyle ;

$R^2$ représente un groupe phényle substitué par un atome de fluor, un atome de chlore, un groupe trifluorométhyle, un groupe méthyle, un groupe éthyle, un groupe propyle, un groupe isopropyle, ou un groupe méthoxy ; et

y représente le chiffre 0 ou 1, ou leurs sels.

5. Procédé selon la revendication 1, pour la préparation du (+)-3-(4-fluorophénylsulfonamido)-9-(2-carboxyéthyl)-1,2,3,4-tétrahydrocarbazol.

6. Procédé selon la revendication 1, pour la préparation du (–)-3-(4-fluorophénylsulfonamido)-9-(2-carboxyéthyl)-1,2,3,4-tétrahydrocarbazol.

7. Procédé selon les revendications 1 à 6, **caractérisé en ce qu'**on effectue la réaction du [benzènesulfonamidoalkyl]cycloalcano[1.2-b]indole avec de l'acrylonitrile dans un domaine de température allant de 0 à +150°C.

8. Procédé selon les revendications 1 à 7, **caractérisé en ce qu'**on met en oeuvre l'acrylonitrile et le [benzènesulfonamidoalkyl]-cycloalcano[1.2-b]indole dans un rapport de 1 à 20 moles pour une mole.

9. Procédé pour la préparation de [benzène-sulfonamidoalkyl]cycloalcano[1.2-b]indoles de formule

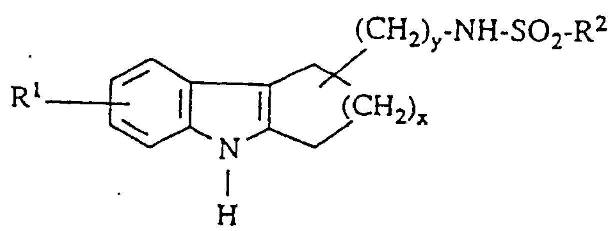

(XIII)

dans laquelle

$R^1$ représente un atome d'hydrogène, un atome de fluor, un atome de chlore, un atome de brome, un groupe trifluorométhyle, un groupe carboxyle, un groupe alcoxy(en $C_1$-$C_6$)carbonyle, un groupe de formule

$$-S(O)_mR^3$$

dans laquelle

$R^3$ représente un groupe alkyle en $C_1$-$C_6$ ou un groupe phényle et

m représente le chiffre 0 ou 2, un groupe de formule

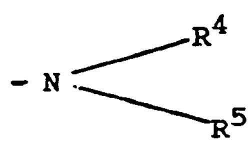

dans laquelle

$R^4$ et $R^5$ sont identiques ou différents et représentent un atome d'hydrogène, un groupe alkyle en $C_1$-$C_6$, un groupe phényle ou un groupe acétyle, ou encore un groupe de formule

$$-OR^6$$

dans laquelle

$R^6$ représente un atome d'hydrogène, un groupe alkyle en $C_1$-$C_6$ ou un groupe phényle, ou encore un groupe alkyle en $C_1$-$C_6$ éventuellement substitué par un groupe carboxyle, un groupe méthoxycarbonyle, un groupe éthoxycarbonyle, un atome de fluor, un atome de chlore, un atome de brome, un groupe hydroxyle, un groupe alcoxy en $C_1$-$C_6$ ou un groupe alkyle en $C_1$-$C_6$ substitué par un groupe cyano ;

$R^2$ représente un groupe phényle éventuellement substitué, jusqu'à trois fois, par un atome de fluor, un atome de chlore, un atome de brome, un groupe cyano, un groupe trifluorométhyle, un groupe trifluorométhoxy, un groupe trifluorométhylthio, un groupe alkyle en $C_1$-$C_6$, un groupe carboxyméthyle, un groupe carboxyéthyle, un groupe méthoxyméthyle, un groupe éthoxyméthyle, un groupe méthoxyéthyle, un groupe éthoxyéthyle, un groupe alcoxy en $C_1$-$C_6$, un groupe alkyl(en $C_1$-$C_6$)-thio, un groupe hydroxyle, un groupe carboxyle, un groupe alcoxy(en $C_1$-$C_6$)-carbonyle, un groupe phényle, un groupe phénoxy, un groupe benzyloxy, un groupe benzylthio ou par le groupe

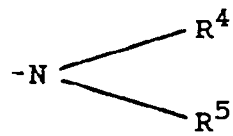

dans lequel

$R^4$ et $R^5$ ont la signification déjà mentionnée ci-dessus ;

x représente le chiffre 1 ou 2, et

y représente le chiffre 0 ou 1, éventuellement sous une forme isomère, **caractérisé en ce qu'**on fait réagir des phénylhydrazines de formule générale

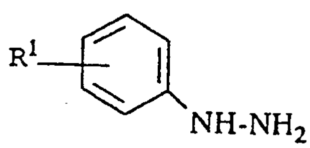

(XIV)

dans laquelle

$R^1$ a la signification définie ci-dessus, avec des cycloalcanonesulfonamides de formule générale

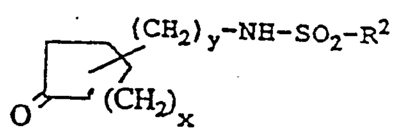

(XV)

dans laquelle

$R^2$, x et y ont la signification mentionnée ci-dessus, en présence de solvants inertes, éventuellement en présence d'un catalyseur.

10. Procédé pour la préparation de cycloalcanosulfonamides de formule

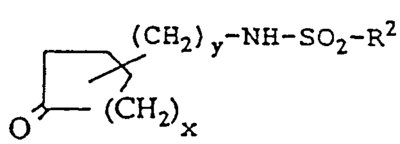

(XVb)

dans laquelle

$R^2$ représente un groupe phényle éventuellement substitué, jusqu'à trois fois, par un atome de fluor, un atome de chlore, un atome de brome, un groupe cyano, un groupe trifluorométhyle, un groupe trifluorométhoxy,

un groupe trifluorométhylthio, un groupe alkyle en $C_1$-$C_6$, un groupe carboxyméthyle, un groupe carboxyéthyle, un groupe méthoxyméthyle, un groupe éthoxyméthyle, un groupe méthoxyéthyle, un groupe éthoxyéthyle, un groupe alcoxy en $C_1$-$C_6$, un groupe alkyl(en $C_1$-$C_6$)-thio, un groupe hydroxyle, un groupe carboxyle, un groupe alcoxy(en $C_1$-$C_6$)-carbonyle, un groupe phényle, un groupe phénoxy, un groupe benzyloxy, un groupe benzylthio ou par le groupe

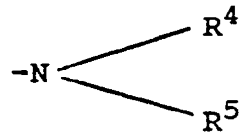

dans lequel

$R^4$ et $R^5$ ont la signification déjà mentionnée ci-dessus ;

x représente le chiffre 1, et

y représente le chiffre 0 ou 1, **caractérisé en ce qu'**on fait réagir des cycloalcanols de formule

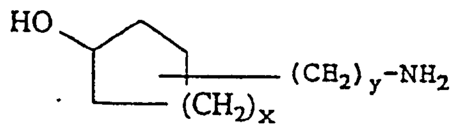

(XVI)

dans laquelle

x et y ont la signification mentionnée ci-dessus, avec des halogénures d'acide sulfonique de formule

$$Hal\text{-}SO_2\text{-}R^2 \qquad (XVII)$$

dans laquelle

$R^2$ a la signification définie ci-dessus et

Hal représente un atome de fluor, un atome de chiore, un atome de brome ou un atome d'iode, dans un solvant organique inerte, éventuellement en présence de bases ; ensuite, on les oxyde dans un solvant inerte avec des composés de chrome (VI)